# EUROPEAN PATENT APPLICATION

(11) **EP 4 617 266 A1**
(43) Date of publication of application: **17.09.2025**
(21) Application number: 23888143.7
(22) Date of filing: 13.11.2023
(51) Int. Cl.: C07D 401/06, C07D 403/06, A61K 31/454, A61K 31/4545, A61P 27/00, A61P 37/00

(54) **PIPERIDINE-SUBSTITUTED BENZOIC ACID COMPOUND, AND PHARMACEUTICAL COMPOSITION AND USE THEREOF**

(30) Priority: 11.11.2022 CN 202211414691
(71) Applicant: Shanghai Yisheng Biopharmaceutical company Limited, Shanghai 201401 (CN)
(72) Inventor: WU, Xuesong, Shanghai 200040 (CN); TANG, Zhiwei, Shanghai 200040 (CN); WANG, Hongbo, Shanghai 200040 (CN); CHEN, Likuo, Shanghai 200040 (CN); CHEN, Chen, Shanghai 200040 (CN); MAO, Yanjia, Shanghai 200040 (CN)
(74) Representative: Murgitroyd & Company
(86) International application number: PCT/CN2023/131348
(87) International publication number: WO 2024/099458

(57) **Abstract**

Disclosed in the present invention are a piperidine-substituted benzoic acid compound, and a pharmaceutical composition and the use thereof. The piperidine-substituted benzoic acid compound of the present invention is a compound as shown in formula (I) or a stereoisomer, a tautomer or an isotopic label thereof, or a pharmaceutically acceptable salt of any one of the foregoing. The compound has a good inhibitory activity on complement factor B, a good affinity activity on complement factor B, and a hemolytic activity and an inhibitory activity of an alternative complement pathway.

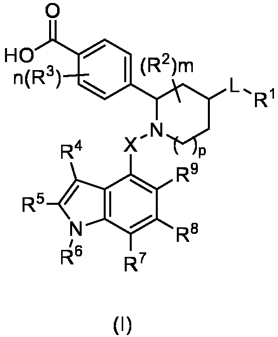

## Description

The present application claims the right of the priority of Chinese patent application 2022114146910 filed on November 11, 2022. The contents of the above Chinese patent application are incorporated herein by reference in their entireties.

### TECHNICAL FIELD

The present disclosure belongs to the field of medicine, and specifically relates to a piperidine-substituted benzoic acid compound, a pharmaceutical composition thereof, and a use thereof.

### BACKGROUND

The complement system is a group of nonspecific globulins present in the normal fresh serum of humans and vertebrates. It is related to enzyme activity. At the end of the 19th century, in the study of immune bacteriolysis and immune hemolysis reactions, this globulin was considered to be a substance played an auxiliary role in the cytolysis of antibodies, hence the name complement. Complement is composed of 9 components, named C1, C2, C3,..., C9. C1 has three subunits, namely C1q, C1r, and C1s. In addition to C1q, most other components are present in serum as precursors of enzymes, which require activation by antigen-antibody complexes or other factors to exert their biological activities. This is referred to as the classical activation pathway of complement. Over the past two decades, alternative activation pathways and several other activation pathways have been discovered. It has also been found that many other factors in serum are involved in the activation process of these pathways. In addition, many factors that inactivate complement have also been found. Therefore, the factors related to complement activity and its regulation are collectively referred to as the complement system. The body regulates the activation process of the complement system through a series of complex factors to make it respond moderately. For example, the biological effects of complement can be amplified through the positive feedback pathway via C3b. However, if the complement system is over-activated, not only will a large amount of complement components be consumed in vain, reducing the body's ability to resist infection; but a large amount of bioactive substances produced during activation will cause the body to have a severe inflammatory response or cause tissue damage, leading to pathological processes. This over-activation and its adverse consequences can be avoided through regulatory mechanisms. Although the initiation mechanisms and activation sequences of the three complement activation pathways differ, they share a common terminal pathway. In this process, the activation of the alternative pathway is not dependent on antigen-antibody complexes. Typically, C3b deposited on the cell surface binds to factor B and becomes readily cleaved by factor D in the serum. In this process, factor B is cleaved into Ba and Bb; then C3b and Bb form a complex to become the C3 convertase C3bBb in the alternative pathway; in this process, complement factor B plays an early and central role in the activation of the alternative pathway of the complement cascade. Here, C3b is both the product formed after the cleavage of C3 by a C3 convertase and a component of the C3 convertase of the alternative pathway, thereby establishing a feedback amplification mechanism where the classical and alternative pathways mutually influenced each other. Current studies have found that a variety of diseases such as hematological, autoimmune, inflammatory and neurodegenerative diseases are related to abnormal complement system function.

Paroxysmal nocturnal hemoglobinuria (PNH) is a nonmalignant clonal disorder resulting from acquired somatic PIG-A gene (phosphatidyl inositol glycan complementation group A) mutations in one or several hematopoietic stem cells. PIG-A mutations lead to abnormal synthesis of glycosyl phosphatidyl inositol (GPI), causing the loss of a group of membrane proteins anchored to the cell surface via GPI, including CD16, CD55, CD59, etc. Clinically, the disease primarily manifests as chronic intravascular hemolysis, hematopoietic failure, and recurrent thrombosis. Typical PNH is characterized by chronic intravascular hemolysis, hemoglobinuria, and hemosiderinuria. However, most patients present atypically, with an insidious onset, prolonged course, and varying degrees of disease severity. The peak age of onset is between 20 and 40 years of age, with rare cases occurring in children or the elderly, and males are significantly more affected than females. Currently, this condition can only be treated with immunotherapy, using eculizumab to alleviate symptoms. However, eculizumab is costly, and patients require continuous blood transfusions administered intravenously. Therefore, developing novel inhibitors targeting the complement system to meet clinical needs is of significant importance for patients.

C3 glomerulopathy (C3G) is a group of diseases in which complement C3 is abnormally deposited in the glomeruli due to hereditary or acquired regulatory defects in the alternative complement pathway (AP). The latest renal pathology classification in 2015 recommends categorizing C3G as a separate type of immune-related nephropathy, points out that the main feature of the disease is the predominant glomerular C3 deposition by immunofluorescence or immunohistochemistry, with minimal or no accompanying immunoglobulin deposition. Based on the different locations of dense deposits under electron microscopy, C3G is primarily classified into dense deposit disease (DDD) and C3 glomerulonephritis (C3GN). The clinical manifestations of C3G vary widely, often presenting as asymptomatic hematuria, proteinuria, or nephritic syndrome, nephrotic syndrome, and even renal insufficiency. Studies have shown that there are certain differences in the clinical manifestations between DDD and C3GN. Compared to DDD, the clinical manifestations of C3GN lack specificity, with no significant occurrence of lipid metabolism abnormalities. However, a small percentage of patients may develop monoclonal gammopathy. The prognosis of C3GN is relatively better for DDD patients, with a lower risk of progressing to ESRD. CFHR5 patients may present with microalbuminuria, microscopic or gross hematuria, resembling the clinical manifestations of IgA nephropathy. However, their serum C3 levels are basically normal, indicating that overactivation of C3 only exists in the glomeruli. CFHR5 patients progressing to ESRD are more commonly observed in children, particularly males. A retrospective study found that treatment with mycophenolate mofetil (MMF) or rituximab did not alter renal survival in patients with C3G. However, hormonal therapy is only effective for some C3GN patients and shows no response in DDD patients. Therefore, it is urgent to develop a novel AP pathway inhibitor that meets clinical demands.

Currently, there are no small-molecule drugs targeting complement factor B inhibitors available for clinical treatment. The currently known and ongoing research projects include an oligonucleotide drug developed by IONIS Pharmaceuticals Inc., which serves as a complement factor B (CFB)-specific inhibitor for treating, preventing, or alleviating diseases associated with dysregulation of the alternative complement pathway.

(WO201508939 A small-molecule complement factor B inhibitor developed by Novartis AG for the treatment of diseases such as age-related macular degeneration (AMD) WO2013164802 WO2013192345 WO2014143638 WO2015009616 WO2015066241, used for the treatment of diseases such as C3G and IgAN (WO2019043609A1). A small-molecule complement factor D inhibitor developed by Achillion Pharmaceuticals Inc. for the treatment of diseases such as age-related macular degeneration (AMD) (WO2018005552). A small-molecule complement factor B inhibitor developed by Shanghai Meiyue Biotech Development Co., Ltd. for the treatment of diseases such as age-related macular degeneration (AMD) (CN114057758A CN114057692A), and a small-molecule complement factor B inhibitor developed by MEDSHINE DISCOVERY Inc. for the treatment of diseases such as age-related macular degeneration (AMD) (WO2022143940A1).

China has the highest number of chronic kidney disease (CKD) patients in the world. There are no small-molecule targeted drugs for complement factor B on the market, but the immunotherapy drug eculizumab currently serves as the first-line treatment, and its high cost and poor healing properties lead to anemia in patients, necessitating blood transfusions for maintenance. Therefore, there is an urgent clinical need for an orally administered small-molecule inhibitor of the complement system to adapt to the medical treatment.

### SUMMARY

The technical problem to be addressed by the present disclosure is that there are few types of existing small molecule complement factor B inhibitors. The present disclosure provides a compound as a complement factor B inhibitor, a pharmaceutical composition thereof, and a use thereof. The compound has good inhibitory activity against complement factor B and can be used to treat diseases such as paroxysmal nocturnal hemoglobinuria, C3 glomerulopathy, IgA nephropathy, and age-related macular degeneration (AMD).

The present disclosure provides a compound of formula (I), a stereoisomer thereof, a tautomer thereof, an isotopically labeled compound thereof, or a pharmaceutically acceptable salt of any one of the foregoing (referring to the foregoing compound of formula (I), the stereoisomer thereof, the tautomer thereof, or the isotopically labeled compound thereof): 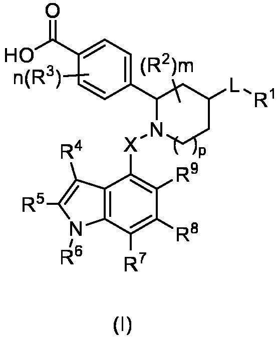 wherein
p is 0, 1, or 2;
L is a single bond, NR⁰, O, or S;
R¹ is hydrogen, C₁₋₆ alkyl, C₂₋₆ alkenyl, C₂₋₆ alkynyl, C₃₋₆ cycloalkyl, C₃₋₆ cycloalkenyl, C₃₋₆ cycloalkynyl, -C₁₋₃ alkyl-C₃₋₆ cycloalkyl, "3- to 12-membered heterocycloalkyl with 1, 2, or 3 heteroatoms selected from 1, 2, or 3 types of N, O, and S", -C₁₋₃ alkyl-"3- to 12-membered heterocycloalkyl with 1, 2, or 3 heteroatoms selected from 1, 2, or 3 types of N, O, and S", or the following group substituted by one or more R^{a} (e.g., 2, 3, 4, or 5; when R^{a} is multiple, R^{a} may be the same or different): C₁₋₆ alkyl, C₂₋₆ alkenyl, C₂₋₆ alkynyl, C₃₋₆ cycloalkyl, C₃₋₆ cycloalkenyl, C₃₋₆ cycloalkynyl, -C₁₋₃ alkyl-C₃₋₆ cycloalkyl, "3- to 12-membered heterocycloalkyl with 1, 2, or 3 heteroatoms selected from 1, 2, or 3 types of N, O, and S", or -C₁₋₃ alkyl-"3- to 12-membered heterocycloalkyl with 1, 2, or 3 heteroatoms selected from 1, 2, or 3 types of N, O, and S";
m and n are each independently 0, 1, 2, 3, or 4;
R², R³, R⁴, R⁵, R⁷, R⁸, and R⁹ are each independently hydrogen, halogen, OH, CN, NO₂, NH₂, C₁₋₆ alkyl, C₁₋₆ alkoxy, C₂₋₆ alkenyl, C₂₋₆ alkynyl, C₃₋₆ cycloalkyl, C₃₋₆ cycloalkenyl, C₃₋₆ cycloalkynyl, -C₁₋₃ alkyl-C₃₋₆ cycloalkyl, or the following group substituted by one or more R^{b} (e.g., 2, 3, 4, or 5; when R^{b} is multiple, R^{b} may be the same or different): C₁₋₆ alkyl, C₁₋₆ alkoxy, C₂₋₆ alkenyl, C₂₋₆ alkynyl, C₃₋₆ cycloalkyl, C₃₋₆ cycloalkenyl, C₃₋₆ cycloalkynyl, or -C₁₋₃ alkyl-C₃₋₆ cycloalkyl;
R⁰ and R⁶ are each independently hydrogen, C₁₋₆ alkyl, C₃₋₆ cycloalkyl, C₃₋₆ cycloalkenyl, C₃₋₆ cycloalkynyl, -C₁₋₃ alkyl-C₃₋₆ cycloalkyl, or the following group substituted by one or more R^{c} (e.g., 2, 3, 4, or 5; when R^{c} is multiple, R^{c} may be the same or different): C₁₋₆ alkyl, C₃₋₆ cycloalkyl, C₃₋₆ cycloalkenyl, C₃₋₆ cycloalkynyl, or -C₁₋₃ alkyl-C₃₋₆ cycloalkyl;
X is -C(=O)-, -C(=NR¹⁰)-, -C(=S)-, -CFR¹¹-, -NR¹²-, -CR¹³R¹⁴-CR¹⁵R¹⁶-, -S-, or - S(=O)₂-;
R¹¹, R¹³, R¹⁴, R¹⁵, and R¹⁶ are each independently hydrogen, halogen, OH, CN, NO₂, NH₂, C₁₋₆ alkyl, C₃₋₆ cycloalkyl, or the following group substituted by one or more R^{d} (e.g., 2, 3, 4, or 5; when R^{d} is multiple, R^{d} may be the same or different): C₁₋₆ alkyl or C₃₋₆ cycloalkyl;
R¹⁰ and R¹² are each independently hydrogen, C₁₋₆ alkyl, or C₁₋₆ alkyl substituted by one or more R^{e} (e.g., 2, 3, 4, or 5; when R^{e} is multiple, R^{e} may be the same or different);
R^{a}, R^{b}, R^{c}, R^{d}, and R^{e} are each independently halogen, OH, CN, NO₂, or NH₂.

In some preferred embodiments of the present disclosure, some groups of the compound of formula (I), the stereoisomer thereof, the tautomer thereof, the isotopically labeled compound thereof, or the pharmaceutically acceptable salt of any one of the foregoing (referring to the foregoing compound of formula (I), the stereoisomer thereof, the tautomer thereof, or the isotopically labeled compound thereof) are defined as follows, while unmentioned groups are the same as those described in any one of the embodiments of the present disclosure (referred to as "in a certain embodiment of the present disclosure").

In a certain embodiment of the present disclosure, in R⁰, R¹, R², R³, R⁴, R⁵, R⁶, R⁷, R⁸, R⁹, R¹⁰, R¹¹, R¹², R¹³, R¹⁴, R¹⁵, and R¹⁶, the C₁₋₆ alkyl in the C₁₋₆ alkyl and substituted C₁₋₆ alkyl is independently methyl, ethyl, n-propyl, isopropyl, n-butyl, isobutyl, *tert-butyl,* pentyl, isopentyl, neopentyl, or hexyl, such as methyl or ethyl.

In a certain embodiment of the present disclosure, in R², R³, R⁴, R⁵, R⁷, R⁸, and R⁹, the C₁₋₆ alkoxy in the C₁₋₆ alkoxy and substituted C₁₋₆ alkyl is independently methoxy, ethoxy, n-propoxy, isopropoxy, n-butoxy, isobutoxy, sec-butoxy, or *tert-*butoxy*,* such as methoxy.

In a certain embodiment of the present disclosure, in R⁰, R¹, R², R³, R⁴, R⁵, R⁶, R⁷, R⁸, R⁹, R¹¹, R¹³, R¹⁴, R¹⁵, and R¹⁶, the C₃₋₆ cycloalkyl in the C₃₋₆ cycloalkyl, -C₁₋₃ alkyl-C₃₋₆ cycloalkyl, substituted C₃₋₆ cycloalkyl, and substituted -C₁₋₃ alkyl-C₃₋₆ cycloalkyl is independently cyclopropyl, cyclobutyl, cyclopentyl, or cyclohexyl, such as cyclopropyl or cyclobutyl.

In a certain embodiment of the present disclosure, in R², R³, R⁴, R⁵, R⁷, R⁸, R⁹, R¹¹, R¹³, R¹⁴, R¹⁵, R¹⁶, R^{a}, R^{b}, R^{c}, R^{d}, and R^{e}, the halogen is independently fluorine, chlorine, bromine, or iodine, such as fluorine.

In a certain embodiment of the present disclosure, L is a single bond, O, or S, preferably a single bond or O.

In a certain embodiment of the present disclosure, p is 1.

In a certain embodiment of the present disclosure, R¹ is C₁₋₆ alkyl, C₃₋₆ cycloalkyl, - C₁₋₃ alkyl-C₃₋₆ cycloalkyl, or the following group substituted by one or more R^{a}: C₁₋₆ alkyl, C₃₋₆ cycloalkyl, or -C₁₋₃ alkyl-C₃₋₆ cycloalkyl.

In a certain embodiment of the present disclosure, each R^{a} is independently halogen, such as fluorine.

In a certain embodiment of the present disclosure, R², R³, R⁴, R⁵, R⁶, and R⁸ are hydrogen.

In a certain embodiment of the present disclosure, R² is hydrogen or OH. In a certain embodiment of the present disclosure, R³, R⁴, R⁵, R⁶, and R⁸ are hydrogen.

In a certain embodiment of the present disclosure, R⁷ is C₁₋₆ alkyl, such as methyl.

In a certain embodiment of the present disclosure, X is -C(=O)-, -CFR¹¹-, -NR¹²-, -S-, or -S(=O)₂-, preferably -C(=O)-, -NR¹²-, or -S(=O)₂-.

In a certain embodiment of the present disclosure, R⁹ is C₁₋₆ alkoxy, such as methoxy.

In a certain embodiment of the present disclosure, R¹¹ is H or halogen.

In a certain embodiment of the present disclosure, R¹² is hydrogen or C₁₋₆ alkyl, preferably hydrogen.

In a certain embodiment of the present disclosure, m is 0 or 1, such as 0.

In a certain embodiment of the present disclosure, n is 0.

In a certain embodiment of the present disclosure, L is a single bond, 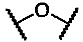 (such as 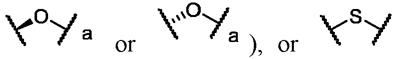 (such as 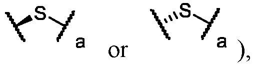 wherein a-terminus is connected to R¹; preferably, a single bond or 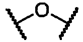 (such as 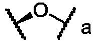 or 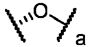 ).

In a certain embodiment of the present disclosure, R¹ is 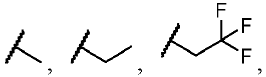 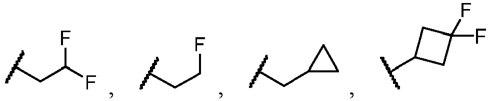 (such as 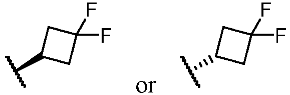 ), 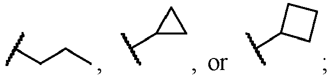 preferably 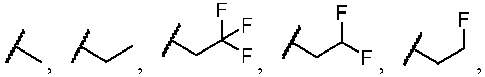 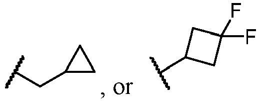 (such as 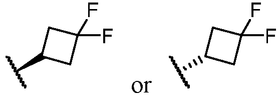 ).

In a certain embodiment of the present disclosure, R¹ is 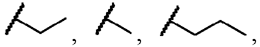 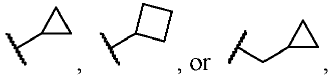 preferably

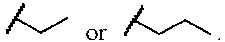

In a certain embodiment of the present disclosure, -L-R¹ is 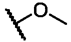 (such as 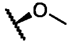 or 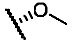 ), 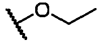 (such as 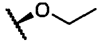 or 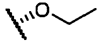 ), 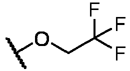 (such as 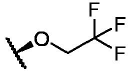 or 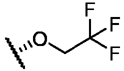 ), 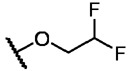 (such as 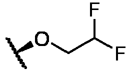 or 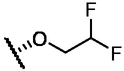 ), 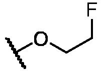 (such as 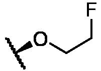 or 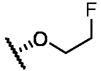 ), 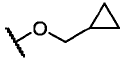 (such as 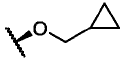 or 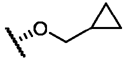 ), 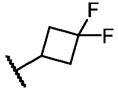 (such as 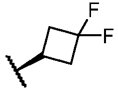 or 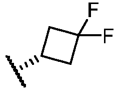 ), 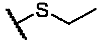 (such as 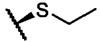 or 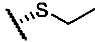 ), 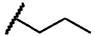 (such as 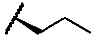 or 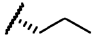 ), 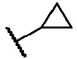 (such as 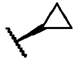 or 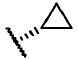 ), 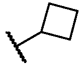 (such as 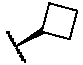 or 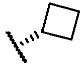 ), or 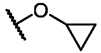 (such as 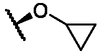 or 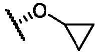 ), preferably 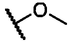 (such as 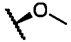 or 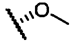 ), 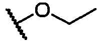 (such as 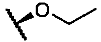 or 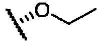 ), 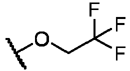 (such as 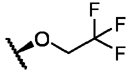 or 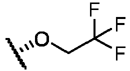 ), 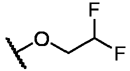 (such as 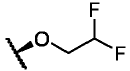 or 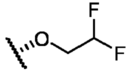 ), 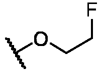 (such as 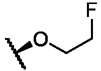 or 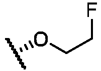 ), 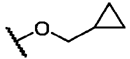 (such as 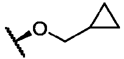 or 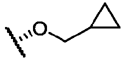 ), 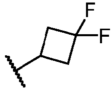 (such as 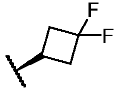 or 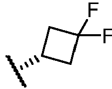 ), or 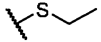 (such as 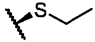 or

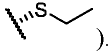

In a certain embodiment of the present disclosure, -L-R¹ is 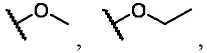 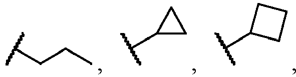 or 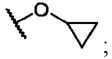 preferably

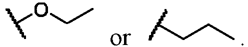

In a certain embodiment of the present disclosure, X is 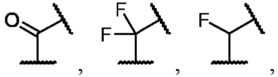 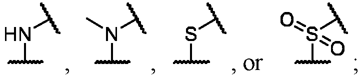 preferably 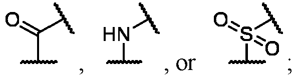 more preferably

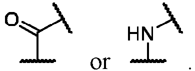

In a certain embodiment of the present disclosure, 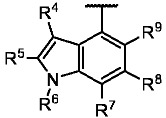 is

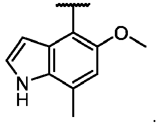

In a certain embodiment of the present disclosure, L is a single bond, O, or S, preferably a single bond or O;
p is 1;
R¹ is C₁₋₆ alkyl, C₃₋₆ cycloalkyl, -C₁₋₃ alkyl-C₃₋₆ cycloalkyl, or the following group substituted by one or more R^{a}: C₁₋₆ alkyl, C₃₋₆ cycloalkyl, or -C₁₋₃ alkyl-C₃₋₆ cycloalkyl;
each R^{a} is independently halogen, such as fluorine;
R² is OH;
R³, R⁴, R⁵, R⁶, and R⁸ are hydrogen;
R⁷ is C₁₋₆ alkyl, such as methyl;
X is -C(=O)-, -CFR¹¹-, -NR¹²-, -S-, or -S(=O)₂-, preferably -C(=O)-, -NR¹²-, or - S(=O)₂-;
R⁹ is C₁₋₆ alkoxy, such as methoxy;
R¹¹ is H or halogen;
R¹² is hydrogen or C₁₋₆ alkyl, preferably hydrogen;
m is 0 or 1, preferably 0;
n is 0.

In a certain embodiment of the present disclosure, the compound of formula (I) has a structure as shown in formula (I)-1, formula (I)-2, formula (I)-3, or formula (I)-4:

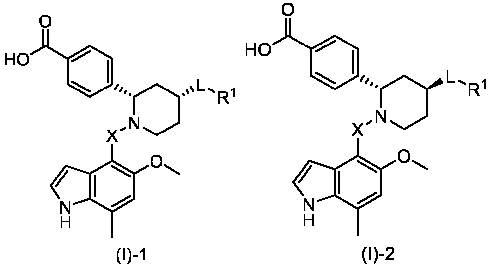

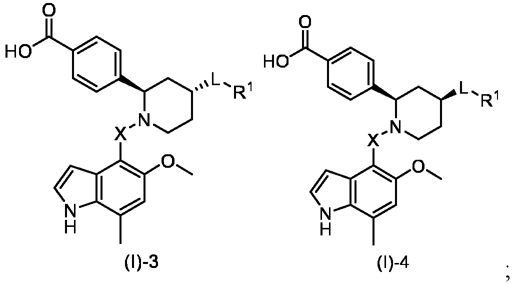

Preferably, the formula (I) is as shown in formula (I)-1 or formula (I)-2; more preferably, the formula (I) is as shown in formula (I)-2.

In a certain embodiment of the present disclosure, the compound of formula (I) is selected from any one of the following compounds:

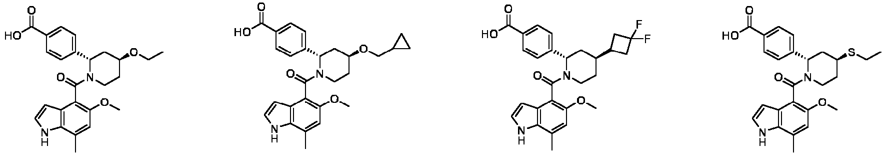

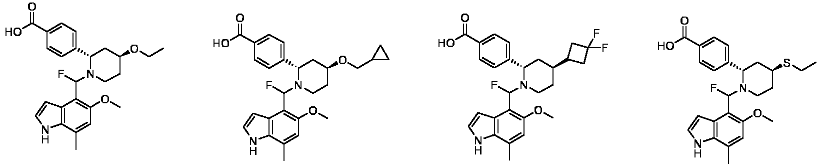

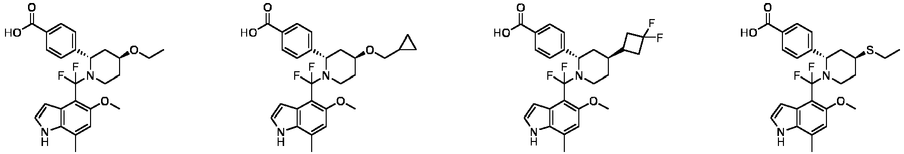

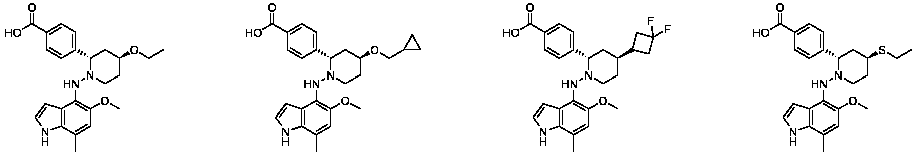

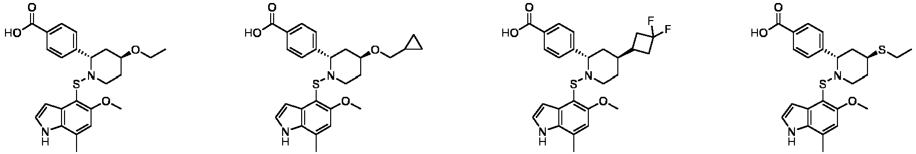

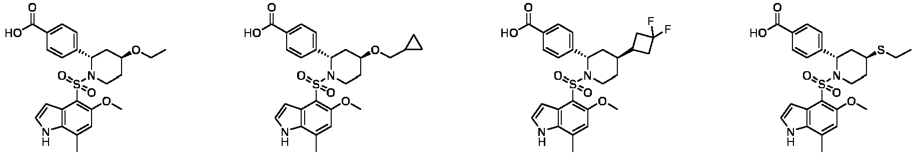

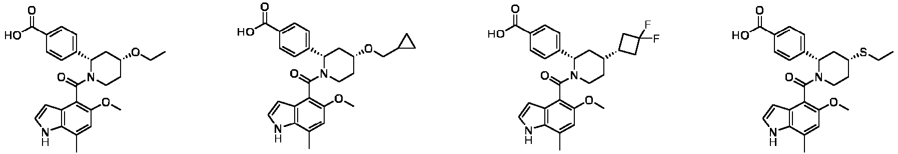

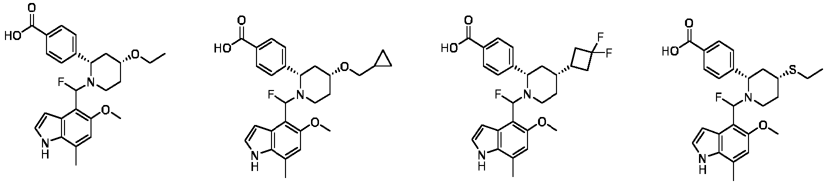

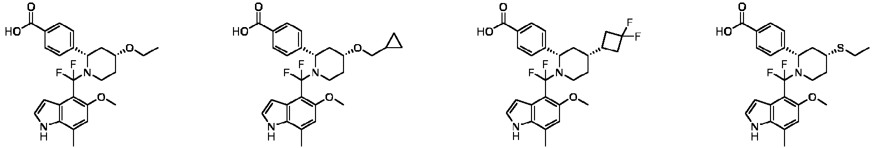

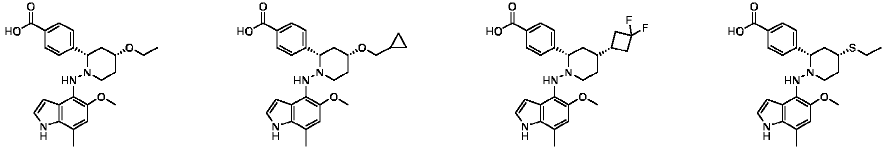

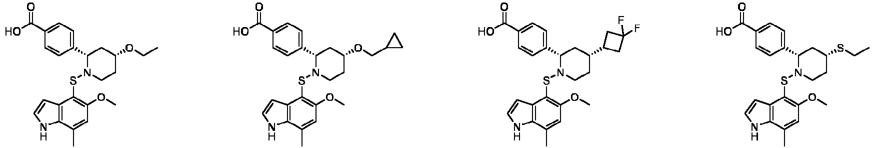

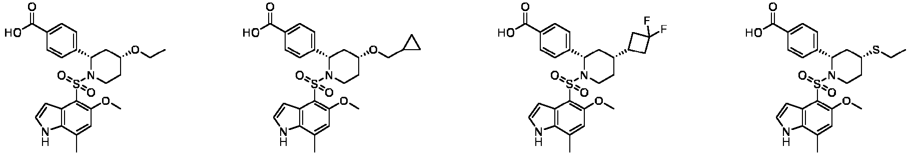

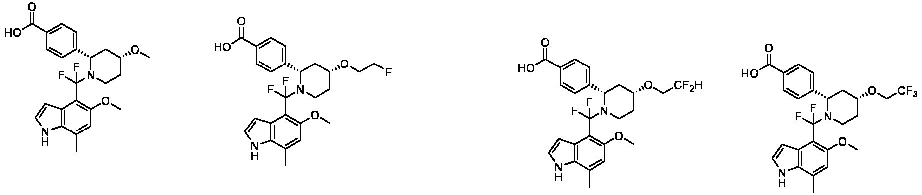

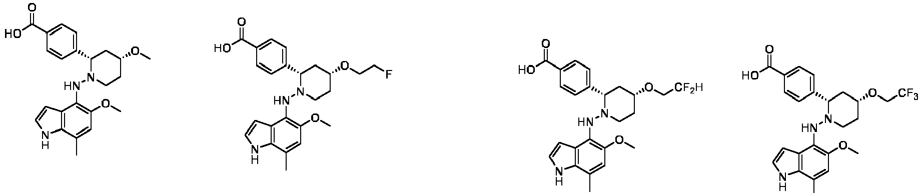

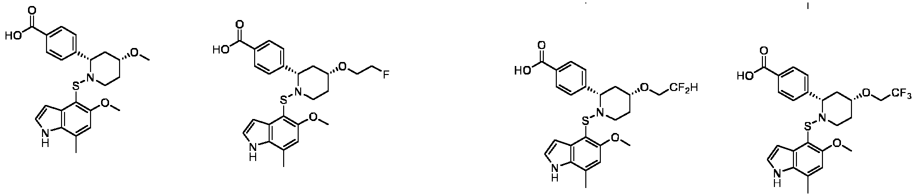

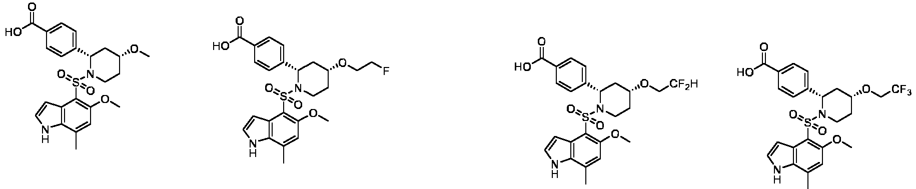

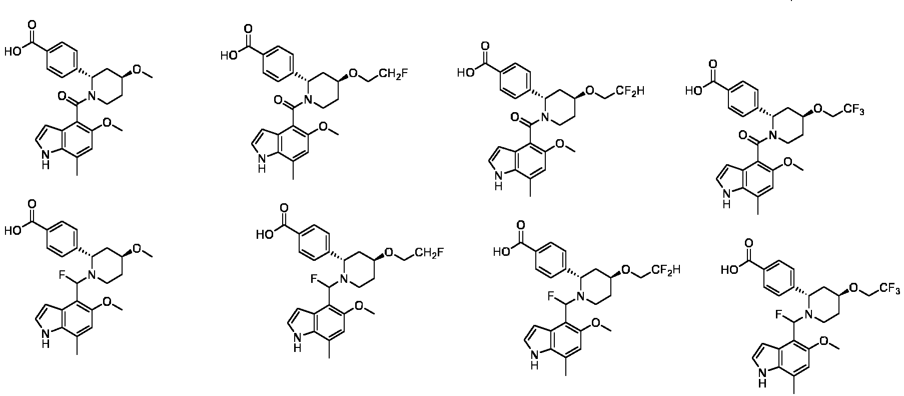

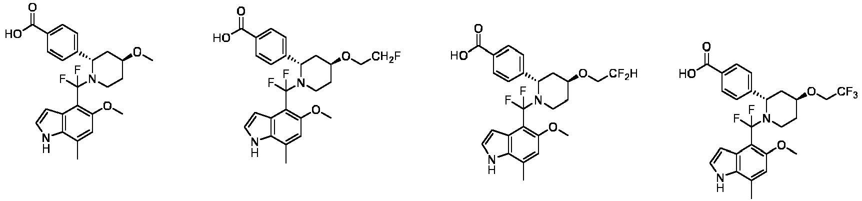

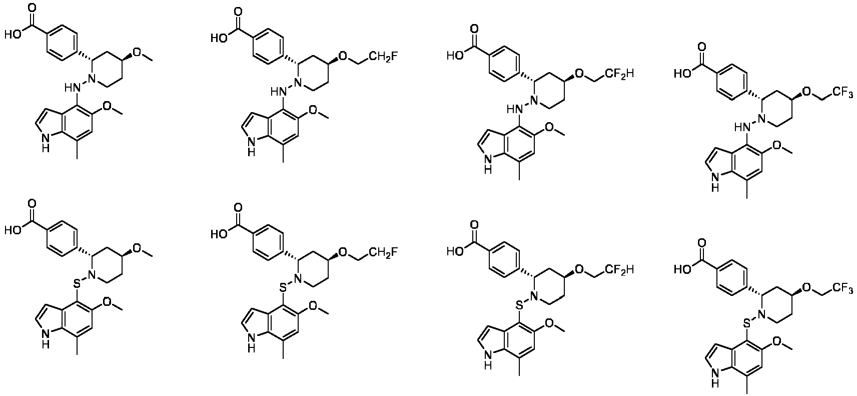

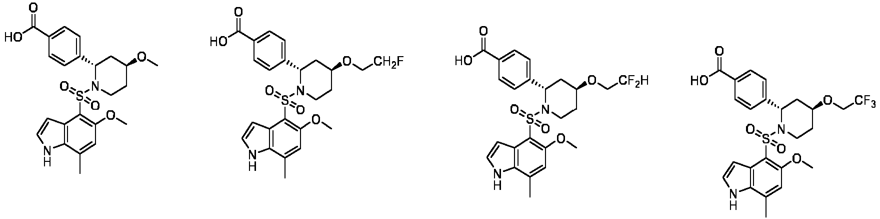

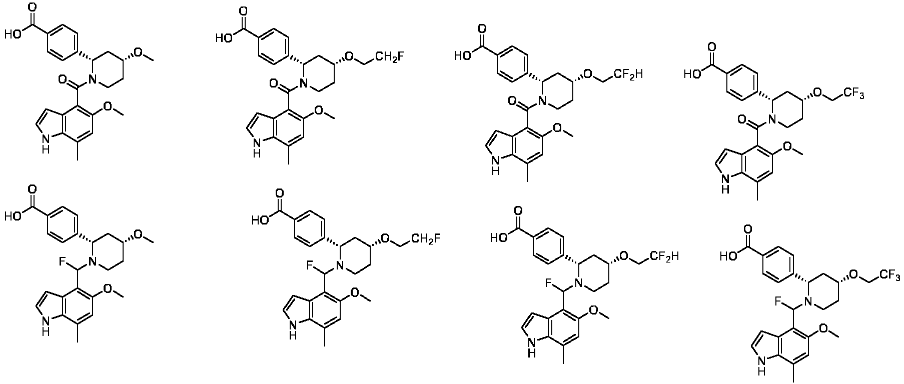

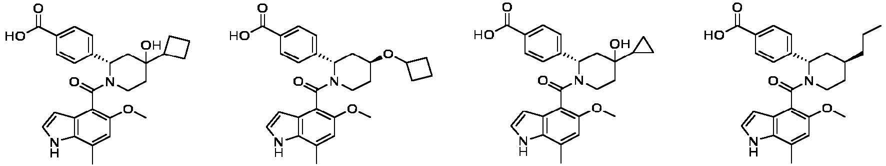

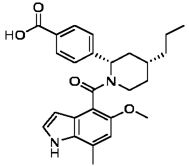

The present disclosure also provides a pharmaceutical composition, which comprises:
(1) the compound of formula (I) as described in any one of the preceding embodiments, the stereoisomer thereof, the tautomer thereof, the isotopically labeled compound thereof, or the pharmaceutically acceptable salt of any one of the foregoing (referring to the foregoing compound of formula (I), the stereoisomer thereof, the tautomer thereof, or the isotopically labeled compound thereof): and
(2) a pharmaceutically acceptable carrier.

The present disclosure also provides a use of the compound of formula (I) as described in any one of the preceding embodiments, the stereoisomer thereof, the tautomer thereof, the isotopically labeled compound thereof, or the pharmaceutically acceptable salt of any one of the foregoing (referring to the foregoing compound of formula (I), the stereoisomer thereof, the tautomer thereof, or the isotopically labeled compound thereof), or the pharmaceutical composition as described in any one of the preceding embodiments, in the manufacture of a medicament for treating and/or preventing a complement factor B-mediated disease.

The present disclosure also provides a method for treating and/or preventing a complement factor B-mediated disease, comprising: administering to a subject in need thereof a therapeutically effective amount of substance X, wherein the substance X is the compound of formula (I) as described in any one of the preceding embodiments, the stereoisomer thereof, the tautomer thereof, the isotopically labeled compound thereof, or the pharmaceutically acceptable salt of any one of the foregoing (referring to the foregoing compound of formula (I), the stereoisomer thereof, the tautomer thereof, or the isotopically labeled compound thereof), or the pharmaceutical composition as described in any one of the preceding embodiments.

Preferably, the complement factor B-mediated disease is a hematologic disease, an autoimmune disease, an inflammatory disease, a neurological disorder, or a disease associated with abnormal complement system function, etc.

Further, the complement factor B-mediated disease includes, but is not limited to, paroxysmal nocturnal hemoglobinuria, C3 glomerulopathy, macular degeneration, age-related macular degeneration, etc.

The present disclosure also provides a use of the compound of formula (I) as described in any one of the preceding embodiments, the stereoisomer thereof, the tautomer thereof, the isotopically labeled compound thereof, or the pharmaceutically acceptable salt of any one of the foregoing (referring to the foregoing compound of formula (I), the stereoisomer thereof, the tautomer thereof, or the isotopically labeled compound thereof), or the pharmaceutical composition as described in any one of the preceding embodiments, in the manufacture of a complement factor B inhibitor.

In the use, the complement factor B inhibitor can be used in mammalian organisms *in vivo;* it can also be used *in vitro,* mainly for experimental purposes, such as providing comparison as a standard sample or a control sample, or making a kit according to the conventional method in the art to provide rapid detection for the inhibitory effect of complement factor B.

The present disclosure further provides a compound of formula (II)-1, formula (II)-2, or formula (II)-3, 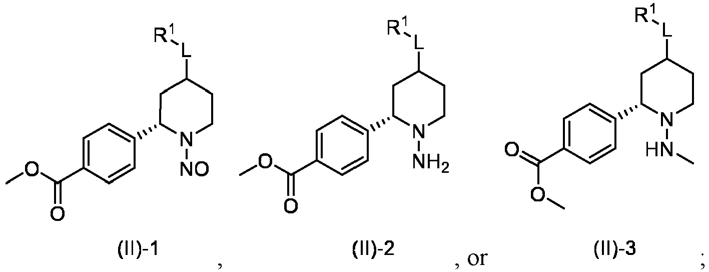 wherein L and R¹ are as defined in any one of the preceding embodiments.

In a certain embodiment of the present disclosure, the compound of formula (II)-1 is selected from any one of the following compounds:

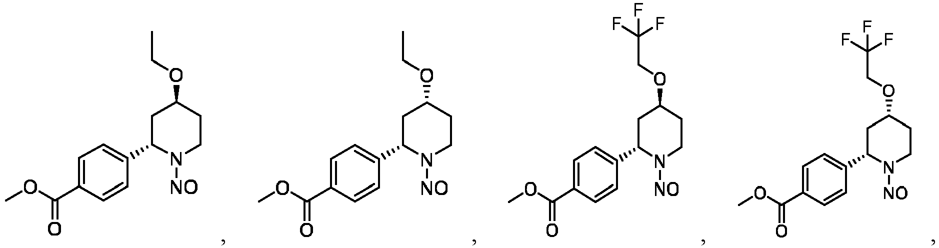

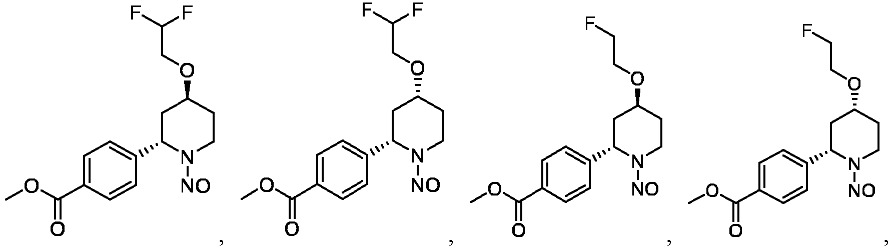

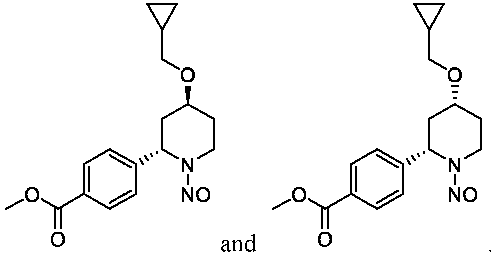

In a certain embodiment of the present disclosure, the compound of formula (II)-2 is selected from any one of the following compounds:

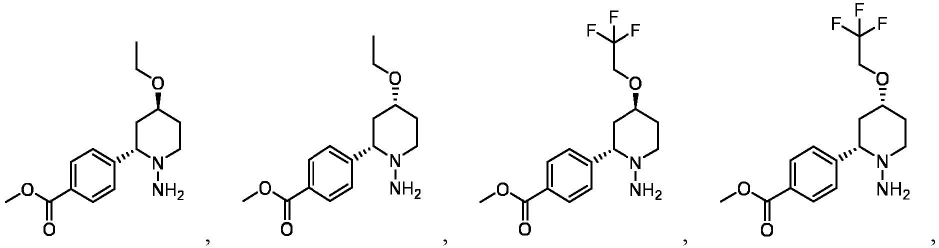

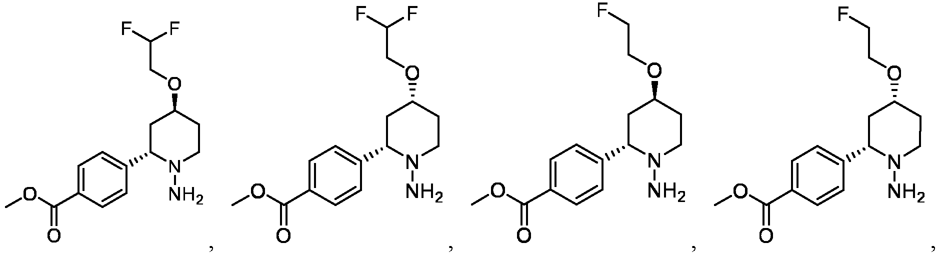

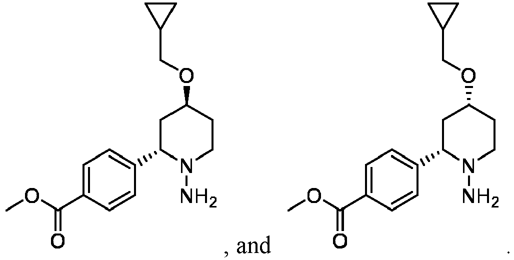

In a certain embodiment of the present disclosure, the compound of formula (II)-3 is selected from any one of the following compounds:

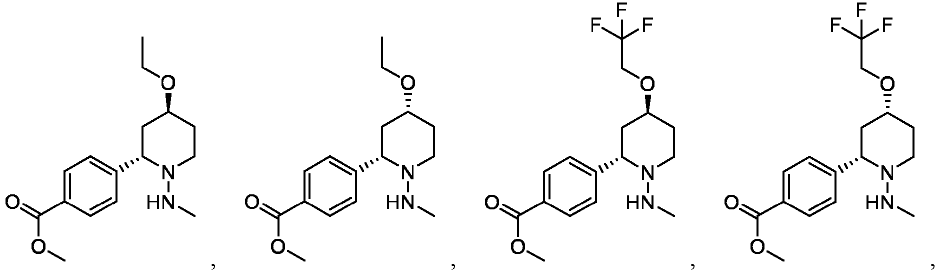

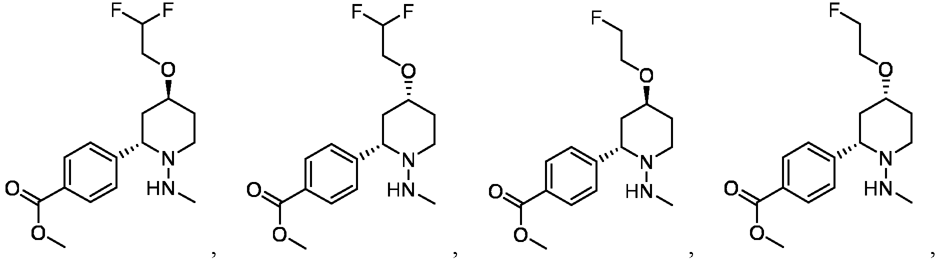

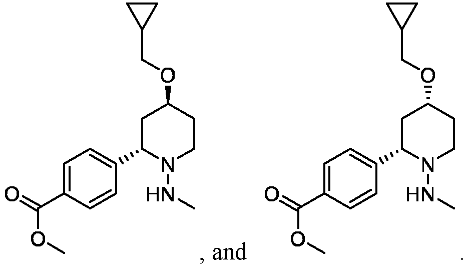

The present disclosure also provides a compound selected from any one of the following compounds:

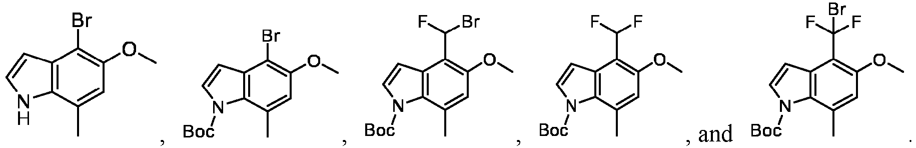

Unless otherwise specified, all technical and scientific terms used herein shall have the standard meanings as understood in the field to which the claimed subject matter pertains. In case of multiple definitions for a term, the definition herein shall prevail.

It should be understood that the singular forms such as "a" or "an" used in the present disclosure include plural references unless otherwise specified. Furthermore, the term "comprising" is an open-ended limitation rather than a closed one, meaning it encompasses the content specified by the present disclosure but does not exclude other aspects.

Unless otherwise specified, the present disclosure employs conventional methods such as mass spectrometry and elemental analysis, with all steps and conditions referenced to standard operating procedures and conditions in the field.

Unless otherwise specified, the present disclosure employs standard nomenclature and standard laboratory procedures and techniques in analytical chemistry, organic synthetic chemistry, and optics. In certain cases, standard techniques are employed for chemical synthesis, chemical analysis, and performance testing of luminescent devices.

Additionally, it should be noted that unless otherwise expressly stated, the expression "...be independently" as used in the present disclosure should be interpreted broadly, meaning that the described individuals are mutually independent and can each independently represent the same or different specific groups. In more detail, the expression "... be independently" can mean that the specific options represented by the same symbol in different groups do not affect each other, or it can indicate that within the same group, the specific options represented by the same symbol do not affect each other.

The term "alkyl" refers to a straight or branched, saturated monovalent hydrocarbon group with a specified number of carbon atoms (e.g., C₁₋₆). Alkyl groups include, but are not limited to: methyl, ethyl, n-propyl, isopropyl, n-butyl, isobutyl, sec-butyl, *tert-butyl,* n-pentyl, n-hexyl, etc.

The term "cycloalkyl" refers to a monocyclic, saturated, monovalent hydrocarbon group with a specified number of carbon atoms (e.g., C₃₋₆). Cycloalkyl groups include but are not limited to: 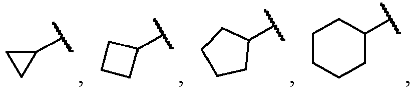 etc.

The" 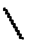 " at the end of a structural moiety indicates that the structural moiety connects to the rest of the molecule through this site. For example, 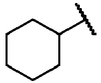 refers to cyclohexyl.

The "-" at the end of a group indicates that the group is connected to the rest of the molecule through this site. For example, CH₃-C(=O)- refers to refers to acetyl.

The term "stereoisomer" refers to either a cis-trans isomer or an optical isomer. Cis-trans isomers are isomers caused by the inability of the double bonds or single bonds of ring-forming carbon atoms to rotate freely, while optical isomers are stereoisomers that exhibit different optical activities due to the absence of antisymmetry in the molecular.

The term "isotopically labeled compound" refers to a compound in which the isotopic abundance of one or more atoms differs from their natural abundance. For example, one or more atoms in a compound are replaced by atoms with a lower mass number than is found in nature-a hydrogen atom in a compound is replaced by deuterium.

The term "pharmaceutically acceptable" refers to being relatively non-toxic, safe, and suitable for patient use.

The term "pharmaceutically acceptable salt" refers to a salt obtained by reacting a compound with a pharmaceutically acceptable acid or base. When the compound contains a relatively acidic functional group, a base addition salt can be obtained by bringing the compound into contact with a sufficient amount of the pharmaceutically acceptable base in a suitable inert solvent. When the compound contains a relatively basic functional group, an acid addition salt can be obtained by bringing the compound into contact with a sufficient amount of pharmaceutically acceptable acid in a suitable inert solvent. Specific reference can be made to Handbook of Pharmaceutical Salts. Properties, Selection, and Use (P. Heinrich Stahl, Camille G. Wermuth, 2011, 2nd Revised Edition).

The term "therapeutically effective amount" refers to an amount administered to a patient that is sufficient to effectively treat the disease. The therapeutically effective amount will vary depending on the type of compound, the type of the disease, the severity of the disease, the age of the patient, and other factors, but can be adjusted as deemed appropriate by those skilled in the art.

The term "treating" refers to eliminating the cause or alleviating the symptoms.

The term "preventing" refers to reducing the risk of developing a disease.

On the basis of not violating the common sense in the field, the preferred conditions above can be arbitrarily combined to obtain the preferred examples of the present disclosure.

The reagents and raw materials used in the present disclosure are commercially available.

The positive and progressive effects of the present disclosure lie in that the compounds of the present disclosure exhibit inhibitory activity against complement factor B, demonstrating excellent affinity activity with complement factor B, hemolytic activity in the alternative complement pathway, and inhibitory activity in the alternative complement pathway (in the complement hemolytic activity assay, the IC₅₀ values for complement factor B inhibitory activity are all < 5 µM, with the majority IC₅₀ < 1.5 µM and some IC₅₀ < 1 µM).

### DETAILED DESCRIPTION OF THE PREFERRED EMBODIMENT

The present disclosure is further described below by the way of examples, but the present disclosure is not thereby limited to the scope of the described examples. The experimental methods for which the specific conditions are not specified in the following examples are selected according to the conventional methods and conditions, or according to the commodity instructions.

The technical solutions of the present disclosure will be further described in detail below in conjunction with specific examples. It should be understood that the following examples are only used to illustrate and explain the present disclosure, and should not be construed as a limitation on the scope of protection of the present disclosure. All techniques realized based on the above contents of the present disclosure are within the scope of protection intended by the present disclosure.

Unless otherwise stated, the raw materials and reagents used in the following examples are commercially available or can be prepared by known methods.

The structures of the compounds are determined by nuclear magnetic resonance (NMR) or/and mass spectrometry (MS). NMR shift (δ) is given in units of 10 (ppm). The determination of NMR is performed using a Bruker ASCEND-400Hz or 600Hz spectrometer, with deuterated dimethyl sulfoxide (DMSO-d₆), deuterated chloroform (CDCl₃), or deuterated methanol (CD₃OD) as solvents, and tetramethylsilane (TMS) as the internal standard.

The determination of MS is performed using Waters2695, Waters2988, and WatersQDa liquid chromatography-mass spectrometers.

The determination of HPLC is performed using Waters2695 Waters2988 (Sunfire^{™}).

Unless otherwise specified, all reactions are conducted under an argon or nitrogen atmosphere. An argon or nitrogen atmosphere refers to connecting the reaction flask to an argon or nitrogen balloon with a volume of approximately 1 liter. A hydrogen atmosphere refers to connecting the reaction flask to a hydrogen balloon with a volume of approximately 1 liter.

Unless otherwise specified, the reaction temperature is room temperature, ranging from 20 to 30°C.

Unless otherwise specified, aqueous hydrochloric acid refers to an aqueous solution of HCl with a mass concentration of 1%.

The intermediates described in the present disclosure can be synthesized using the representative reactions in Schemes 1-5; the compound of formula (I) can be synthesized using the representative reactions in Schemes 6-11, with the concentrations and ratios of the respective raw materials being conventional for such reactions in the art. 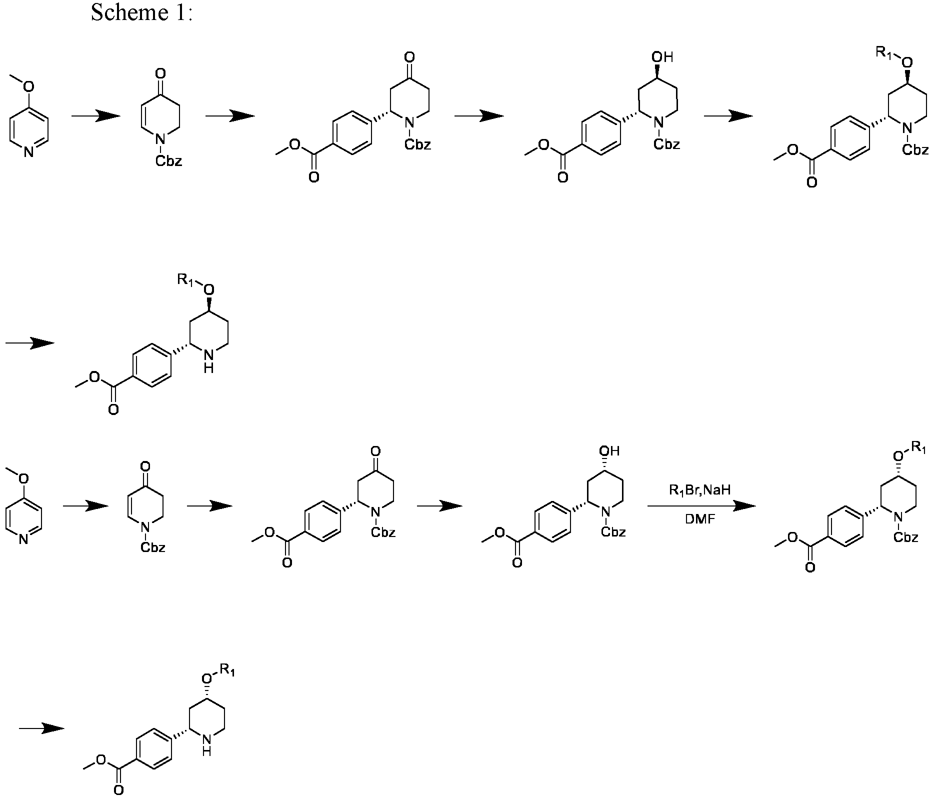 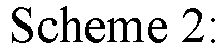 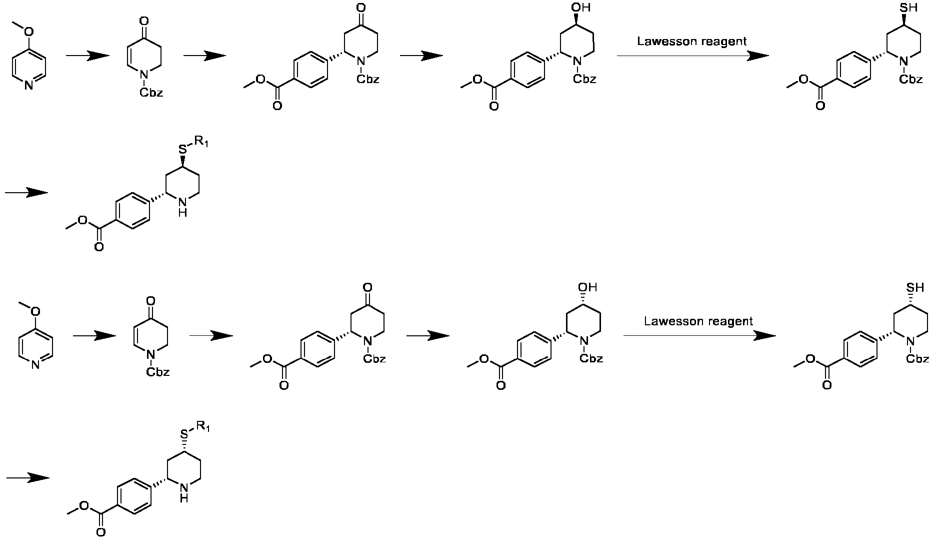 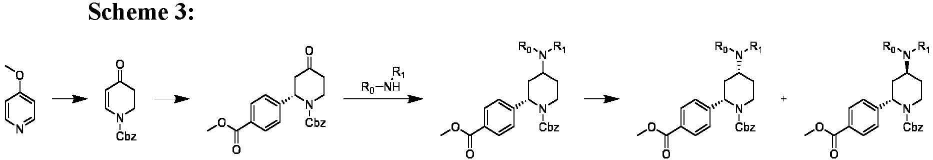 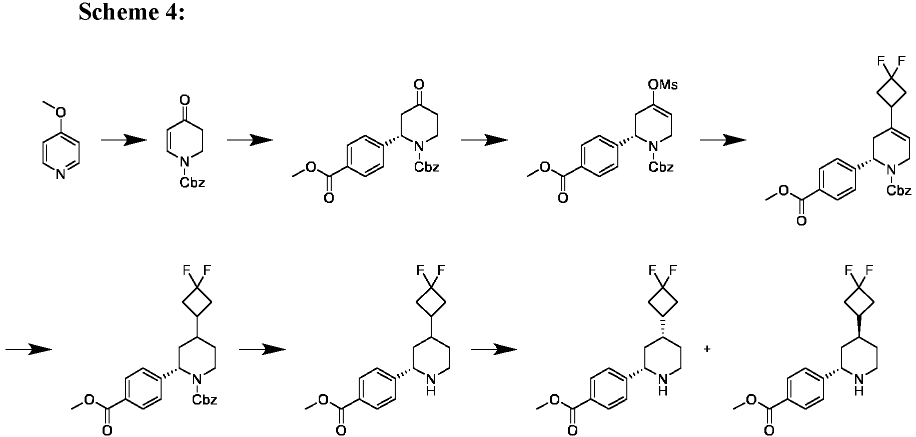 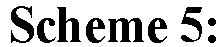 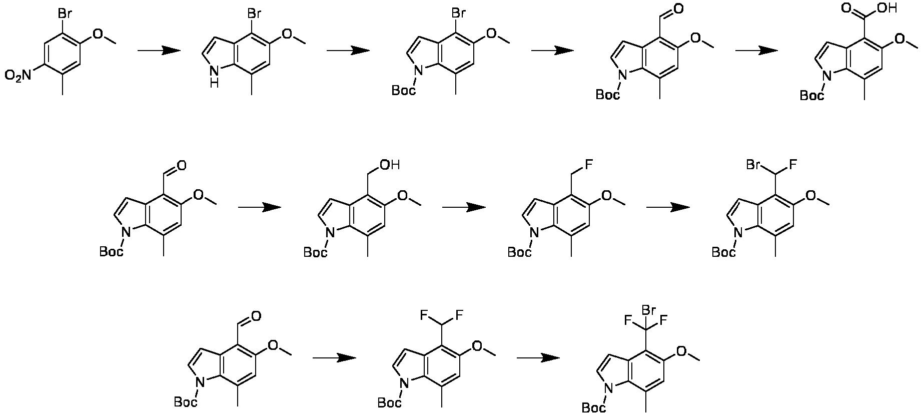 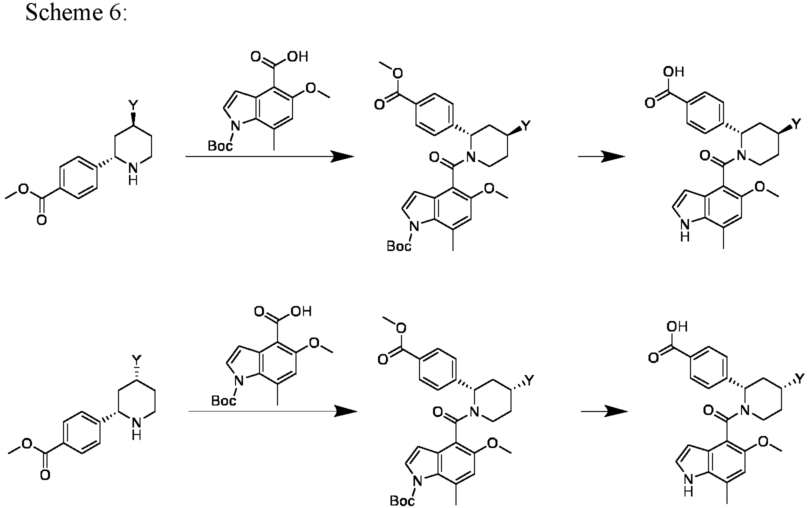 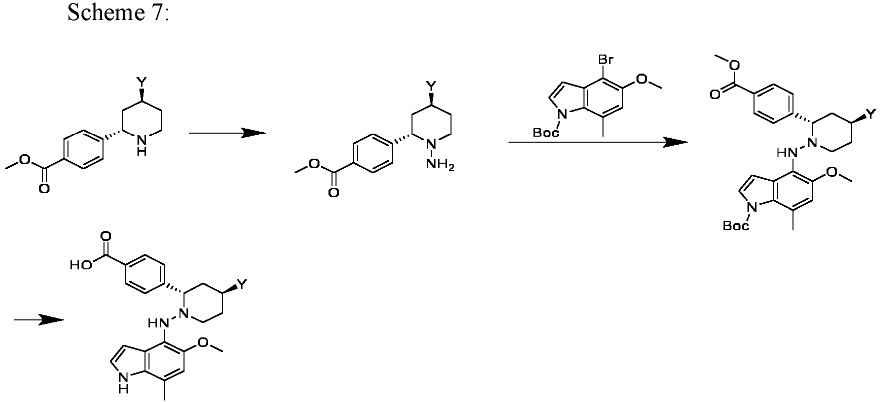 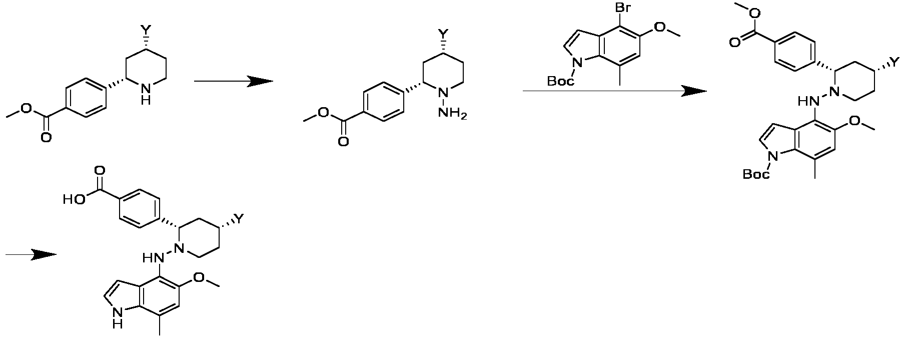 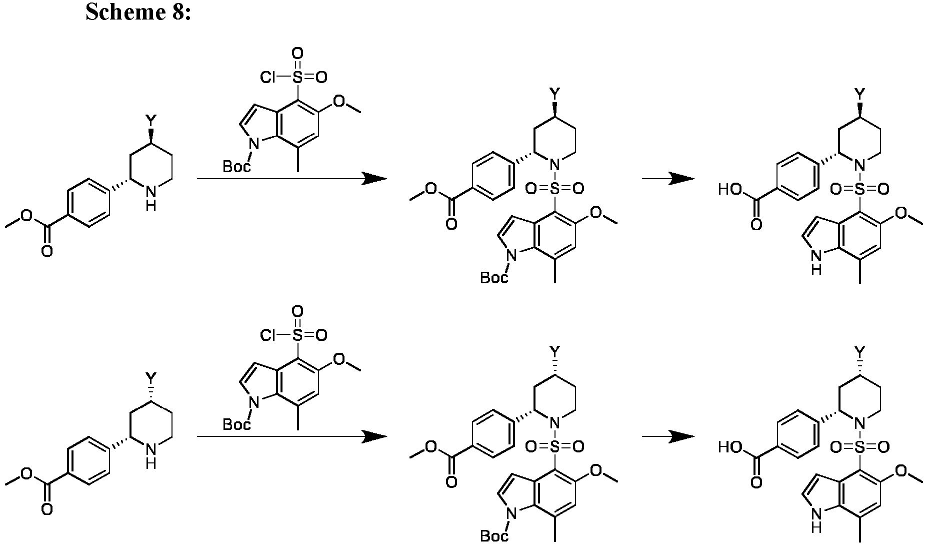 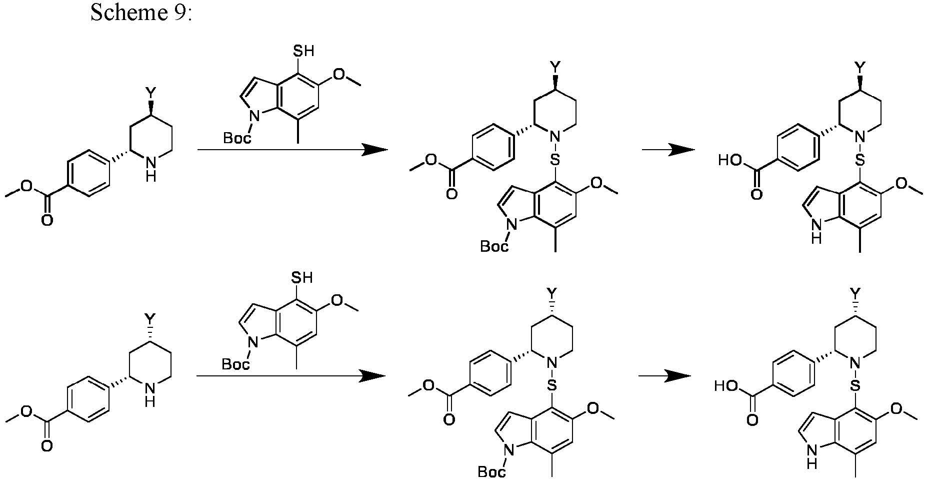 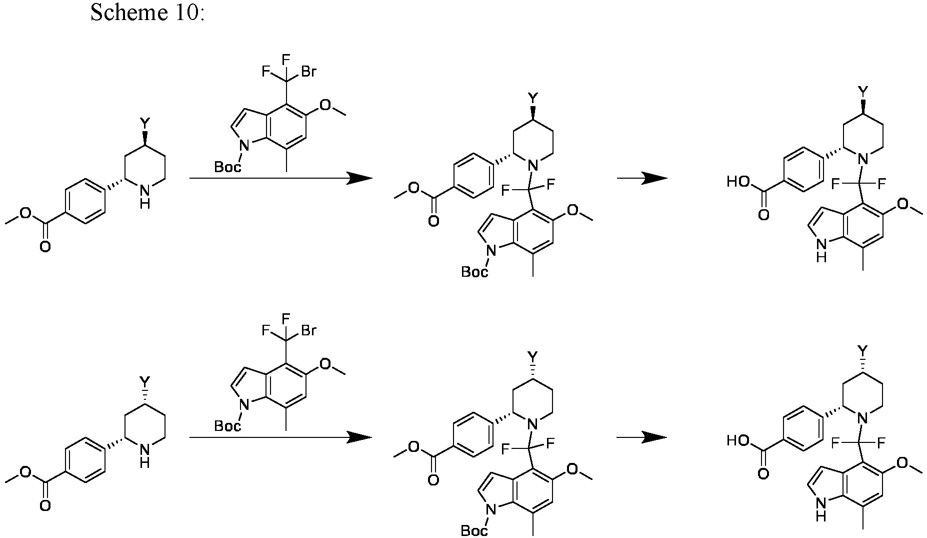 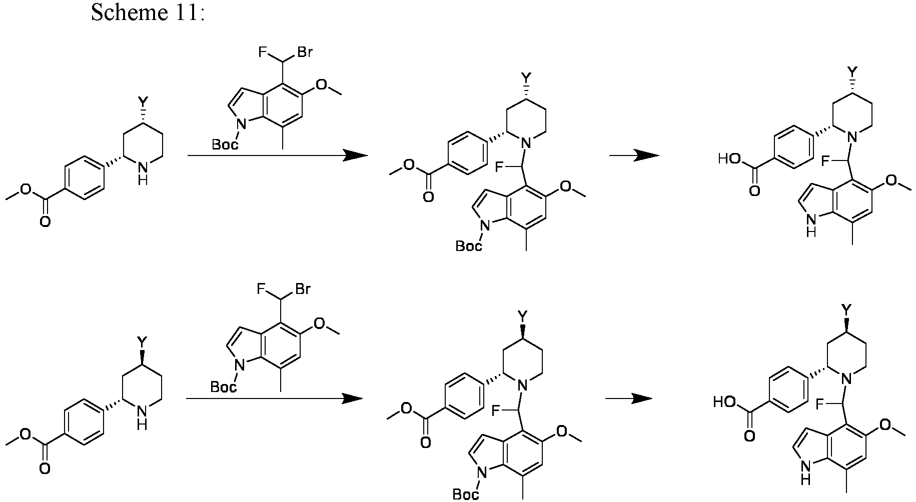 wherein in Schemes 6-11, Y is -O-R¹, -S-R¹, -NR⁰R¹, or 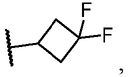 wherein R⁰ and R¹ are as defined in the present disclosure.

For RP-HPLC, the following preparation method was employed:
Stationary phase: Waters SunFire Prep C18 OBD 5 µm 30 × 100 mm
Mobile phase: Gradient, water/acetonitrile containing 1% HCl

For column chromatography, the following method was used:
Stationary phase: 300-400 mesh silica gel
Mobile phase: Gradient, petroleum ether/ethyl acetate; dichloromethane/methanol

### Example 1 Synthesis of Intermediate 1-1: Benzyl 4-oxo-3,4-dihydropyridine-1(2H)-carboxylate

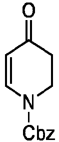

4-Methoxypyridine was placed in a 1 L four-necked flask, dissolved in 500 mL methanol, and the internal temperature was lowered to below -78°C. A solution of benzyl chloroformate (1 eq) in anhydrous tetrahydrofuran (50 mL) was added dropwise. After the dropwise addition was completed, sodium borohydride solid was added in batches 3 hours later, and the internal temperature was maintained below -78°C. After reacting for 3 hours, 200 g water was added, followed by the addition of 15.3 mL concentrated hydrochloric acid and 50 mL water, and the internal temperature was maintained between 0 and 5°C. The phases were separated, washed twice with dichloromethane and 2 N hydrochloric acid, and then washed twice with 3 wt% sodium carbonate solution. The organic phase was dried over anhydrous sodium sulfate, filtered, and concentrated under reduced pressure to afford the title compound as a white solid. MS m/z (ESI): 232.20 [M+1], ¹HNMR (400 MHz, CDCl₃) δ 7.75 (s, 1H), 7.32 - 7.26 (m, 5H), 5.24 (d, J = 6.2 Hz, 1H), 5.17 (s, 2H), 3.97 - 3.90 (m, 2H), 2.49 - 2.41 (m, 2H).

### Example 2 Synthesis of Intermediate 1-2: Benzyl (S)-2-(4-(methoxycarbonyl)phenyl)-4-oxopiperidine-1-carboxylate

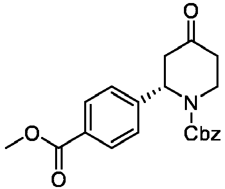

Intermediate 1-1, 4-methoxycarbonylphenylboronic acid (1 eq), (S)-xylBINAP, and Rh(Acac)(C₂H₅)₂ were placed in a 1 L reaction flask. 1,4-Dioxane (500 mL) and water (200 mL) were added, and the reaction mixture was continuously purged with nitrogen gas for 10 min. The reaction mixture was then heated to reflux. After 24 hours, the reaction mixture was cooled to room temperature, concentrated under reduced pressure, then dissolved in ethyl acetate, and subjected to suction filtration. The filtrate was subjected to column chromatography to obtain the title compound. MS m/z (ESI): 368.20 [M+1], ¹HNMR (600 MHz, CDCl₃) δ 7.92 (d, J = 8.3 Hz, 2H), 7.33 - 7.20 (m, 7H), 5.75 (s, 1H), 5.21 - 5.08 (m, 2H), 4.24 (s, 1H), 3.84 (s, 3H), 3.16 (s, 1H), 2.95 - 2.79 (m, 2H), 2.53 - 2.43 (m, 1H), 2.32 (d, J = 16.1 Hz, 1H).

### Example 3 Synthesis of Intermediate 1-3: Benzyl (2S,4S)-4-hydroxy-2-(4-(methoxycarbonyl)phenyl)piperidine-1-carboxylate

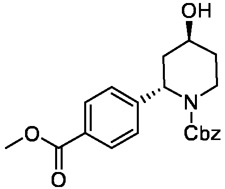

Intermediate 1-2 was placed in a 500 mL four-necked flask, and 50 mL of dichloromethane and 50 mL of carbon tetrachloride were added to the reaction flask. (R)-(+)-2-Methyl-CBS-oxazaborolidine was dissolved in 30 mL of toluene. The system was purged with nitrogen, and the internal temperature was lowered to -78°C. BH₃/THF was added dropwise while maintaining the internal temperature at -78°C until the dropwise addition was completed. After 5 hours, water was added to quench the reaction. The reaction mixture was extracted three times with ethyl acetate. The organic phases were combined and concentrated under reduced pressure to obtain the title compound as a white solid (9.5 g, yield: 95.4%). MS m/z (ESI): 380.20 [M+1], ¹HNMR (400 MHz, CDCl₃) δ 7.93 (dd, J = 8.5, 1.9 Hz, 2H), 7.32 - 7.20 (m, 7H), 5.62 (s, 1H), 5.10 (d, J = 21.8 Hz, 2H), 4.15 (dd, J = 34.6, 16.9 Hz, 1H), 3.84 (d, J = 3.2 Hz, 3H), 3.73 - 3.63 (m, 1H), 2.77 (td, J = 13.7, 2.9 Hz, 1H), 2.57 (d, J = 13.3 Hz, 1H), 1.86 - 1.73 (m, 2H), 1.42 (dd, J = 11.8, 7.1 Hz, 1H).

### Example 4 Synthesis of Intermediate 1-4: Benzyl (2S,4S)-4-ethoxy-2-(4-(methoxycarbonyl)phenyl)piperidine-1-carboxylate

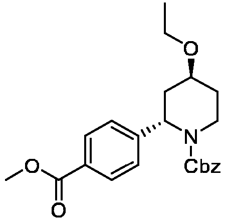

Sodium hydride was placed in a 50 mL three-necked flask, and 10 mL of anhydrous N,N-dimethylformamide was added. The flask was placed in an ice bath. After 10 min, intermediate 1-3 was dissolved in 10 mL of anhydrous N,N-dimethylformamide and added dropwise to the reaction flask. After 30 min, iodoethane (1 eq) was added dropwise. After the dropwise addition was completed, water and ethyl acetate were added 30 min later. The organic phase was concentrated under reduced pressure to obtain the title compound as a colorless oil. MS m/z (ESI): 398.20 [M+1].

### Example 5 Synthesis of Intermediate 1-5: Methyl 4-((2S,4S)-4-ethoxypiperidin-2-yl)benzoate hydrochloride

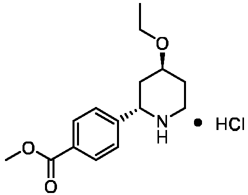

Intermediate 1-4 was placed in a single-necked reaction flask, followed by the addition of 10 mL of methanol, then 2 N hydrochloric acid, and subsequently 10% palladium on carbon. The system was purged with hydrogen three times, and the reaction was carried out overnight. After the reaction was completed, the mixture was subjected to suction filtration and then concentrated under reduced pressure to obtain a white solid. The solid was slurried with methyl *tert-butyl* ether for impurity removal, then filtered to obtain the title compound as a white solid powder. MS m/z (ESI): 264.20 [M+1], ¹HNMR (600 MHz, CDCl₃) δ 9.74 (s, 2H), 8.00 (d, J = 7.6 Hz, 2H), 7.69 (d, J = 7.8 Hz, 2H), 4.41 (s, 1H), 3.92 (s, 3H), 3.80 (s, 1H), 3.52 - 3.40 (m, 2H), 3.16 (s, 1H), 2.90 (s, 1H), 2.28 - 2.06 (m, 3H), 1.92 (d, J = 14.1 Hz, 1H), 1.21 (t, J = 6.9 Hz, 3H).

### Example 6 Synthesis of Intermediate 1-6: Methyl 4-((2S,4S)-4-ethoxy-1-nitrosopiperidin-2-yl)benzoate

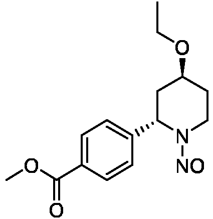

Intermediate 1-5 was placed in a 25 mL two-necked flask and dissolved in 5 mL of DCM. The reaction flask was placed in an ice bath, followed by the addition of pyridine and then nitrosonium tetrafluoroborate. After the reaction was completed, water and ethyl acetate were added. The organic phase was dried and concentrated under reduced pressure to obtain the title compound (14 mg, yield). MS m/z (ESI): 293.20 [M+1].

### Example 7 Synthesis of Intermediate 1-7: Methyl 4-((2S,4S)-1-amino-4-ethoxypiperidin-2-yl)benzoate

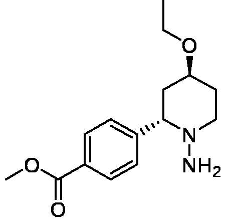

Intermediate 1-6 was placed in a reaction flask, dissolved in 5 mL of glacial acetic acid, followed by the addition of zinc powder (6.0 mg). After 5 hours, the reaction was completed. The mixture was filtered, and the filtrate was concentrated to obtain the title compound. MS m/z (ESI): 279.20 [M+1].

### Synthesis of Intermediate 1-8

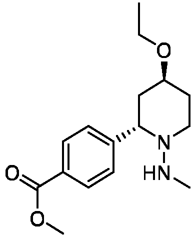

Intermediate 1-7 was placed in a reaction flask, dissolved in dichloromethane, and iodomethane was added dropwise. After the reaction was completed, saturated brine and dichloromethane were added. The organic phase was concentrated under reduced pressure and subjected to column chromatography to obtain the title compound. MS m/z (ESI): 293.20 [M+1].

### Example 8 Synthesis of Intermediate 2-1: Benzyl (2S,4R)-4-hydroxy-2-(4-(methoxycarbonyl)phenyl)piperidine-1-carboxylate

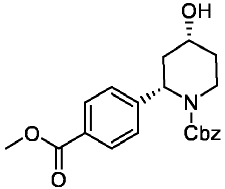

Intermediate 1-2 was placed in a 500 mL four-necked flask, and 50 mL of dichloromethane and 50 mL of carbon tetrachloride were added to the reaction flask. (S)-2-Methyl-CBS-oxazaborolidine was dissolved in 30 mL of toluene. The system was purged with nitrogen, and the internal temperature was lowered to -78°C. BH₃/THF was added dropwise while maintaining the internal temperature at -78°C until the dropwise addition was completed. After 5 hours, water was added to quench the reaction. The reaction mixture was extracted three times with ethyl acetate. The organic phases were combined and concentrated under reduced pressure to obtain the title compound as a white solid. MS m/z (ESI): 380.20 [M+1], ¹HNMR (400 MHz, CDCl₃) δ 7.93 (dd, J = 8.5, 1.9 Hz, 2H), 7.32 - 7.20 (m, 7H), 5.62 (s, 1H), 5.10 (d, J = 21.8 Hz, 2H), 4.15 (dd, J = 34.6, 16.9 Hz, 1H), 3.84 (d, J = 3.2 Hz, 3H), 3.73 - 3.63 (m, 1H), 2.77 (td, J = 13.7, 2.9 Hz, 1H), 2.57 (d, J = 13.3 Hz, 1H), 1.86 - 1.73 (m, 2H), 1.42 (dd, J = 11.8, 7.1 Hz, 1H).

### Example 9 Synthesis of Intermediate 2-2: Benzyl (2S,4R)-4-ethoxy-2-(4-(methoxycarbonyl)phenyl)piperidine-1-carboxylate

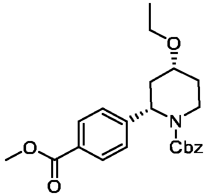

Sodium hydride was placed in a 50 mL three-necked flask, and 10 mL of anhydrous N,N-dimethylformamide was added. The flask was placed in an ice bath. After 10 min, intermediate 2-1 was dissolved in 10 mL of anhydrous N,N-dimethylformamide and added dropwise to the reaction flask. After 30 min, iodoethane was added dropwise. After the dropwise addition was completed, water and ethyl acetate were added 30 min later. The organic phase was concentrated under reduced pressure to obtain the title compound as a colorless oil. MS m/z (ESI): 398.20 [M+1]. 1HNMR (400 MHz, CDCl₃) δ 8.00 (d, J = 8.4 Hz, 2H), 7.32 (dt, J = 17.7, 7.9 Hz, 7H), 5.68 (s, 1H), 5.19 (s, 2H), 4.27 (d, J = 13.4 Hz, 1H), 3.92 (s, 3H), 3.56 - 3.42 (m, 2H), 3.40 - 3.28 (m, 1H), 2.84 (td, J = 13.6, 2.9 Hz, 1H), 2.65 (d, J = 13.4 Hz, 1H), 2.06 - 1.91 (m, 1H), 1.84 (ddd, J = 13.4, 11.4, 5.8 Hz, 1H), 1.54 - 1.40 (m, 1H), 1.18 (t, J = 7.0 Hz, 3H).

### Example 10 Synthesis of Intermediate 2-3: Methyl 4-((2S,4R)-4-ethoxypiperidin-2-yl)benzoate hydrochloride

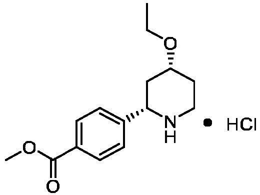

Intermediate 2-2 was placed in a single-necked reaction flask, followed by the addition of 10 mL of methanol, then 2 N hydrochloric acid, and subsequently 10% palladium on carbon. The system was purged with hydrogen three times, and the reaction was carried out overnight. After the reaction was completed, the mixture was subjected to suction filtration and then concentrated under reduced pressure to obtain a white solid. The solid was slurried with methyl *tert-butyl* ether for impurity removal, then filtered to obtain the title compound as a white solid powder. MS m/z (ESI): 264.20 [M+1], ¹HNMR (600 MHz, CDCl₃) δ 9.74 (s, 2H), 8.00 (d, J = 7.6 Hz, 2H), 7.69 (d, J = 7.8 Hz, 2H), 4.41 (s, 1H), 3.92 (s, 3H), 3.80 (s, 1H), 3.52 - 3.40 (m, 2H), 3.16 (s, 1H), 2.90 (s, 1H), 2.28 - 2.06 (m, 3H), 1.92 (d, J = 14.1 Hz, 1H), 1.21 (t, J = 6.9 Hz, 3H).

### Example 11 Synthesis of Intermediate 2-4: Methyl 4-((2S,4R)-4-ethoxy-1-nitrosopiperidin-2-yl)benzoate

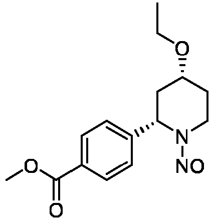

Intermediate 2-3 was placed in a 25 mL two-necked flask and dissolved in 5 mL of DCM. The reaction flask was placed in an ice bath, followed by the addition of pyridine and then nitrosonium tetrafluoroborate. After the reaction was completed, water and ethyl acetate were added. The organic phase was dried and concentrated under reduced pressure to obtain the title compound. MS m/z (ESI): 293.20 [M+1]. ¹H NMR (400 MHz, CDCl₃) δ 8.02 - 7.90 (m, 2H), 7.27 (d, *J* = 8.1 Hz, 1H), 7.04 (d, *J* = 7.9 Hz, 1H), 6.48 - 5.93 (m, 1H), 4.90 - 4.56 (m, 1H), 4.05 (q, *J* = 7.1 Hz, 1H), 3.85 (d, *J* = 4.6 Hz, 3H), 3.68 - 3.37 (m, 4H), 2.26 - 2.14 (m, 1H), 2.08 - 1.99 (m, 1H), 1.72 - 1.55 (m, 2H).

### Example 12 Synthesis of Intermediate 2-5: Methyl 4-((2S,4R)-4-ethoxy-1-nitrosopiperidin-2-yl)benzoate

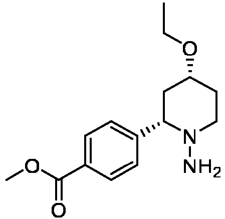

Intermediate 2-4 was placed in a reaction flask, dissolved in 5 mL of glacial acetic acid, followed by the addition of zinc powder. After 5 hours, the reaction was completed. The mixture was filtered, and the filtrate was concentrated to obtain the title compound. MS m/z (ESI): 279.20 [M+1].

### Synthesis of Intermediate 2-6

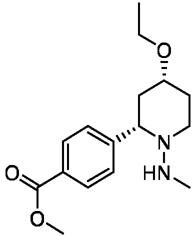

Intermediate 2-5 was placed in a reaction flask, dissolved in dichloromethane, and iodomethane was added dropwise. After the reaction was completed, saturated brine and dichloromethane were added. The organic phase was concentrated under reduced pressure and subjected to column chromatography to obtain the title compound. MS m/z (ESI): 293.20 [M+1].

### Example 13 Synthesis of Intermediate 3-1

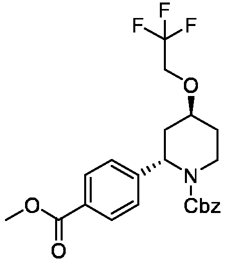

Sodium hydride was placed in a 50 mL three-necked flask, and 10 mL of anhydrous N,N-dimethylformamide was added. The flask was placed in an ice bath. After 10 min, intermediate 1-3 was dissolved in 10 mL of anhydrous N,N-dimethylformamide and added dropwise to the reaction flask. After 30 min, 2,2,2-trifluoroethyl trifluoromethanesulfonate was added dropwise. After the dropwise addition was completed, water and ethyl acetate were added 30 min later. The organic phase was concentrated under reduced pressure to obtain the title compound as a colorless oil. MS m/z (ESI): 452.20 [M+1].

### Example 14 Synthesis of Intermediate 3-2

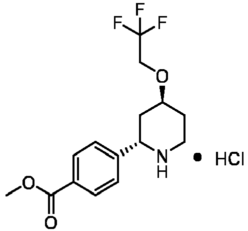

Intermediate 3-1 was placed in a single-necked reaction flask, followed by the addition of 10 mL of methanol, then 2 N hydrochloric acid, and subsequently 10% palladium on carbon. The system was purged with hydrogen three times, and the reaction was carried out overnight. After the reaction was completed, the mixture was subjected to suction filtration and then concentrated under reduced pressure to obtain a white solid. The solid was slurried with methyl *tert-butyl* ether for impurity removal, then filtered to obtain the title compound as a white solid powder. MS m/z(ESI): 318.12[M+1].

### Example 15 Synthesis of Intermediate 3-3

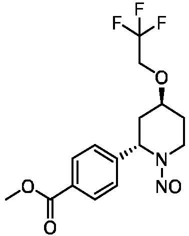

Intermediate 3-2 was placed in a 25 mL two-necked flask and dissolved in 5 mL of DCM. The reaction flask was placed in an ice bath, followed by the addition of pyridine and then nitrosonium tetrafluoroborate. After the reaction was completed, water and ethyl acetate were added. The organic phase was dried and concentrated under reduced pressure to obtain the title compound. MS m/z (ESI): 347.20 [M+1].

### Example 16 Synthesis of Intermediate 3-4

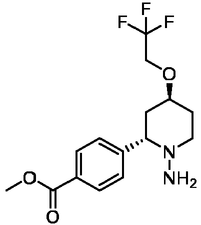

Intermediate 3-3 was placed in a reaction flask, dissolved in 5 mL of glacial acetic acid, followed by the addition of zinc powder. After 5 hours, the reaction was completed. The mixture was filtered, and the filtrate was concentrated to obtain the title compound. MS m/z (ESI): 333.20 [M+1].

### Synthesis of Intermediate 3-5

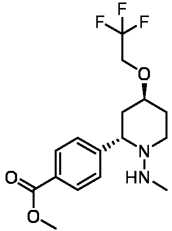

Intermediate 3-4 was placed in a reaction flask, dissolved in dichloromethane, and iodomethane was added dropwise. After the reaction was completed, saturated brine and dichloromethane were added. The organic phase was concentrated under reduced pressure and subjected to column chromatography to obtain the title compound. MS m/z (ESI): 347.16 [M+1].

### Example 17 Synthesis of Intermediate 4-1

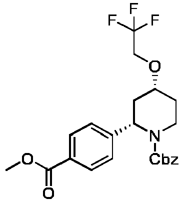

Sodium hydride was placed in a 50 mL three-necked flask, and 10 mL of anhydrous N,N-dimethylformamide was added. The flask was placed in an ice bath. After 10 min, intermediate 2-1 was dissolved in 10 mL of anhydrous N,N-dimethylformamide and added dropwise to the reaction flask. After 30 min, 2,2,2-trifluoroethyl trifluoromethanesulfonate was added dropwise. After the dropwise addition was completed, water and ethyl acetate were added 30 min later, and the organic phase was concentrated under reduced pressure to obtain the title compound as a colorless oil. MS m/z (ESI): 452.20 [M+1].

### Example 18 Synthesis of Intermediate 4-2

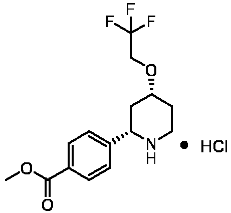

Intermediate 4-1 was placed in a single-necked reaction flask, followed by the addition of 10 mL of methanol, then 2 N hydrochloric acid, and subsequently 10% palladium on carbon. The system was purged with hydrogen three times, and the reaction was carried out overnight. After the reaction was completed, the mixture was subjected to suction filtration and then concentrated under reduced pressure to obtain a white solid. The solid was slurried with methyl *tert-butyl* ether for impurity removal, then filtered to obtain the title compound as a white solid powder. MS m/z (ESI): 318.12 [M+1].

### Example 19 Synthesis of Intermediate 4-3

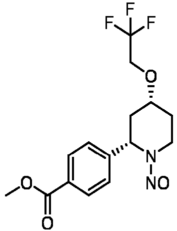

Intermediate 4-2 was placed in a 25 mL two-necked flask and dissolved in 5 mL of DCM. The reaction flask was placed in an ice bath, followed by the addition of pyridine and then nitrosonium tetrafluoroborate. After the reaction was completed, water and ethyl acetate were added. The organic phase was dried and concentrated under reduced pressure to obtain the title compound. MS m/z (ESI): 347.20 [M+1].

### Example 20 Synthesis of Intermediate 4-4

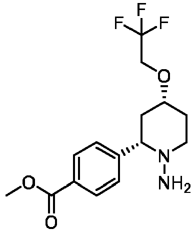

Intermediate 4-3 was placed in a reaction flask, dissolved in 5 mL of glacial acetic acid, followed by the addition of zinc powder. After 5 hours, the reaction was completed. The mixture was filtered, and the filtrate was concentrated to obtain the title compound. MS m/z (ESI): 333.20 [M+1].

### Synthesis of Intermediate 4-5

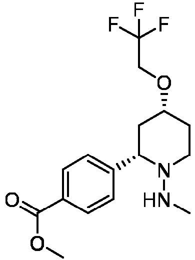

Intermediate 4-4 was placed in a reaction flask, dissolved in dichloromethane, and iodomethane was added dropwise. After the reaction was completed, saturated brine and dichloromethane were added. The organic phase was concentrated under reduced pressure and subjected to column chromatography to obtain the title compound. MS m/z (ESI): 347.20 [M+1].

### Example 21 Synthesis of Intermediate 5-1

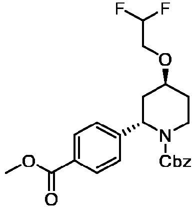

Sodium hydride was placed in a 50 mL three-necked flask, and 10 mL of anhydrous N,N-dimethylformamide was added. The flask was placed in an ice bath. After 10 min, intermediate 1-3 was dissolved in 10 mL of anhydrous N,N-dimethylformamide and added dropwise to the reaction flask. After 30 min, 2,2-difluoroethyl trifluoromethanesulfonate was added dropwise. After the dropwise addition was completed, water and ethyl acetate were added 30 min later. The organic phase was concentrated under reduced pressure to obtain the title compound as a colorless oil. MS m/z (ESI): 434.20 [M+1].

### Example 22 Synthesis of Intermediate 5-2

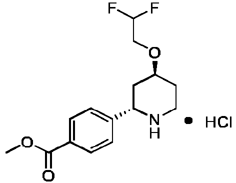

Intermediate 5-1 was placed in a single-necked reaction flask, followed by the addition of 10 mL of methanol, then 2 N hydrochloric acid, and subsequently 10% palladium on carbon. The system was purged with hydrogen three times, and the reaction was carried out overnight. After the reaction was completed, the mixture was subjected to suction filtration and then concentrated under reduced pressure to obtain a white solid. The solid was slurried with methyl *tert-butyl* ether for impurity removal, then filtered to obtain the title compound as a white solid powder. MS m/z (ESI): 300.12 [M+1].

### Example 23 Synthesis of Intermediate 5-3

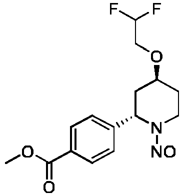

Intermediate 5-2 was placed in a 25 mL two-necked flask and dissolved in 5 mL of DCM. The reaction flask was placed in an ice bath, followed by the addition of pyridine and then nitrosonium tetrafluoroborate. After the reaction was completed, water and ethyl acetate were added. The organic phase was dried and concentrated under reduced pressure to obtain the title compound. MS m/z (ESI): 329.20 [M+1].

### Example 24 Synthesis of Intermediate 5-4

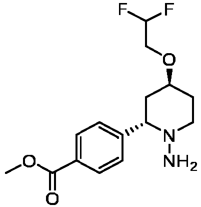

Intermediate 5-3 was placed in a reaction flask, dissolved in 5 mL of glacial acetic acid, followed by the addition of zinc powder. After 5 hours, the reaction was completed. The mixture was filtered, and the filtrate was concentrated to obtain the title compound. MS m/z (ESI): 315.20 [M+1].

### Synthesis of Intermediate 5-5

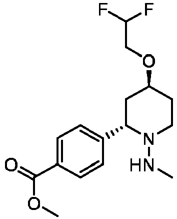

Intermediate 5-4 was placed in a reaction flask, dissolved in dichloromethane, and iodomethane was added dropwise. After the reaction was completed, saturated brine and dichloromethane were added. The organic phase was concentrated under reduced pressure and subjected to column chromatography to obtain the title compound. MS m/z (ESI): 329.20 [M+1].

### Example 25 Synthesis of Intermediate 6-1

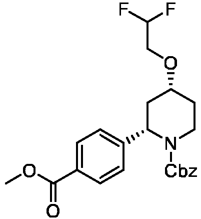

Sodium hydride was placed in a 50 mL three-necked flask, and 10 mL of anhydrous N,N-dimethylformamide was added. The flask was placed in an ice bath. After 10 min, intermediate 2-1 was dissolved in 10 mL of anhydrous N,N-dimethylformamide and added dropwise to the reaction flask. After 30 min, 2,2-difluoroethyl trifluoromethanesulfonate was added dropwise. After the dropwise addition was completed, water and ethyl acetate were added 30 min later. The organic phase was concentrated under reduced pressure to obtain the title compound as a colorless oil. MS m/z (ESI): 434.20 [M+1].

### Example 26 Synthesis of Intermediate 6-2

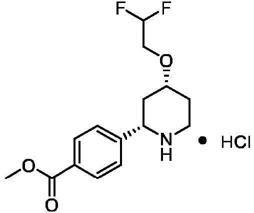

Intermediate 6-1 was placed in a single-necked reaction flask, followed by the addition of 10 mL of methanol, then 2 N hydrochloric acid, and subsequently 10% palladium on carbon. The system was purged with hydrogen three times, and the reaction was carried out overnight. After the reaction was completed, the mixture was subjected to suction filtration and then concentrated under reduced pressure to obtain a white solid. The solid was slurried with methyl *tert-butyl* ether for impurity removal, then filtered to obtain the title compound as a white solid powder. MS m/z (ESI): 300.12 [M+1].

### Example 27 Synthesis of Intermediate 6-3

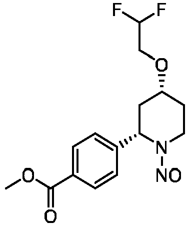

Intermediate 6-2 was placed in a 25 mL two-necked flask and dissolved in 5 mL of DCM. The reaction flask was placed in an ice bath, followed by the addition of pyridine and then nitrosonium tetrafluoroborate. After the reaction was completed, water and ethyl acetate were added. The organic phase was dried and concentrated under reduced pressure to obtain the title compound. MS m/z (ESI): 329.20 [M+1].

### Example 28 Intermediate 6-4

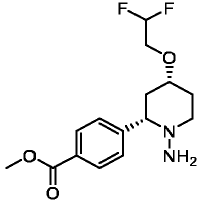

Intermediate 6-3 was placed in a reaction flask, dissolved in 5 mL of glacial acetic acid, followed by the addition of zinc powder. After 5 hours, the reaction was completed. The mixture was filtered, and the filtrate was concentrated to obtain the title compound. MS m/z (ESI): 315.20 [M+1].

### Synthesis of Intermediate 6-5

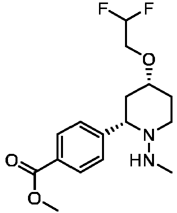

Intermediate 6-4 was placed in a reaction flask, dissolved in dichloromethane, and iodomethane was added dropwise. After the reaction was completed, saturated brine and dichloromethane were added. The organic phase was concentrated under reduced pressure and subjected to column chromatography to obtain the title compound. MS m/z (ESI): 329.20 [M+1].

### Example 29 Synthesis of Intermediate 7-1

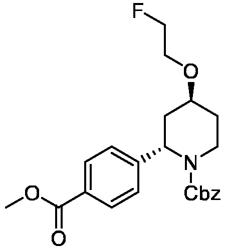

Sodium hydride was placed in a 50 mL three-necked flask, and 10 mL of anhydrous N,N-dimethylformamide was added. The flask was placed in an ice bath. After 10 min, intermediate 1-3 was dissolved in 10 mL of anhydrous N,N-dimethylformamide and added dropwise to the reaction flask. After 30 min, 2-fluoroethyl trifluoromethanesulfonate was added dropwise. After the dropwise addition was completed, water and ethyl acetate were added 30 min later. The organic phase was concentrated under reduced pressure to obtain the title compound as a colorless oil. MS m/z (ESI): 416.18 [M+1].

### Example 30 Synthesis of Intermediate 7-2

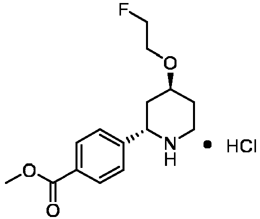

Intermediate 7-1 was placed in a single-necked reaction flask, followed by the addition of 10 mL of methanol, then 2 N hydrochloric acid, and subsequently 10% palladium on carbon. The system was purged with hydrogen three times, and the reaction was carried out overnight. After the reaction was completed, the mixture was subjected to suction filtration and then concentrated under reduced pressure to obtain a white solid. The solid was slurried with methyl *tert-butyl* ether for impurity removal, then filtered to obtain the title compound as a white solid powder. MS m/z (ESI): 282.12 [M+1].

### Example 31 Synthesis of Intermediate 7-3

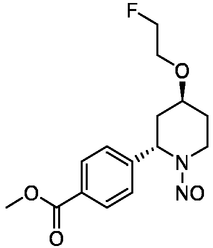

Intermediate 7-2 was placed in a 25 mL two-necked flask and dissolved in 5 mL of DCM. The reaction flask was placed in an ice bath, followed by the addition of pyridine and then nitrosonium tetrafluoroborate. After the reaction was completed, water and ethyl acetate were added. The organic phase was dried and concentrated under reduced pressure to obtain the title compound. MS m/z (ESI): 311.20 [M+1].

### Example 32 Synthesis of Intermediate 7-4

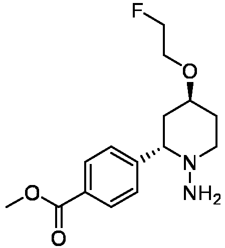

Intermediate 7-3 was placed in a reaction flask, dissolved in 5 mL of glacial acetic acid, followed by the addition of zinc powder. After 5 hours, the reaction was completed. The mixture was filtered, and the filtrate was concentrated to obtain the title compound. MS m/z (ESI): 297.20 [M+1].

### Synthesis of Intermediate 7-5

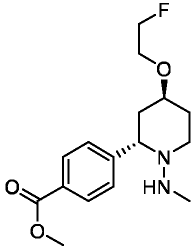

Intermediate 7-4 was placed in a reaction flask, dissolved in dichloromethane, and iodomethane was added dropwise. After the reaction was completed, saturated brine and dichloromethane were added. The organic phase was concentrated under reduced pressure and subjected to column chromatography to obtain the title compound. MS m/z (ESI): 310.20 [M+1].

### Example 33 Synthesis of Intermediate 8-1

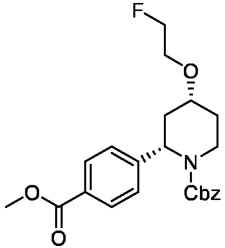

Sodium hydride was placed in a 50 mL three-necked flask, and 10 mL of anhydrous N,N-dimethylformamide was added. The flask was placed in an ice bath. After 10 min, intermediate 2-1 was dissolved in 10 mL of anhydrous N,N-dimethylformamide and added dropwise to the reaction flask. After 30 min, 2-fluoroethyl trifluoromethanesulfonate was added dropwise. After the dropwise addition was completed, water and ethyl acetate were added 30 min later. The organic phase was concentrated under reduced pressure to obtain the title compound as a colorless oil. MS m/z (ESI): 416.18 [M+1].

### Example 34 Synthesis of Intermediate 8-2

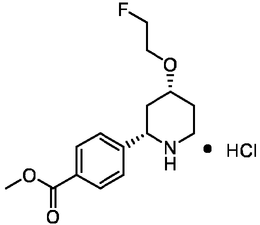

Intermediate 8-1 was placed in a single-necked reaction flask, followed by the addition of 10 mL of methanol, then 2 N hydrochloric acid, and subsequently 10% palladium on carbon. The system was purged with hydrogen three times, and the reaction was carried out overnight. After the reaction was completed, the mixture was subjected to suction filtration and then concentrated under reduced pressure to obtain a white solid. The solid was slurried with methyl *tert-butyl* ether for impurity removal, then filtered to obtain the title compound as a white solid powder. MS m/z (ESI): 282.12 [M+1].

### Example 35 Synthesis of Intermediate 8-3

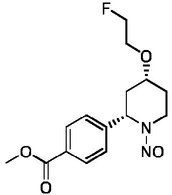

Intermediate 8-2 was placed in a 25 mL two-necked flask and dissolved in 5 mL of DCM. The reaction flask was placed in an ice bath, followed by the addition of pyridine and then nitrosonium tetrafluoroborate. After the reaction was completed, water and ethyl acetate were added. The organic phase was dried and concentrated under reduced pressure to obtain the title compound. MS m/z (ESI): 311.20 [M+1].

### Example 36 Synthesis of Intermediate 8-4

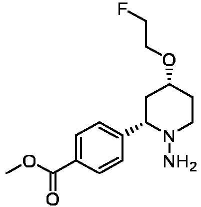

Intermediate 8-3 was placed in a reaction flask, dissolved in 5 mL of glacial acetic acid, followed by the addition of zinc powder. After 5 hours, the reaction was completed. The mixture was filtered, and the filtrate was concentrated to obtain the title compound. MS m/z (ESI): 297.20 [M+1].

### Synthesis of Intermediate 8-5

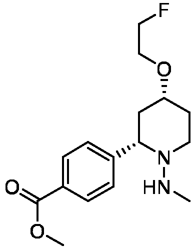

Intermediate 8-4 was placed in a reaction flask, dissolved in dichloromethane, and iodomethane was added dropwise. After the reaction was completed, saturated brine and dichloromethane were added. The organic phase was concentrated under reduced pressure and subjected to column chromatography to obtain the title compound. MS m/z (ESI): 310.20 [M+1].

### Example 37 Synthesis of Intermediate 9-1

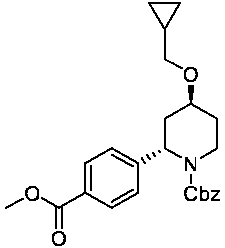

Sodium hydride was placed in a 50 mL three-necked flask, and 10 mL of anhydrous N,N-dimethylformamide was added. The flask was placed in an ice bath. After 10 min, intermediate 1-3 was dissolved in 10 mL of anhydrous N,N-dimethylformamide and added dropwise to the reaction flask. After 30 min, cyclohexylmethyl bromide was added dropwise. After the dropwise addition was completed, water and ethyl acetate were added 30 min later. The organic phase was concentrated under reduced pressure to obtain the title compound as a colorless oil. MS m/z (ESI): 424.18 [M+1]. ¹HNMR(400 MHz, CDCl₃) δ 8.00 (d, J = 8.3 Hz, 2H), 7.44 - 7.27 (m, 7H), 5.19 (s, 2H), 3.92 (s, 3H), 3.36 (t, J = 11.1 Hz, 1H), 3.26 (d, J = 6.8 Hz, 2H), 2.83 (dd, J = 13.4, 10.9 Hz, 1H), 2.66 (d, J = 13.4 Hz, 1H), 2.06 - 1.80 (m, 2H), 1.25 (s, 2H), 1.01 (s, 1H), 0.87 (d, J = 10.3 Hz, 2H), 0.52 (d, J = 8.0 Hz, 2H), 0.17 (q, J = 4.9 Hz, 2H).

### Example 38 Synthesis of Intermediate 9-2

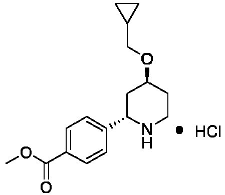

Intermediate 9-1 was placed in a single-necked reaction flask, followed by the addition of 10 mL of methanol, then 2 N hydrochloric acid, and subsequently 10% palladium on carbon. The system was purged with hydrogen three times, and the reaction was carried out overnight. After the reaction was completed, the mixture was subjected to suction filtration and then concentrated under reduced pressure to obtain a white solid. The solid was slurried with methyl *tert-butyl* ether for impurity removal, then filtered to obtain the title compound as a white solid powder. MS m/z (ESI): 290.12 [M+1]. ¹HNMR(400 MHz, MeOD) δ 8.12 (d, J = 8.4 Hz, 2H), 7.61 (d, J = 8.3 Hz, 2H), 4.71 - 4.56 (m, 1H), 3.98 (s, 1H), 3.92 (s, 3H), 3.49 (dd, J = 18.3, 7.8 Hz, 1H), 3.38 (dd, J = 17.1, 4.9 Hz, 3H), 2.35 - 2.11 (m, 3H), 2.04 - 1.91 (m, 1H), 1.19 - 1.06 (m, 1H), 0.61 - 0.51 (m, 2H), 0.33 - 0.21 (m, 2H).

### Example 39 Synthesis of Intermediate 9-3

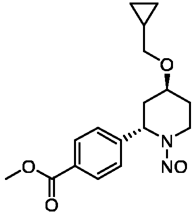

Intermediate 9-2 was placed in a 25 mL two-necked flask and dissolved in 5 mL of DCM. The reaction flask was placed in an ice bath, followed by the addition of pyridine and then nitrosonium tetrafluoroborate. After the reaction was completed, water and ethyl acetate were added. The organic phase was dried and concentrated under reduced pressure to obtain the title compound. MS m/z (ESI): 319.20 [M+1].

### Example 40 Synthesis of Intermediate 9-4

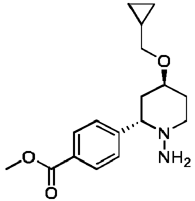

Intermediate 9-3 was placed in a reaction flask, dissolved in 5 mL of glacial acetic acid, followed by the addition of zinc powder. After 5 hours, the reaction was completed. The mixture was filtered, and the filtrate was concentrated to obtain the title compound. MS m/z (ESI): 305.20 [M+1].

### Synthesis of Intermediate 9-5

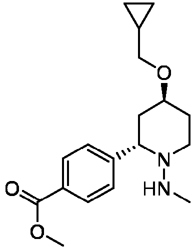

Intermediate 9-4 was placed in a reaction flask, dissolved in dichloromethane, and iodomethane was added dropwise. After the reaction was completed, saturated brine and dichloromethane were added. The organic phase was concentrated under reduced pressure and subjected to column chromatography to obtain the title compound. MS m/z (ESI): 319.20 [M+1].

### Example 41 Synthesis of Intermediate 10-1

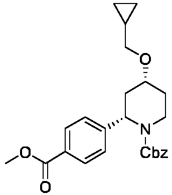

Sodium hydride was placed in a 50 mL three-necked flask, and 10 mL of anhydrous *N,N*-dimethylformamide was added. The flask was placed in an ice bath. After 10 min, intermediate 2-1 was dissolved in 10 mL of anhydrous N,N-dimethylformamide and added dropwise to the reaction flask. After 30 min, cyclohexylmethyl bromide was added dropwise. After the dropwise addition was completed, water and ethyl acetate were added 30 min later. The organic phase was concentrated under reduced pressure to obtain the title compound as a colorless oil. MS m/z (ESI): 424.18 [M+1].

### Example 42 Synthesis of Intermediate 10-2

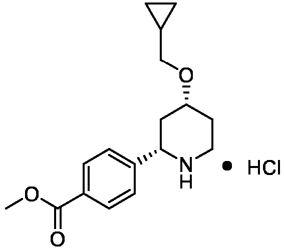

Intermediate 10-1 was placed in a single-necked reaction flask, followed by the addition of 10 mL of methanol, then 2 N hydrochloric acid, and subsequently 10% palladium on carbon. The system was purged with hydrogen three times, and the reaction was carried out overnight. After the reaction was completed, the mixture was subjected to suction filtration and then concentrated under reduced pressure to obtain a white solid. The solid was slurried with methyl tert-butyl ether for impurity removal, then filtered to obtain the title compound as a white solid powder. MS m/z (ESI): 290.12 [M+1].

### Example 43 Synthesis of Intermediate 10-3

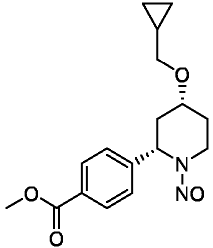

Intermediate 10-2 was placed in a 25 mL two-necked flask and dissolved in 5 mL of DCM. The reaction flask was placed in an ice bath, followed by the addition of pyridine and then nitrosonium tetrafluoroborate. After the reaction was completed, water and ethyl acetate were added. The organic phase was dried and concentrated under reduced pressure to obtain the title compound. MS m/z (ESI): 319.20 [M+1].

### Example 44 Synthesis of Intermediate 10-4

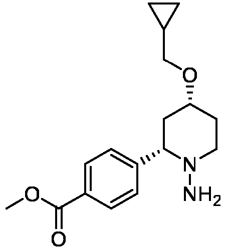

Intermediate 10-3 was placed in a reaction flask, dissolved in 5 mL of glacial acetic acid, followed by the addition of zinc powder. After 5 hours, the reaction was completed. The mixture was filtered, and the filtrate was concentrated to obtain the title compound. MS m/z (ESI): 305.20 [M+1].

### Synthesis of Intermediate 10-5

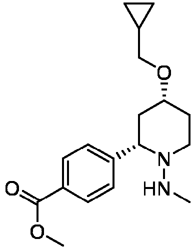

Intermediate 10-4 was placed in a reaction flask, dissolved in dichloromethane, and iodomethane was added dropwise. After the reaction was completed, saturated brine and dichloromethane were added. The organic phase was concentrated under reduced pressure and subjected to column chromatography to obtain the title compound. MS m/z (ESI): 319.20 [M+1].

### Example 45 Synthesis of Intermediate 11-1

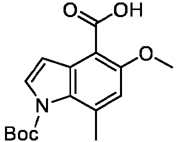

*tert*-Butyl 4-formyl-5-methoxy-7-methyl-1H-indole-1-carboxylate was added to a 50 mL three-necked flask and dissolved in DCM solution. The mixture was placed in an ice bath, then 20% hydrogen peroxide solution was added, and the reaction was carried out overnight. After the reaction was completed, the reaction mixture was subjected to phase separation and extraction. The organic phase was washed three times with saturated brine, concentrated under reduced pressure, and subjected to column chromatography to obtain the title compound. MS m/z (ESI): 306.20[M+1], ¹HNMR(600 MHz, CDCl₃) δ 7.64 (d,J = 3.7 Hz, 1H), 7.60 (d,J = 3.7 Hz, 1H), 6.82 (s, 1H), 4.11 (s, 3H), 2.68 (s, 3H), 1.63 (s, 9H).

### Example 46 Synthesis of Intermediate 12-1

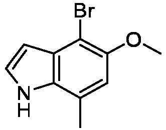

1-Bromo-2-methoxy-4-methyl-5-nitrobenzene was placed in a Schlenk tube, purged with nitrogen three times, and reacted at -20°C. A solution of vinylmagnesium bromide in tetrahydrofuran was added dropwise. After the reaction was carried out for 20 min, the reaction flask was placed at room temperature and the reaction was carried out overnight. After the reaction was completed, the reaction mixture was quenched with a saturated aqueous solution of ammonium chloride and extracted with dichloromethane. The organic phase was concentrated under reduced pressure and subjected to column chromatography to obtain the title compound (). MS m/z (ESI): 240.20 [M+1]. ¹HNMR (600 MHz, CDCl₃) δ 7.54 (d, J = 3.7 Hz, 1H), 6.74 (s, 1H), 6.59 (d, J = 3.7 Hz, 1H), 3.93 (s, 3H), 2.61 (s, 3H).

### Example 47 Synthesis of Intermediate 12-2

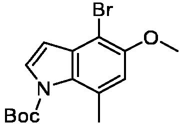

Intermediate 12-1 was added to a reaction flask, followed by the addition of di*-tert-*butyl dicarbonate, 4-dimethylaminopyridine, and *N,N-diisopropylethylamine.* The mixture was reacted at room temperature for 2 hours. After the reaction was completed, a saturated ammonium chloride solution was added, and the mixture was extracted with dichloromethane. The organic phase was concentrated under reduced pressure to obtain the title compound as a white solid powder. MS m/z (ESI): 340.20 [M+1], ¹HNMR (600 MHz, CDCl₃) δ 7.54 (d, J = 3.7 Hz, 1H), 6.74 (s, 1H), 6.59 (d, J = 3.7 Hz, 1H), 3.93 (s, 3H), 2.61 (s, 3H), 1.63 (s, 9H).

### Example 48 Synthesis of Intermediate 13-1: tert-Butyl 4-(hydroxymethyl)-5-methoxy-7-methyl-1H-indole-1-carboxylate:

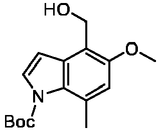

*tert*-Butyl 4-formyl-5-methoxy-7-methyl-1H-indole-1-carboxylate solid was added to a 100 mL three-necked flask, dissolved in methanol, followed by the portion-wise addition of sodium borohydride solid. After the reaction was completed, the reaction mixture was quenched with 2 N hydrochloric acid, extracted with dichloromethane, and concentrated under reduced pressure to obtain the title compound as a white solid powder. MS m/z (ESI): 292.20 [M+1], ¹HNMR (400 MHz, CDCl₃) δ 7.52 (d, J = 3.8 Hz, 1H), 6.74 (s, 1H), 6.61 (d, J = 3.8 Hz, 1H), 4.88 (s, 2H), 3.89 (s, 3H), 2.62 (s, 3H), 1.62 (s, 9H).

### Example 49 Synthesis of Intermediate 13-2: tert-Butyl 4-(chloromethyl)-5-methoxy-7-methyl-1H-indole-1-carboxylate

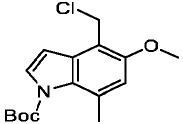

Intermediate 13-1 was placed in a 100 mL three-necked flask, dissolved in dichloromethane, followed by the addition of triethylamine. The reaction flask was placed in an ice bath, and then thionyl chloride was added. The reaction was carried out in the ice bath for 2 hours. After the reaction was completed, the reaction was quenched with brine. The organic phase was concentrated under reduced pressure and subjected to column chromatography to obtain the title compound. MS m/z (ESI): 310.20 [M+1].

### Example 50 Synthesis of Intermediate 13-3: tert-Butyl 4-(fluoromethyl)-5-methoxy-7-methyl-1H-indole-1-carboxylate

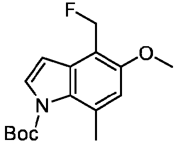

Intermediate 13-1 was placed in a 100 mL three-necked flask and dissolved in dichloromethane. The reaction flask was placed in an ice bath, and diethylaminosulfur trifluoride was added. The reaction was carried out overnight in an ice bath. After the reaction was completed, saturated brine was added to quench the reaction. After the phases were separated, the organic phase was concentrated under reduced pressure to obtain the title compound. MS m/z (ESI): 294.10 [M+1].

### Example 51 Synthesis of Intermediate 13-4: tert-Butyl 4-(bromofluoromethyl)-5-methoxy-7-methyl-1H-indole-1-carboxylate

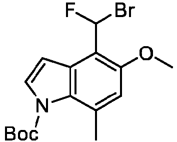

Intermediate 13-3 was placed in a 100 mL three-necked flask and dissolved in dichloromethane. The reaction flask was placed in an ice bath, andN-bromosuccinimide solid was added. After 2 hours, upon completion of the reaction, saturated brine was added to quench the reaction. The organic phase was concentrated under reduced pressure and subjected to column chromatography to obtain the title compound. MS m/z (ESI): 372.10 [M+1].

### Example 52 Synthesis of Intermediate 14-1: tert-Butyl 4-(difluoromethyl)-5-methoxy-7-methyl-1H-indole-1-carboxylate

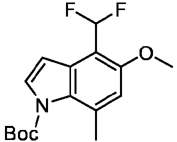

*tert*-Butyl 4-formyl-5-methoxy-7-methyl-1H-indole-1-carboxylate solid was added to a 100 mL three-necked flask and dissolved in dichloromethane. The reaction flask was placed in an ice bath, and diethylaminosulfur trifluoride was added. After the reaction was completed, saturated sodium bicarbonate solution was added to quench the reaction. The organic phase was concentrated under reduced pressure and subjected to column chromatography to obtain the title compound. MS m/z (ESI): 312.30 [M+1].

### Example 53 Synthesis of Intermediate 14-2: tert-Butyl 4-(bromodifluoromethyl)-5-methoxy-7-methyl-1H-indole-1-carboxylate

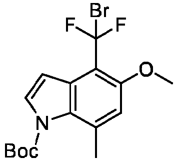

Intermediate 14-1 was placed in a 100 mL three-necked flask and dissolved in dichloromethane. The reaction flask was placed in an ice bath, and bromine water was added. After the reaction was completed, the mixture was washed three times with saturated sodium bicarbonate. The organic phase was concentrated under reduced pressure and subjected to column chromatography to obtain the title compound. MS m/z (ESI): 390.00 [M+1].

### Example 54 Synthesis of Compound 1-1: tert-Butyl 4-((2S,4S)-4-ethoxy-2-(4-(methoxycarbonyl)phenyl)piperidine-1-carbonyl)-5-methoxy-7-methyl-1H-indole-1-carboxylate

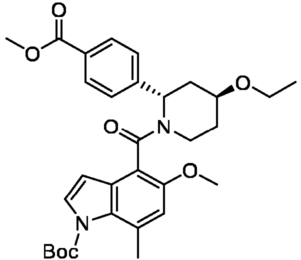

Intermediate 1-5 was added to a 10 mL three-necked flask and dissolved in dichloromethane. *N,N*-Diisopropylethylamine was added, and the mixture was placed in an ice bath, followed by the addition of intermediate 11-1. After stirring for 10 min, *tert-butyl* 2-(7-azabenzotriazol-1-yl)-*N,N,N',N'*-tetramethyluronium hexafluorophosphate was added. After the reaction was completed, dichloromethane and saturated brine were added, and the organic phase was concentrated under reduced pressure to obtain the title compound. MS m/z (ESI): 551.26 [M+1]. ¹HNMR(600 MHz, CDCl₃) δ 8.04 - 7.84 (m, 2H), 7.52 (ddd,J = 30.1, 29.6, 8.1 Hz, 2H), 7.37 (dd,J = 32.9, 27.2 Hz, 1H), 7.22 (d,J = 8.1 Hz, 1H), 6.73 (dd,J = 38.0, 16.8 Hz, 1H), 6.54 - 6.33 (m, 1H), 5.38 - 4.71 (m, 1H), 3.97 - 3.83 (m, 6H), 3.71 (dd,J = 31.7, 4.6 Hz, 1H), 3.43 - 3.12 (m, 4H), 2.72 - 2.54 (m, 4H), 1.63 (d,J = 6.5 Hz, 9H), 1.41 - 1.11 (m, 6H).

### Example 55 Synthesis of Compound 1: 4-((2S,4S)-4-Ethoxy-1-(5-methoxy-7-methyl-1H-indole-4-carbonyl)piperidin-2-yl)benzoic acid

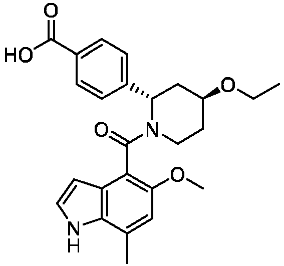

Compound 1-1 was placed in a 10 mL three-necked flask, dissolved in tetrahydrofuran and methanol, followed by the addition of a 1 M aqueous lithium hydroxide solution. The mixture was heated to 45°C and maintained at this temperature overnight. After the reaction was completed, the reaction mixture was concentrated under reduced pressure and subjected to reversed-phase preparative chromatography (gradient elution with aqueous hydrochloric acid: acetonitrile) to obtain the title compound. MS m/z (ESI): 437.20 [M+1], ¹HNMR (600 MHz, MeOD) δ 8.02 (ddd, J = 66.1, 32.9, 7.3 Hz, 2H), 7.72-7.45 (m, 2H), 7.38-7.21 (m, 1H), 6.77 (dd, J = 66.8, 34.7 Hz, 1H), 6.43-6.27 (m, 1H), 5.07 (d, J = 90.3 Hz, 1H), 3.97-3.61 (m, 3H), 3.60-3.41 (m, 4H), 2.95 (dddd, J = 63.1, 56.8, 46.2, 45.4 Hz, 2H), 2.67-2.46 (m, 3H), 2.17-1.30 (m, 3H), 1.21-1.13 (m, 3H).

### Example 56 Synthesis of Compound 2-1: tert-Butyl 4-((2S,4R)-4-ethoxy-2-(4-(methoxycarbonyl)phenyl)piperidine-1-carbonyl)-5-methoxy-7-methyl-1H-indole-1-carboxylate

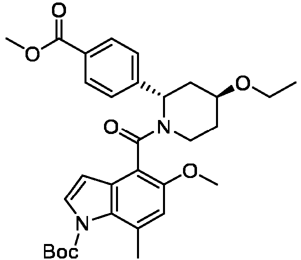

Intermediate 2-3 was added to a 10 mL three-necked flask and dissolved in dichloromethane. N,N-Diisopropylethylamine was added, and the mixture was placed in an ice bath, followed by the addition of intermediate 11-1. After stirring for 10 min, *tert-butyl* 2-(7-azabenzotriazol-1-yl)-*N,N,N',N'*-tetramethyluronium hexafluorophosphate was added. After the reaction was completed, dichloromethane and saturated brine were added, and the organic phase was concentrated under reduced pressure to obtain the title compound. MS m/z (ESI): 551.26 [M+1]. ¹HNMR(600 MHz, CDCl₃) δ 8.04 - 7.84 (m, 2H), 7.52 (ddd,J = 30.1, 29.6, 8.1 Hz, 2H), 7.37 (dd,J = 32.9, 27.2 Hz, 1H), 7.22 (d,J = 8.1 Hz, 1H), 6.73 (dd,J = 38.0, 16.8 Hz, 1H), 6.54 - 6.33 (m, 1H), 5.38 - 4.71 (m, 1H), 3.97 - 3.83 (m, 6H), 3.71 (dd,J = 31.7, 4.6 Hz, 1H), 3.43 - 3.12 (m, 4H), 2.72 - 2.54 (m, 4H), 1.63 (d,J = 6.5 Hz, 9H), 1.41 - 1.11 (m, 6H).

### Example 57 Synthesis of Compound 2: 4-((2S,4R)-4-Ethoxy-1-(5-methoxy-7-methyl-1H-indole-4-carbonyl)piperidin-2-yl)benzoic acid

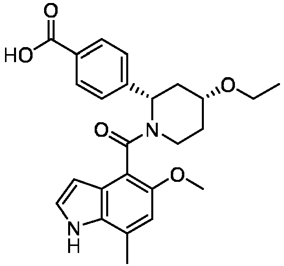

Compound 2-1 was placed in a 10 mL three-necked flask, dissolved in tetrahydrofuran and methanol, followed by the addition of a 1 M aqueous lithium hydroxide solution. The mixture was heated to 45°C and maintained at this temperature overnight. After the reaction was completed, the reaction mixture was concentrated under reduced pressure and subjected to reversed-phase preparative chromatography (gradient elution with aqueous hydrochloric acid: acetonitrile) to obtain the title compound. MS m/z (ESI): 437.20 [M+1], ¹HNMR (600 MHz, MeOD) δ 8.02 (ddd, J = 66.1, 32.9, 7.3 Hz, 2H), 7.72 - 7.45 (m, 2H), 7.38 - 7.21 (m, 1H), 6.77 (dd, J = 66.8, 34.7 Hz, 1H), 6.43 - 6.27 (m, 1H), 5.07 (d, J = 90.3 Hz, 1H), 3.97 - 3.61 (m, 3H), 3.60 - 3.41 (m, 4H), 2.95 (dddd, J = 63.1, 56.8, 46.2, 45.4 Hz, 2H), 2.67 - 2.46 (m, 3H), 2.17 - 1.30 (m, 3H), 1.21 - 1.13 (m, 3H).

### Example 58 Synthesis of Compound 3-1: tert-Butyl 5-methoxy-4-((2S,4S)-2-(4-(methoxycarbonyl)phenyl)-4-(2,2,2-trifluoroethoxy)piperidine-1-carbonyl)-7-methyl-1H-indole-1-carboxylate

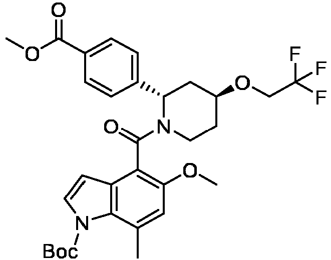

Intermediate 3-2 was added to a 10 mL three-necked flask and dissolved in dichloromethane. *N,N*-Diisopropylethylamine was added, and the mixture was placed in an ice bath, followed by the addition of intermediate 11-1. After stirring for 10 min, *tert-butyl* 2-(7-azabenzotriazol-1-yl)-*N,N,N',N'*-tetramethyluronium hexafluorophosphate was added. After the reaction was completed, dichloromethane and saturated brine were added, and the organic phase was concentrated under reduced pressure to obtain the title compound. MS m/z (ESI): 605.26 [M+1].

### Example 59 Synthesis of Compound 3: 4-((2S,4S)-1-(5-Methoxy-7-methyl-1H-indole-4-carbonyl)-4-(2,2,2-trifluoroethoxy)piperidin-2-yl)benzoic acid

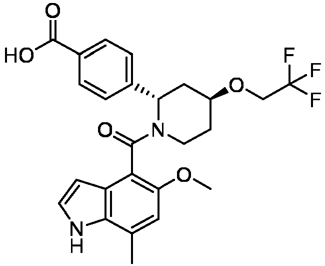

Compound 3-1 was placed in a 10 mL three-necked flask, dissolved in tetrahydrofuran and methanol, followed by the addition of a 1 M aqueous lithium hydroxide solution. The mixture was heated to 45°C and maintained at this temperature overnight. After the reaction was completed, the reaction mixture was concentrated under reduced pressure and subjected to reversed-phase preparative chromatography (gradient elution with aqueous hydrochloric acid: acetonitrile) to obtain the title compound. MS m/z (ESI): 491.20 [M+1].

### Example 60 Synthesis of Compound 4-1: tert-Butyl 5-methoxy-4-((2S,4R)-2-(4-(methoxycarbonyl)phenyl)-4-(2,2,2-trifluoroethoxy)piperidine-1-carbonyl)-7-methyl-1H-indole-1-carboxylate

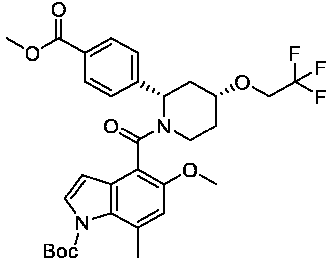

Intermediate 4-2 was added to a 10 mL three-necked flask and dissolved in dichloromethane. *N,N*-Diisopropylethylamine was added, and the mixture was placed in an ice bath, followed by the addition of intermediate 11-1. After stirring for 10 min, *tert*-butyl 2-(7-azabenzotriazol-1-yl)-*N,N,N',N'*-tetramethyluronium hexafluorophosphate was added. After the reaction was completed, dichloromethane and saturated brine were added, and the organic phase was concentrated under reduced pressure to obtain the title compound. MS m/z (ESI): 605.26 [M+1].

### Example 61 Synthesis of Compound 4: 4-((2S,4R)-1-(5-Methoxy-7-methyl-1H-indole-4-carbonyl)-4-(2,2,2-trifluoroethoxy)piperidin-2-yl)benzoic acid

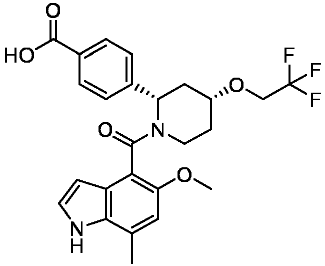

Compound 4-1 was placed in a 10 mL three-necked flask, dissolved in tetrahydrofuran and methanol, followed by the addition of a 1 M aqueous lithium hydroxide solution. The mixture was heated to 45°C and maintained at this temperature overnight. After the reaction was completed, the reaction mixture was concentrated under reduced pressure and subjected to reversed-phase preparative chromatography (gradient elution with aqueous hydrochloric acid: acetonitrile) to obtain the title compound. MS m/z (ESI): 491.20 [M+1].

### Example 62 Synthesis of Compound 5-1: tert-Butyl 4-((2S,4S)-4-(2,2-difluoroethoxy)-2-(4-(methoxycarbonyl)phenyl)piperidine-1-carbonyl)-5-methoxy-7-methyl-1H-indole-1-carboxylate

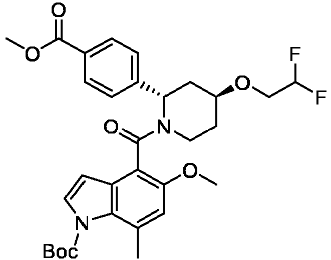

Intermediate 5-2 was added to a 10 mL three-necked flask and dissolved in dichloromethane. *N,N*-Diisopropylethylamine was added, and the mixture was placed in an ice bath, followed by the addition of intermediate 11-1. After stirring for 10 min, *tert*-butyl 2-(7-azabenzotriazol-1-yl)-*N,N,N',N'*-tetramethyluronium hexafluorophosphate was added. After the reaction was completed, dichloromethane and saturated brine were added, and the organic phase was concentrated under reduced pressure to obtain the title compound. MS m/z (ESI): 587.30 [M+1].

### Example 63 Synthesis of Compound 5: 4-((2S,4S)-4-(2,2-Difluoroethoxy)-1-(5-methoxy-7-methyl-1H-indole-4-carbonyl)piperidin-2-yl)benzoic acid

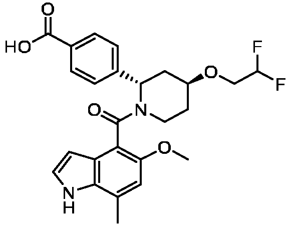

Compound 5-1 was placed in a 10 mL three-necked flask, dissolved in tetrahydrofuran and methanol, followed by the addition of a 1 M aqueous lithium hydroxide solution. The mixture was heated to 45°C and maintained at this temperature overnight. After the reaction was completed, the reaction mixture was concentrated under reduced pressure and subjected to reversed-phase preparative chromatography (gradient elution with aqueous hydrochloric acid: acetonitrile) to obtain the title compound. MS m/z (ESI): 473.20 [M+1].

### Example 64 Synthesis of Compound 6-1: tert-Butyl 4-((2S,4R)-4-(2,2-difluoroethoxy)-2-(4-(methoxycarbonyl)phenyl)piperidine-1-carbonyl)-5-methoxy-7-methyl-1H-indole-1-carboxylate

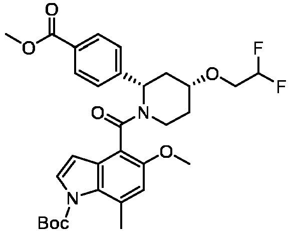

Intermediate 6-2 was added to a 10 mL three-necked flask and dissolved in dichloromethane. *N,N*-Diisopropylethylamine was added, and the mixture was placed in an ice bath, followed by the addition of intermediate 11-1. After stirring for 10 min, *tert*-butyl 2-(7-azabenzotriazol-1-yl)-*N,N,N',N'*-tetramethyluronium hexafluorophosphate was added. After the reaction was completed, dichloromethane and saturated brine were added, and the organic phase was concentrated under reduced pressure to obtain the title compound. MS m/z (ESI): 587.30 [M+1].

### Example 65 Synthesis of Compound 6: 4-((2S,4R)-4-(2,2-Difluoroethoxy)-1-(5-methoxy-7-methyl-1H-indole-4-carbonyl)piperidin-2-yl)benzoic acid

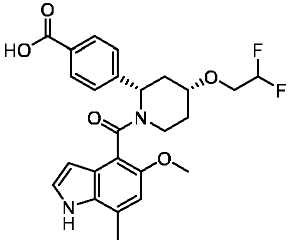

Compound 6-1 was placed in a 10 mL three-necked flask, dissolved in tetrahydrofuran and methanol, followed by the addition of a 1 M aqueous lithium hydroxide solution. The mixture was heated to 45°C and maintained at this temperature overnight. After the reaction was completed, the reaction mixture was concentrated under reduced pressure and subjected to reversed-phase preparative chromatography (gradient elution with aqueous hydrochloric acid: acetonitrile) to obtain the title compound. MS m/z (ESI): 473.20 [M+1].

### Example 66 Synthesis of Compound 7-1: tert-Butyl 4-((2S,4S)-4-(2-fluoroethoxy)-2-(4-(methoxycarbonyl)phenyl)piperidine-1-carbonyl)-5-methoxy-7-methyl-1H-indole-1-carboxylate

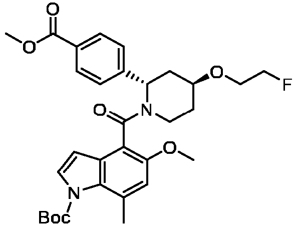

Intermediate 7-2 was added to a 10 mL three-necked flask and dissolved in dichloromethane. *N,N*-Diisopropylethylamine was added, and the mixture was placed in an ice bath, followed by the addition of intermediate 11-1. After stirring for 10 min, *tert*-butyl 2-(7-azabenzotriazol-1-yl)-*N,N,N',N'*-tetramethyluronium hexafluorophosphate was added. After the reaction was completed, dichloromethane and saturated brine were added, and the organic phase was concentrated under reduced pressure to obtain the title compound. MS m/z (ESI): 569.30 [M+1].

### Example 67 Synthesis of Compound 7: 4-((2S,4S)-4-(2-Fluoroethoxy)-1-(5-methoxy-7-methyl-1H-indole-4-carbonyl)piperidin-2-yl)benzoic acid

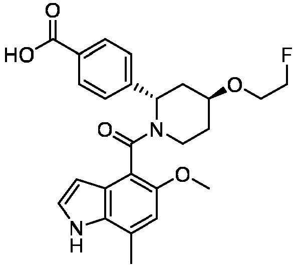

Compound 7-1 was placed in a 10 mL three-necked flask, dissolved in tetrahydrofuran and methanol, followed by the addition of a 1 M aqueous lithium hydroxide solution. The mixture was heated to 45°C and maintained at this temperature overnight. After the reaction was completed, the reaction mixture was concentrated under reduced pressure and subjected to reversed-phase preparative chromatography (gradient elution with aqueous hydrochloric acid: acetonitrile) to obtain the title compound. MS m/z (ESI): 455.20 [M+1].

### Example 68 Synthesis of Compound 8-1: tert-Butyl 4-((2S,4R)-4-(2-fluoroethoxy)-2-(4-(methoxycarbonyl)phenyl)piperidine-1-carbonyl)-5-methoxy-7-methyl-1H-indole-1-carboxylate

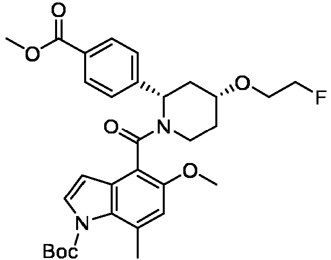

Intermediate 8-2 was added to a 10 mL three-necked flask and dissolved in dichloromethane. *N,N-*Diisopropylethylamine was added, and the mixture was placed in an ice bath, followed by the addition of intermediate 11-1. After stirring for 10 min, *tert*-butyl 2-(7-azabenzotriazol-1-yl)-*N,N,N',N'*-tetramethyluronium hexafluorophosphate was added. After the reaction was completed, dichloromethane and saturated brine were added, and the organic phase was concentrated under reduced pressure to obtain the title compound. MS m/z (ESI): 569.30 [M+1].

### Example 69 Synthesis of Compound 8: 4-((2S,4R)-4-(2-Fluoroethoxy)-1-(5-methoxy-7-methyl-1H-indole-4-carbonyl)piperidin-2-yl)benzoic acid

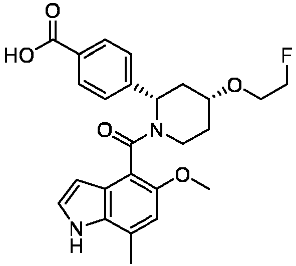

Compound 8-1 was placed in a 10 mL three-necked flask, dissolved in tetrahydrofuran and methanol, followed by the addition of a 1 M aqueous lithium hydroxide solution. The mixture was heated to 45°C and maintained at this temperature overnight. After the reaction was completed, the reaction mixture was concentrated under reduced pressure and subjected to reversed-phase preparative chromatography (gradient elution with aqueous hydrochloric acid: acetonitrile) to obtain the title compound. MS m/z (ESI): 455.20 [M+1].

### Example 70 Synthesis of Compound 9-1: tert-Butyl 4-((2S,4S)-4-(cyclopropylmethoxy)-2-(4-(methoxycarbonyl)phenyl)piperidine-1-carbonyl)-5-methoxy-7-methyl-1H-indole-1-carboxylate

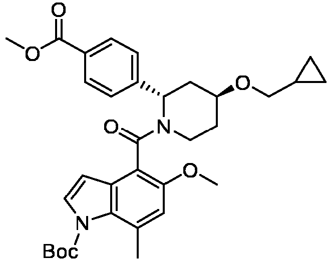

Intermediate 9-2 was added to a 10 mL three-necked flask and dissolved in dichloromethane. *N,N-*Diisopropylethylamine was added, and the mixture was placed in an ice bath, followed by the addition of intermediate 11-1. After stirring for 10 min, *tert*-butyl 2-(7-azabenzotriazol-1-yl)-*N,N,N',N'*-tetramethyluronium hexafluorophosphate was added. After the reaction was completed, dichloromethane and saturated brine were added, and the organic phase was concentrated under reduced pressure to obtain the title compound. MS m/z (ESI): 577.30 [M+1].

### Example 71 Synthesis of Compound 9: 4-((2S,4S)-4-(Cyclopropylmethoxy)-1-(5-methoxy-7-methyl-1H-indole-4-carbonyl)piperidin-2-yl)benzoic acid

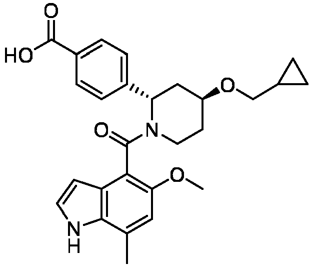

Compound 9-1 was placed in a 10 mL three-necked flask, dissolved in tetrahydrofuran and methanol, followed by the addition of a 1 M aqueous lithium hydroxide solution. The mixture was heated to 45°C and maintained at this temperature overnight. After the reaction was completed, the reaction mixture was concentrated under reduced pressure and subjected to reversed-phase preparative chromatography (gradient elution with aqueous hydrochloric acid: acetonitrile) to obtain the title compound. MS m/z (ESI): 463.55 [M+1]. ¹HNMR (600 MHz, MeOD) δ 8.13 - 7.88 (m, 2H), 7.76 - 7.45 (m, 2H), 7.32 (dt, J = 68.1, 33.3 Hz, 1H), 6.77 (dd, J = 67.1, 35.1 Hz, 1H), 6.35 (dd, J = 19.9, 3.0 Hz, 1H), 3.99 - 3.88 (m, 3H), 3.68 - 3.40 (m, 2H), 3.37 - 3.33 (m, 3H), 2.99 - 2.80 (m, 2H), 2.60 - 2.46 (m, 3H), 1.87 (ddd, J = 34.4, 30.0, 24.2 Hz, 2H), 1.76 - 1.20 (m, 2H).

### Example 72 Synthesis of Compound 10-1: tert-Butyl 4-((2S,4R)-4-(cyclopropylmethoxy)-2-(4-(methoxycarbonyl)phenyl)piperidine-1-carbonyl)-5-methoxy-7-methyl-1H-indole-1-carboxylate

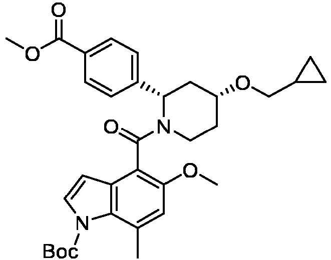

Intermediate 10-2 was added to a 10 mL three-necked flask and dissolved in dichloromethane. *N,N-*Diisopropylethylamine was added, and the mixture was placed in an ice bath, followed by the addition of intermediate 11-1. After stirring for 10 min, *tert-butyl* 2-(7-azabenzotriazol-1-yl)-*N,N,N',N'*-tetramethyluronium hexafluorophosphate was added. After the reaction was completed, dichloromethane and saturated brine were added, and the organic phase was concentrated under reduced pressure to obtain the title compound. MS m/z (ESI): 577.30 [M+1].

### Example 73 Synthesis of Compound 10: 4-((2S,4R)-4-(Cyclopropylmethoxy)-1-(5-methoxy-7-methyl-1H-indole-4-carbonyl)piperidin-2-yl)benzoic acid

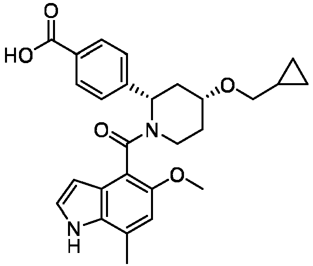

Compound 10-1 was placed in a 10 mL three-necked flask, dissolved in tetrahydrofuran and methanol, followed by the addition of a 1 M aqueous lithium hydroxide solution. The mixture was heated to 45°C and maintained at this temperature overnight. After the reaction was completed, the reaction mixture was concentrated under reduced pressure and subjected to reversed-phase preparative chromatography (gradient elution with aqueous hydrochloric acid: acetonitrile) to obtain the title compound. MS m/z (ESI): 463.55 [M+1]. ¹HNMR (600 MHz, MeOD) δ 8.13 - 7.88 (m, 2H), 7.76 - 7.45 (m, 2H), 7.32 (dt, J = 68.1, 33.3 Hz, 1H), 6.77 (dd, J = 67.1, 35.1 Hz, 1H), 6.35 (dd, J = 19.9, 3.0 Hz, 1H), 3.99 - 3.88 (m, 3H), 3.68 - 3.40 (m, 2H), 3.37 - 3.33 (m, 3H), 2.99 - 2.80 (m, 2H), 2.60 - 2.46 (m, 3H), 1.87 (ddd, J = 34.4, 30.0, 24.2 Hz, 2H), 1.76 - 1.20 (m, 2H).

### Example 74 Synthesis of Compound 11-1: tert-Butyl 4-(((2S,4S)-4-ethoxy-2-(4-(methoxycarbonyl)phenyl)piperidin-1-yl)amino)-5-methoxy-7-methyl-1H-indole-1-carboxylate

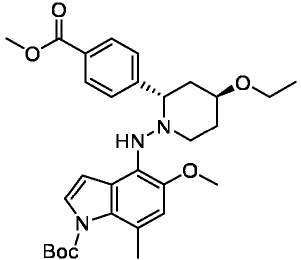

Intermediate 1-7 was placed in a 10 mL three-necked flask, and intermediate 12-2 solid was added, followed by the addition of 2-dicyclohexylphosphino-2',4',6'-triisopropylbiphenyl and tris(dibenzylideneacetone)dipalladium, and the final addition of sodium *tert*-butoxide solid. The mixture was dissolved in toluene, and the system was purged with nitrogen three times. The reaction was heated to 100°C and carried out overnight. After the reaction was completed, the reaction was quenched with a heated saturated aqueous ammonium chloride solution. The organic phase was concentrated under reduced pressure and subjected to column chromatography to obtain the title compound. MS m/z (ESI): 538.30 [M+1].

### Example 75 Synthesis of Compound 11: 4-((2S,4S)-4-Ethoxy-1-((5-methoxy-7-methyl-1H-indol-4-yl)amino)piperidin-2-yl)benzoic acid

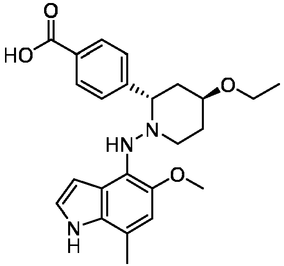

Compound 11-1 was placed in a 10 mL three-necked flask, dissolved in tetrahydrofuran and methanol, followed by the addition of a 1 M aqueous lithium hydroxide solution. The mixture was heated to 45°C and maintained at this temperature overnight. After the reaction was completed, the reaction mixture was concentrated under reduced pressure and subjected to reversed-phase preparative chromatography (gradient elution with aqueous hydrochloric acid: acetonitrile) to obtain the title compound. MS m/z (ESI): 463.55 [M+1].

### Example 76 Synthesis of Compound 12-1: tert-Butyl 4-(((2S,4R)-4-ethoxy-2-(4-(methoxycarbonyl)phenyl)piperidin-1-yl)amino)-5-methoxy-7-methyl-1H-indole-1-carboxylate

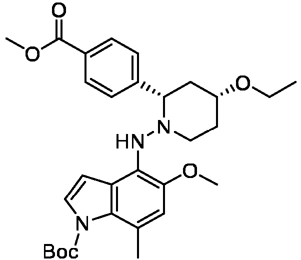

Intermediate 2-5 was placed in a 10 mL three-necked flask, and intermediate 12-2 solid was added, followed by the addition of 2-dicyclohexylphosphino-2',4',6'-triisopropylbiphenyl and tris(dibenzylideneacetone)dipalladium, and the final addition of sodium *tert*-butoxide solid. The mixture was dissolved in toluene, and the system was purged with nitrogen three times. The reaction was heated to 100°C and carried out overnight. After the reaction was completed, the reaction was quenched with a heated saturated aqueous ammonium chloride solution. The organic phase was concentrated under reduced pressure and subjected to column chromatography to obtain the title compound. MS m/z (ESI): 538.30 [M+1].

### Example 77 Synthesis of Compound 12: 4-((2S,4R)-4-Ethoxy-1-((5-methoxy-7-methyl-1H-indol-4-yl)amino)piperidin-2-yl)benzoic acid

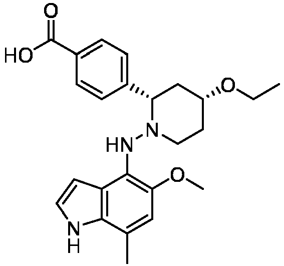

Compound 12-1 was placed in a 10 mL three-necked flask, dissolved in tetrahydrofuran and methanol, followed by the addition of a 1 M aqueous lithium hydroxide solution. The mixture was heated to 45°C and maintained at this temperature overnight. After the reaction was completed, the reaction mixture was concentrated under reduced pressure and subjected to reversed-phase preparative chromatography (gradient elution with aqueous hydrochloric acid: acetonitrile) to obtain the title compound. MS m/z (ESI): 463.55 [M+1].

### Example 78 Synthesis of Compound 13-1: tert-Butyl 5-methoxy-4-(((2S,4S)-2-(4-(methoxycarbonyl)phenyl)-4-(2,2,2-trifluoroethoxy)piperidin-1-yl)amino)-7-methyl-1H-indole-1-carboxylate

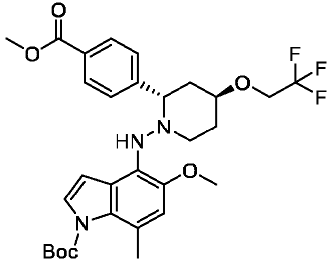

Intermediate 3-4 was placed in a 10 mL three-necked flask, and intermediate 12-2 solid was added, followed by the addition of 2-dicyclohexylphosphino-2',4',6'-triisopropylbiphenyl and tris(dibenzylideneacetone)dipalladium, and the final addition of sodium *tert*-butoxide solid. The mixture was dissolved in toluene, and the system was purged with nitrogen three times. The reaction was heated to 100°C and carried out overnight. After the reaction was completed, the reaction was quenched with a heated saturated aqueous ammonium chloride solution. The organic phase was concentrated under reduced pressure and subjected to column chromatography to obtain the title compound. MS m/z (ESI): 592.30 [M+1].

### Example 79 Synthesis of Compound 13: 4-((2S,4S)-1-((5-Methoxy-7-methyl-1H-indol-4-yl)amino)-4-(2,2,2-trifluoroethoxy)piperidin-2-yl)benzoic acid

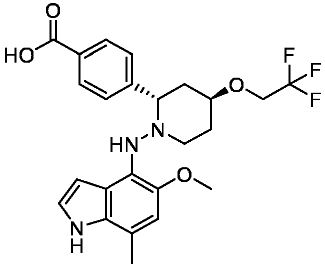

Compound 13-1 was placed in a 10 mL three-necked flask, dissolved in tetrahydrofuran and methanol, followed by the addition of a 1 M aqueous lithium hydroxide solution. The mixture was heated to 45°C and maintained at this temperature overnight. After the reaction was completed, the reaction mixture was concentrated under reduced pressure and subjected to reversed-phase preparative chromatography (gradient elution with aqueous hydrochloric acid: acetonitrile) to obtain the title compound. MS m/z (ESI): 478.55 [M+1].

### Example 80 Synthesis of Compound 14-1: tert-Butyl 5-methoxy-4-(((2S,4R)-2-(4-(methoxycarbonyl)phenyl)-4-(2,2,2-trifluoroethoxy)piperidin-1-yl)amino)-7-methyl-1H-indole-1-carboxylate

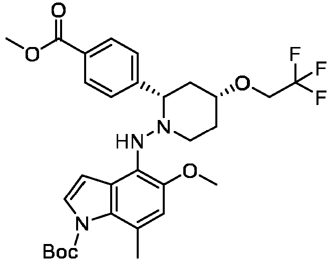

Intermediate 4-4 was placed in a 10 mL three-necked flask, and intermediate 12-2 solid was added, followed by the addition of 2-dicyclohexylphosphino-2',4',6'-triisopropylbiphenyl and tris(dibenzylideneacetone)dipalladium, and the final addition of sodium *tert*-butoxide solid. The mixture was dissolved in toluene, and the system was purged with nitrogen three times. The reaction was heated to 100°C and carried out overnight. After the reaction was completed, the reaction was quenched with a heated saturated aqueous ammonium chloride solution. The organic phase was concentrated under reduced pressure and subjected to column chromatography to obtain the title compound. MS m/z (ESI): 592.30 [M+1].

### Example 81 Synthesis of Compound 14: 4-((2S,4R)-1-((5-Methoxy-7-methyl-1H-indol-4-yl)amino)-4-(2,2,2-trifluoroethoxy)piperidin-2-yl)benzoic acid

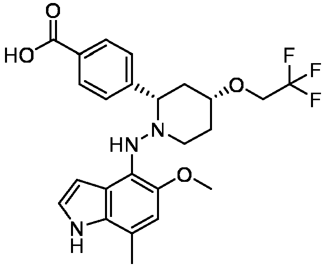

Compound 14-1 was placed in a 10 mL three-necked flask, dissolved in tetrahydrofuran and methanol, followed by the addition of a 1 M aqueous lithium hydroxide solution. The mixture was heated to 45°C and maintained at this temperature overnight. After the reaction was completed, the reaction mixture was concentrated under reduced pressure and subjected to reversed-phase preparative chromatography (gradient elution with aqueous hydrochloric acid: acetonitrile) to obtain the title compound. MS m/z (ESI): 478.55 [M+1].

### Example 82 Synthesis of Compound 15-1: tert-Butyl 4-(((2S,4S)-4-(2,2-difluoroethoxy)-2-(4-(methoxycarbonyl)phenyl)piperidin-1-yl)amino)-5-methoxy-7-methyl-1H-indole-1-carboxylate

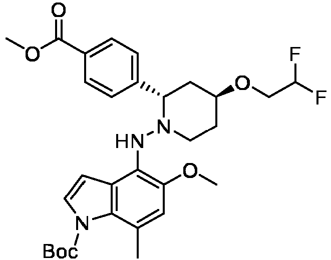

Intermediate 5-4 was placed in a 10 mL three-necked flask, and intermediate 12-2 solid was added, followed by the addition of 2-dicyclohexylphosphino-2',4',6'-triisopropylbiphenyl and tris(dibenzylideneacetone)dipalladium, and the final addition of sodium *tert*-butoxide solid. The mixture was dissolved in toluene, and the system was purged with nitrogen three times. The reaction was heated to 100°C and carried out overnight. After the reaction was completed, the reaction was quenched with a heated saturated aqueous ammonium chloride solution. The organic phase was concentrated under reduced pressure and subjected to column chromatography to obtain the title compound. MS m/z (ESI): 574.30 [M+1].

### Example 83 Synthesis of Compound 15: 4-((2S,4S)-4-(2,2-Difluoroethoxy)-1-((5-methoxy-7-methyl-1H-indol-4-yl)amino)piperidin-2-yl)benzoic acid

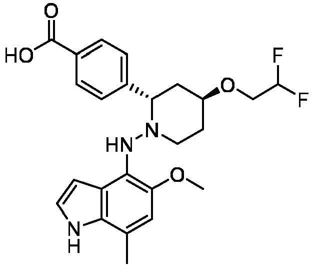

Compound 15-1 was placed in a 10 mL three-necked flask, dissolved in tetrahydrofuran and methanol, followed by the addition of a 1 M aqueous lithium hydroxide solution. The mixture was heated to 45°C and maintained at this temperature overnight. After the reaction was completed, the reaction mixture was concentrated under reduced pressure and subjected to reversed-phase preparative chromatography (gradient elution with aqueous hydrochloric acid: acetonitrile) to obtain the title compound. MS m/z (ESI): 460.55 [M+1].

### Example 84 Synthesis of Compound 16-1: tert-Butyl 4-(((2S,4R)-4-(2,2-difluoroethoxy)-2-(4-(methoxycarbonyl)phenyl)piperidin-1-yl)amino)-5-methoxy-7-methyl-1H-indole-1-carboxylate

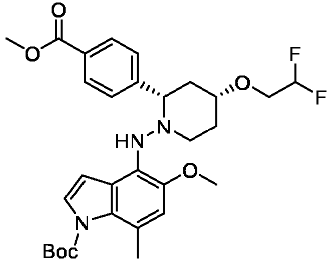

Intermediate 6-4 was placed in a 10 mL three-necked flask, and intermediate 12-2 solid was added, followed by the addition of 2-dicyclohexylphosphino-2',4',6'-triisopropylbiphenyl and tris(dibenzylideneacetone)dipalladium, and the final addition of sodium *tert*-butoxide solid. The mixture was dissolved in toluene, and the system was purged with nitrogen three times. The reaction was heated to 100°C and carried out overnight. After the reaction was completed, the reaction was quenched with a heated saturated aqueous ammonium chloride solution. The organic phase was concentrated under reduced pressure and subjected to column chromatography to obtain the title compound. MS m/z (ESI): 574.30 [M+1].

### Example 85 Synthesis of Compound 16: 4-((2S,4R)-4-(2,2-Difluoroethoxy)-1-((5-methoxy-7-methyl-1H-indol-4-yl)amino)piperidin-2-yl)benzoic acid

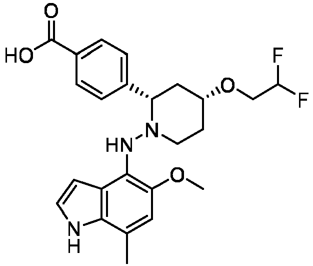

Compound 16-1 was placed in a 10 mL three-necked flask, dissolved in tetrahydrofuran and methanol, followed by the addition of a 1 M aqueous lithium hydroxide solution. The mixture was heated to 45°C and maintained at this temperature overnight. After the reaction was completed, the reaction mixture was concentrated under reduced pressure and subjected to reversed-phase preparative chromatography (gradient elution with aqueous hydrochloric acid: acetonitrile) to obtain the title compound. MS m/z (ESI): 460.55 [M+1].

### Example 86 Synthesis of Compound 17-1: tert-Butyl 4-(((2S,4S)-4-(2-fluoroethoxy)-2-(4-(methoxycarbonyl)phenyl)piperidin-1-yl)amino)-5-methoxy-7-methyl-1H-indole-1-carboxylate

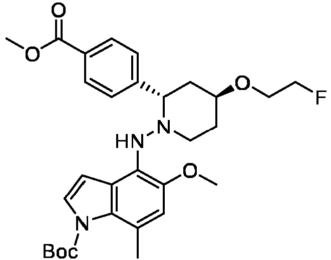

Intermediate 7-4 was placed in a 10 mL three-necked flask, and intermediate 12-2 solid was added, followed by the addition of 2-dicyclohexylphosphino-2',4',6'-triisopropylbiphenyl and tris(dibenzylideneacetone)dipalladium, and the final addition of sodium *tert-butoxide* solid. The mixture was dissolved in toluene, and the system was purged with nitrogen three times. The reaction was heated to 100°C and carried out overnight. After the reaction was completed, the reaction was quenched with a heated saturated aqueous ammonium chloride solution. The organic phase was concentrated under reduced pressure and subjected to column chromatography to obtain the title compound. MS m/z (ESI): 556.30 [M+1].

### Example 87 Synthesis of Compound 17: 4-((2S,4S)-4-(2-Fluoroethoxy)-1-((5-methoxy-7-methyl-1H-indol-4-yl)amino)piperidin-2-yl)benzoic acid

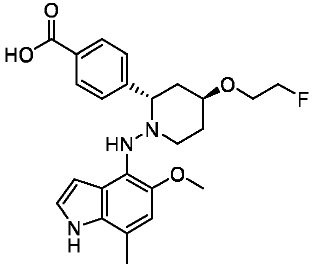

Compound 17-1 was placed in a 10 mL three-necked flask, dissolved in tetrahydrofuran and methanol, followed by the addition of a 1 M aqueous lithium hydroxide solution. The mixture was heated to 45°C and maintained at this temperature overnight. After the reaction was completed, the reaction mixture was concentrated under reduced pressure and subjected to reversed-phase preparative chromatography (gradient elution with aqueous hydrochloric acid: acetonitrile) to obtain the title compound. MS m/z (ESI): 442.55 [M+1].

### Example 88 Synthesis of Compound 18-1: tert-Butyl 4-(((2S,4R)-4-(2-fluoroethoxy)-2-(4-(methoxycarbonyl)phenyl)piperidin-1-yl)amino)-5-methoxy-7-methyl-1H-indole-1-carboxylate

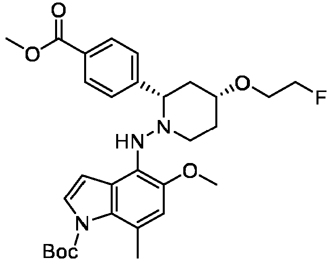

Intermediate 8-4 was placed in a 10 mL three-necked flask, and intermediate 12-2 solid was added, followed by the addition of 2-dicyclohexylphosphino-2',4',6'-triisopropylbiphenyl and tris(dibenzylideneacetone)dipalladium, and the final addition of sodium *tert*-butoxide solid. The mixture was dissolved in toluene, and the system was purged with nitrogen three times. The reaction was heated to 100°C and carried out overnight. After the reaction was completed, the reaction was quenched with a heated saturated aqueous ammonium chloride solution. The organic phase was concentrated under reduced pressure and subjected to column chromatography to obtain the title compound. MS m/z (ESI): 556.30 [M+1].

### Example 89 Synthesis of Compound 18: 4-((2S,4R)-4-(2-Fluoroethoxy)-1-((5-methoxy-7-methyl-1H-indol-4-yl)amino)piperidin-2-yl)benzoic acid

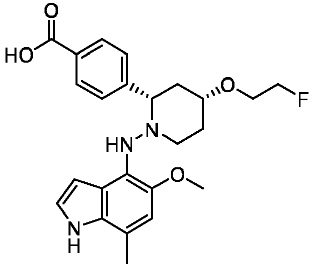

Compound 18-1 was placed in a 10 mL three-necked flask, dissolved in tetrahydrofuran and methanol, followed by the addition of a 1 M aqueous lithium hydroxide solution. The mixture was heated to 45°C and maintained at this temperature overnight. After the reaction was completed, the reaction mixture was concentrated under reduced pressure and subjected to reversed-phase preparative chromatography (gradient elution with aqueous hydrochloric acid: acetonitrile) to obtain the title compound. MS m/z (ESI): 442.55 [M+1].

### Example 90 Synthesis of Compound 19-1: tert-Butyl 4-((2S,4S)-4-(cyclopropylmethoxy)-2-(4-(methoxycarbonyl)phenyl)piperidin-1-yl)amino)-5-methoxy-7-methyl-1H-indole-1-carboxylate

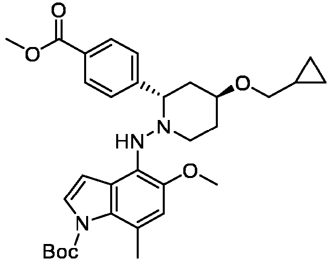

Intermediate 9-4 was placed in a 10 mL three-necked flask, and intermediate 12-2 solid was added, followed by the addition of 2-dicyclohexylphosphino-2',4',6'-triisopropylbiphenyl and tris(dibenzylideneacetone)dipalladium, and the final addition of sodium *tert*-butoxide solid. The mixture was dissolved in toluene, and the system was purged with nitrogen three times. The reaction was heated to 100°C and carried out overnight. After the reaction was completed, the reaction was quenched with a heated saturated aqueous ammonium chloride solution. The organic phase was concentrated under reduced pressure and subjected to column chromatography to obtain the title compound. MS m/z (ESI): 564.30 [M+1].

### Example 91 Synthesis of Compound 19: 4-((2S,4S)-4-(Cyclopropylmethoxy)-1-((5-methoxy-7-methyl-1H-indol-4-yl)amino)piperidin-2-yl)benzoic acid

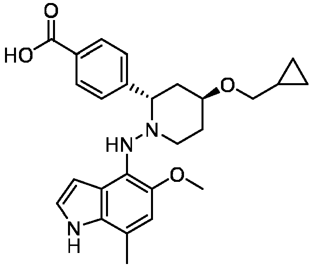

Compound 19-1 was placed in a 10 mL three-necked flask, dissolved in tetrahydrofuran and methanol, followed by the addition of a 1 M aqueous lithium hydroxide solution. The mixture was heated to 45°C and maintained at this temperature overnight. After the reaction was completed, the reaction mixture was concentrated under reduced pressure and subjected to reversed-phase preparative chromatography (gradient elution with aqueous hydrochloric acid: acetonitrile) to obtain the title compound. MS m/z (ESI): 450.55 [M+1].

### Example 92 Synthesis of Compound 20-1: tert-Butyl 4-((2S,4R)-4-(cyclopropylmethoxy)-2-(4-(methoxycarbonyl)phenyl)piperidin-1-yl)amino)-5-methoxy-7-methyl-1H-indole-1-carboxylate

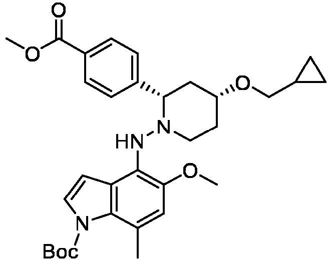

Intermediate 9-4 was placed in a 10 mL three-necked flask, and intermediate 12-2 solid was added, followed by the addition of 2-dicyclohexylphosphino-2',4',6'-triisopropylbiphenyl and tris(dibenzylideneacetone)dipalladium, and the final addition of sodium *tert*-butoxide solid. The mixture was dissolved in toluene, and the system was purged with nitrogen three times. The reaction was heated to 100°C and carried out overnight. After the reaction was completed, the reaction was quenched with a heated saturated aqueous ammonium chloride solution. The organic phase was concentrated under reduced pressure and subjected to column chromatography to obtain the title compound. MS m/z (ESI): 564.30 [M+1]. **Example 93 Synthesis of Compound 20:** 4-((2S,4R)-4-(Cyclopropylmethoxy)-1-((5-methoxy-7-methyl-1H-indol-4-yl)amino)piperidin-2-yl)benzoic acid

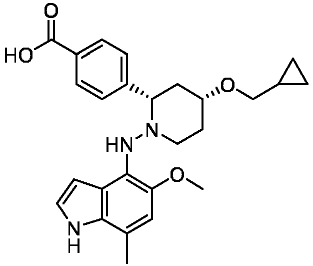

Compound 20-1 was placed in a 10 mL three-necked flask, dissolved in tetrahydrofuran and methanol, followed by the addition of a 1 M aqueous lithium hydroxide solution. The mixture was heated to 45°C and maintained at this temperature overnight. After the reaction was completed, the reaction mixture was concentrated under reduced pressure and subjected to reversed-phase preparative chromatography (gradient elution with aqueous hydrochloric acid: acetonitrile) to obtain the title compound. MS m/z (ESI): 450.55 [M+1].

### Example 94 Synthesis of Compound 21-1: tert-Butyl 4-(((2S,4S)-4-ethoxy-2-(4-(methoxycarbonyl)phenyl)piperidin-1-yl)fluoromethyl)-5-methoxy-7-methyl-1H-indole-1-carboxylate

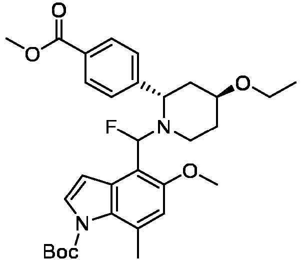

Intermediate 1-5 was added to a 10 mL three-necked flask, dissolved in dichloromethane, followed by the addition of triethylamine and then intermediate 13-4. The reaction was carried out overnight at room temperature. After the reaction was completed, the reaction was quenched with saturated ammonium chloride solution. The organic phase was concentrated under reduced pressure and subjected to column chromatography to obtain the title compound. MS m/z (ESI): 555.30 [M+1].

### Example 95 Synthesis of Compound 21: 4-((2S,4S)-4-Ethoxy-1-(fluoro(5-methoxy-7-methyl-1H-indol-4-yl)methyl)piperidin-2-yl)benzoic acid

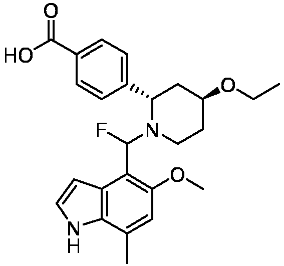

Compound 21-1 was placed in a 10 mL three-necked flask, dissolved in tetrahydrofuran and methanol, followed by the addition of a 1 M aqueous lithium hydroxide solution. The mixture was heated to 45°C and maintained at this temperature overnight. After the reaction was completed, the reaction mixture was concentrated under reduced pressure and subjected to reversed-phase preparative chromatography (gradient elution with aqueous hydrochloric acid: acetonitrile) to obtain the title compound. MS m/z (ESI): 441.21 [M+1].

### Example 96 Synthesis of Compound 22-1: tert-Butyl 4-(((2S,4R)-4-ethoxy-2-(4-(methoxycarbonyl)phenyl)piperidin-1-yl)fluoromethyl)-5-methoxy-7-methyl-1H-indole-1-carboxylate

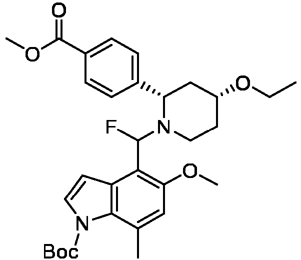

Intermediate 2-3 was added to a 10 mL three-necked flask, dissolved in dichloromethane, followed by the addition of triethylamine and then intermediate 13-4. The reaction was carried out overnight at room temperature. After the reaction was completed, the reaction was quenched with saturated ammonium chloride solution. The organic phase was concentrated under reduced pressure and subjected to column chromatography to obtain the title compound. MS m/z (ESI): 555.30 [M+1].

### Example 97 Synthesis of Compound 22: 4-((2S,4R)-4-Ethoxy-1-(fluoro(5-methoxy-7-methyl-1H-indol-4-yl)methyl)piperidin-2-yl)benzoic acid

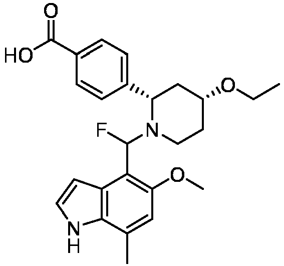

Compound 22-1 was placed in a 10 mL three-necked flask, dissolved in tetrahydrofuran and methanol, followed by the addition of a 1 M aqueous lithium hydroxide solution. The mixture was heated to 45°C and maintained at this temperature overnight. After the reaction was completed, the reaction mixture was concentrated under reduced pressure and subjected to reversed-phase preparative chromatography (gradient elution with aqueous hydrochloric acid: acetonitrile) to obtain the title compound. MS m/z (ESI): 441.21 [M+1].

### Example 98 Synthesis of Compound 23-1: tert-Butyl 4-(fluoro((2S,4S)-2-(4-(methoxycarbonyl)phenyl)-4-(2,2,2-trifluoroethoxy)piperidin-1-yl)methyl)-5-methoxy-7-methyl-1H-indole-1-carboxylate

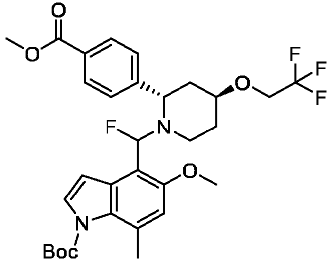

Intermediate 3-2 was added to a 10 mL three-necked flask, dissolved in dichloromethane, followed by the addition of triethylamine and then intermediate 13-4. The reaction was carried out overnight at room temperature. After the reaction was completed, the reaction was quenched with saturated ammonium chloride solution. The organic phase was concentrated under reduced pressure and subjected to column chromatography to obtain the title compound. MS m/z (ESI): 609.30 [M+1].

### Example 99 Synthesis of Compound 23: 4-((2S,4S)-1-(Fluoro(5-methoxy-7-methyl-1H-indol-4-yl)methyl)-4-(2,2,2-trifluoroethoxy)piperidin-2-yl)benzoic acid

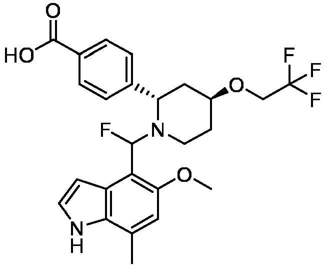

Compound 23-1 was placed in a 10 mL three-necked flask, dissolved in tetrahydrofuran and methanol, followed by the addition of a 1 M aqueous lithium hydroxide solution. The mixture was heated to 45°C and maintained at this temperature overnight. After the reaction was completed, the reaction mixture was concentrated under reduced pressure and subjected to reversed-phase preparative chromatography (gradient elution with aqueous hydrochloric acid: acetonitrile) to obtain the title compound. MS m/z (ESI): 495.21 [M+1].

### Example 100 Synthesis of Compound 24-1: tert-Butyl 4-(fluoro((2S,4R)-2-(4-(methoxycarbonyl)phenyl)-4-(2,2,2-trifluoroethoxy)piperidin-1-yl)methyl)-5-methoxy-7-methyl-1H-indole-1-carboxylate

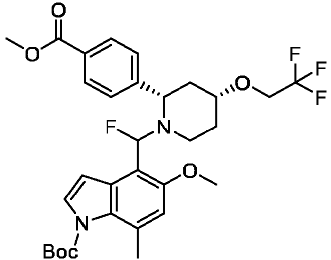

Intermediate 4-2 was added to a 10 mL three-necked flask, dissolved in dichloromethane, followed by the addition of triethylamine and then intermediate 13-4. The reaction was carried out overnight at room temperature. After the reaction was completed, the reaction was quenched with saturated ammonium chloride solution. The organic phase was concentrated under reduced pressure and subjected to column chromatography to obtain the title compound. MS m/z (ESI): 609.30 [M+1].

### Example 101 Synthesis of Compound 24: 4-((2S,4R)-1-(Fluoro(5-methoxy-7-methyl-1H-indol-4-yl)methyl)-4-(2,2,2-trifluoroethoxy)piperidin-2-yl)benzoic acid

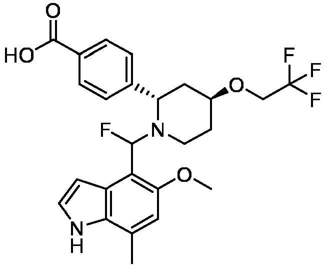

Compound 24-1 was placed in a 10 mL three-necked flask, dissolved in tetrahydrofuran and methanol, followed by the addition of a 1 M aqueous lithium hydroxide solution. The mixture was heated to 45°C and maintained at this temperature overnight. After the reaction was completed, the reaction mixture was concentrated under reduced pressure and subjected to reversed-phase preparative chromatography (gradient elution with aqueous hydrochloric acid: acetonitrile) to obtain the title compound. MS m/z (ESI): 495.21 [M+1].

### Example 102 Synthesis of Compound 25-1: tert-Butyl 4-(((2S,4S)-4-(2,2-difluoroethoxy)-2-(4-(methoxycarbonyl)phenyl)piperidin-1-yl)fluoromethyl)-5-methoxy-7-methyl-1H-indole-1-carboxylate

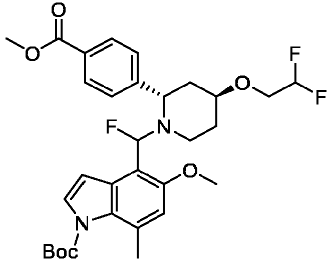

Intermediate 5-2 was added to a 10 mL three-necked flask, dissolved in dichloromethane, followed by the addition of triethylamine and then intermediate 13-4. The reaction was carried out overnight at room temperature. After the reaction was completed, the reaction was quenched with saturated ammonium chloride solution. The organic phase was concentrated under reduced pressure and subjected to column chromatography to obtain the title compound. MS m/z (ESI): 591.30 [M+1].

### Example 103 Synthesis of Compound 25: 4-((2S,4S)-4-(2,2-Difluoroethoxy)-1-(fluoro(5-methoxy-7-methyl-1H-indol-4-yl)methyl)piperidin-2-yl)benzoic acid

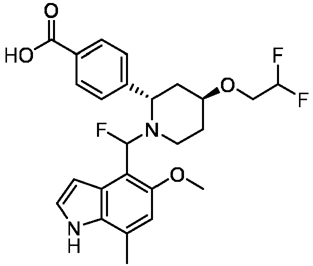

Compound 25-1 was placed in a 10 mL three-necked flask, dissolved in tetrahydrofuran and methanol, followed by the addition of a 1 M aqueous lithium hydroxide solution. The mixture was heated to 45°C and maintained at this temperature overnight. After the reaction was completed, the reaction mixture was concentrated under reduced pressure and subjected to reversed-phase preparative chromatography (gradient elution with aqueous hydrochloric acid: acetonitrile) to obtain the title compound. MS m/z (ESI): 477.21 [M+1].

### Example 104 Synthesis of Compound 26-1: tert-Butyl 4-(((2S,4R)-4-(2,2-difluoroethoxy)-2-(4-(methoxycarbonyl)phenyl)piperidin-1-yl)fluoromethyl)-5-methoxy-7-methyl-1H-indole-1-carboxylate

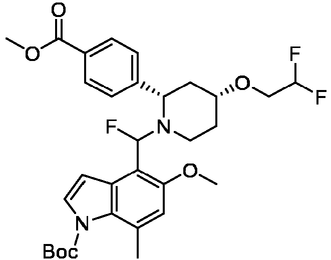

Intermediate 6-2 was added to a 10 mL three-necked flask, dissolved in dichloromethane, followed by the addition of triethylamine and then intermediate 13-4. The reaction was carried out overnight at room temperature. After the reaction was completed, the reaction was quenched with saturated ammonium chloride solution. The organic phase was concentrated under reduced pressure and subjected to column chromatography to obtain the title compound. MS m/z (ESI): 591.30 [M+1].

### Example 105 Synthesis of Compound 26: 4-((2S,4S)-4-(2,2-Difluoroethoxy)-1-(fluoro(5-methoxy-7-methyl-1H-indol-4-yl)methyl)piperidin-2-yl)benzoic acid

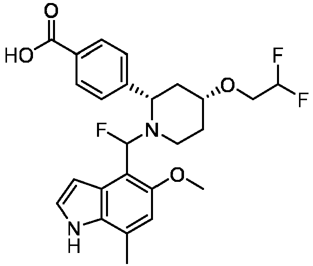

Compound 26-1 was placed in a 10 mL three-necked flask, dissolved in tetrahydrofuran and methanol, followed by the addition of a 1 M aqueous lithium hydroxide solution. The mixture was heated to 45°C and maintained at this temperature overnight. After the reaction was completed, the reaction mixture was concentrated under reduced pressure and subjected to reversed-phase preparative chromatography (gradient elution with aqueous hydrochloric acid: acetonitrile) to obtain the title compound. MS m/z (ESI): 477.21 [M+1].

### Example 106 Synthesis of Compound 27-1: tert-Butyl 4-(fluoro((2S,4S)-4-(2-fluoroethoxy)-2-(4-(methoxycarbonyl)phenyl)piperidin-1-yl)methyl)-5-methoxy-7-methyl-1H-indole-1-carboxylate

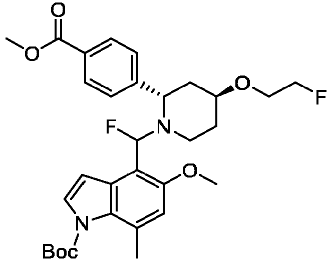

Intermediate 7-2 was added to a 10 mL three-necked flask, dissolved in dichloromethane, followed by the addition of triethylamine and then intermediate 13-4. The reaction was carried out overnight at room temperature. After the reaction was completed, the reaction was quenched with saturated ammonium chloride solution. The organic phase was concentrated under reduced pressure and subjected to column chromatography to obtain the title compound. MS m/z (ESI): 573.30 [M+1].

### Example 107 Synthesis of Compound 27: 4-((2S,4S)-1-(Fluoro(5-methoxy-7-methyl-1H-indol-4-yl)methyl)-4-(2-fluoroethoxy)piperidin-2-yl)benzoic acid

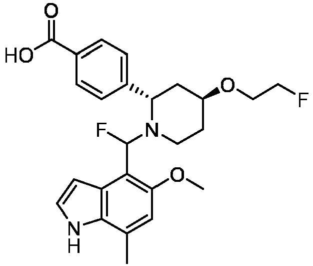

Compound 27-1 was placed in a 10 mL three-necked flask, dissolved in tetrahydrofuran and methanol, followed by the addition of a 1 M aqueous lithium hydroxide solution. The mixture was heated to 45°C and maintained at this temperature overnight. After the reaction was completed, the reaction mixture was concentrated under reduced pressure and subjected to reversed-phase preparative chromatography (gradient elution with aqueous hydrochloric acid: acetonitrile) to obtain the title compound. MS m/z (ESI): 459.21 [M+1].

### Example 108 Synthesis of Compound 28-1: tert-Butyl 4-(fluoro((2S,4R)-4-(2-fluoroethoxy)-2-(4-(methoxycarbonyl)phenyl)piperidin-1-yl)methyl)-5-methoxy-7-methyl-1H-indole-1-carboxylate

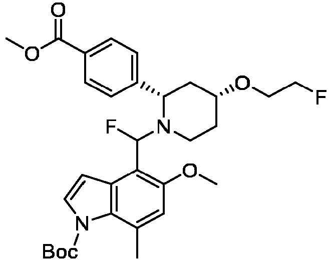

Intermediate 8-2 was added to a 10 mL three-necked flask, dissolved in dichloromethane, followed by the addition of triethylamine and then intermediate 13-4. The reaction was carried out overnight at room temperature. After the reaction was completed, the reaction was quenched with saturated ammonium chloride solution. The organic phase was concentrated under reduced pressure and subjected to column chromatography to obtain the title compound. MS m/z (ESI): 573.30 [M+1].

### Example 109 Synthesis of Compound 28: 4-((2S,4R)-1-(Fluoro(5-methoxy-7-methyl-1H-indol-4-yl)methyl)-4-(2-fluoroethoxy)piperidin-2-yl)benzoic acid

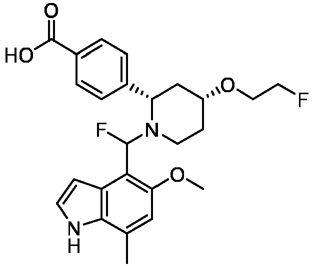

Compound 28-1 was placed in a 10 mL three-necked flask, dissolved in tetrahydrofuran and methanol, followed by the addition of a 1 M aqueous lithium hydroxide solution. The mixture was heated to 45°C and maintained at this temperature overnight. After the reaction was completed, the reaction mixture was concentrated under reduced pressure and subjected to reversed-phase preparative chromatography (gradient elution with aqueous hydrochloric acid: acetonitrile) to obtain the title compound. MS m/z (ESI): 459.21 [M+1].

### Example 110 Synthesis of Compound 29-1: tert-Butyl 4-((2S,4S)-4-(cyclopropylmethoxy)-2-(4-(methoxycarbonyl)phenyl)piperidin-1-yl)fluoromethyl)-5-methoxy-7-methyl-1H-indole-1-carboxylate

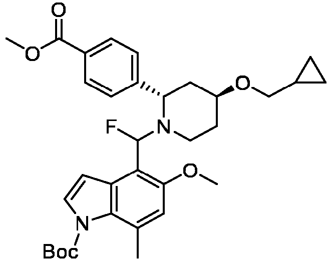

Intermediate 9-2 was added to a 10 mL three-necked flask, dissolved in dichloromethane, followed by the addition of triethylamine and then intermediate 13-4. The reaction was carried out overnight at room temperature. After the reaction was completed, the reaction was quenched with saturated ammonium chloride solution. The organic phase was concentrated under reduced pressure and subjected to column chromatography to obtain the title compound. MS m/z (ESI): 581.30 [M+1].

### Example 111 Synthesis of Compound 29: 4-((2S,4S)-4-(Cyclopropylmethoxy)-1-(fluoro(5-methoxy-7-methyl-1H-indol-4-yl)methyl)piperidin-2-yl)benzoic acid

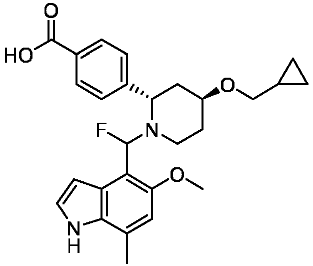

Compound 29-1 was placed in a 10 mL three-necked flask, dissolved in tetrahydrofuran and methanol, followed by the addition of a 1 M aqueous lithium hydroxide solution. The mixture was heated to 45°C and maintained at this temperature overnight. After the reaction was completed, the reaction mixture was concentrated under reduced pressure and subjected to reversed-phase preparative chromatography (gradient elution with aqueous hydrochloric acid: acetonitrile) to obtain the title compound. MS m/z (ESI): 467.21 [M+1].

### Example 112 Synthesis of Compound 30-1: tert-Butyl 4-((2S,4R)-4-(cyclopropylmethoxy)-2-(4-(methoxycarbonyl)phenyl)piperidin-1-yl)fluoromethyl)-5-methoxy-7-methyl-1H-indole-1-carboxylate

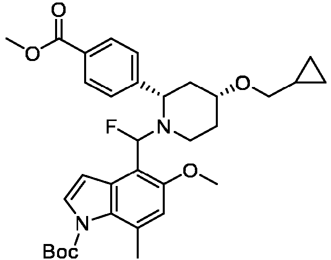

Intermediate 10-2 was added to a 10 mL three-necked flask, dissolved in dichloromethane, followed by the addition of triethylamine and then intermediate 13-4. The reaction was carried out overnight at room temperature. After the reaction was completed, the reaction was quenched with saturated ammonium chloride solution. The organic phase was concentrated under reduced pressure and subjected to column chromatography to obtain the title compound. MS m/z (ESI): 581.30 [M+1].

### Example 113 Synthesis of Compound 30: 4-((2S,4R)-4-(Cyclopropylmethoxy)-1-(fluoro(5-methoxy-7-methyl-1H-indol-4-yl)methyl)piperidin-2-yl)benzoic acid

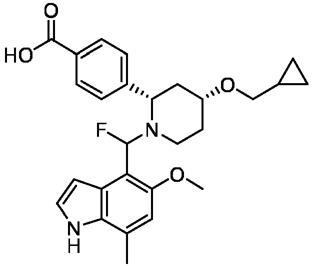

Compound 30-1 was placed in a 10 mL three-necked flask, dissolved in tetrahydrofuran and methanol, followed by the addition of a 1 M aqueous lithium hydroxide solution. The mixture was heated to 45°C and maintained at this temperature overnight. After the reaction was completed, the reaction mixture was concentrated under reduced pressure and subjected to reversed-phase preparative chromatography (gradient elution with aqueous hydrochloric acid: acetonitrile) to obtain the title compound. MS m/z (ESI): 467.21 [M+1].

### Example 114 Synthesis of Compound 31-1: tert-Butyl 4-(((2S,4S)-4-ethoxy-2-(4-(methoxycarbonyl)phenyl)piperidin-1-yl)difluoromethyl)-5-methoxy-7-methyl-1H-indole-1-carboxylate

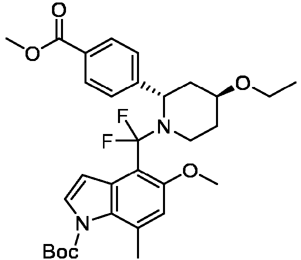

Intermediate 1-5 was added to a 10 mL three-necked flask, dissolved in dichloromethane, followed by the addition of triethylamine and then intermediate 14-2. The reaction was carried out overnight at room temperature. After the reaction was completed, the reaction was quenched with saturated ammonium chloride solution. The organic phase was concentrated under reduced pressure and subjected to column chromatography to obtain the title compound. MS m/z (ESI): 573.30 [M+1].

### Example 115 Synthesis of Compound 31: 4-((2S,4S)-1-(Difluoro(5-methoxy-7-methyl-1H-indol-4-yl)methyl)-4-ethoxypiperidin-2-yl)benzoic acid

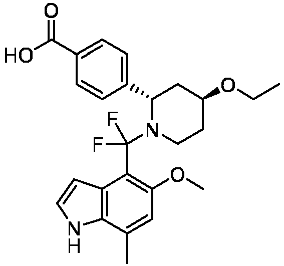

Compound 31-1 was placed in a 10 mL three-necked flask, dissolved in tetrahydrofuran and methanol, followed by the addition of a 1 M aqueous lithium hydroxide solution. The mixture was heated to 45°C and maintained at this temperature overnight. After the reaction was completed, the reaction mixture was concentrated under reduced pressure and subjected to reversed-phase preparative chromatography (gradient elution with aqueous hydrochloric acid: acetonitrile) to obtain the title compound. MS m/z (ESI): 459.21 [M+1].

### Example 116 Synthesis of Compound 32-1: tert-Butyl 4-(((2S,4R)-4-ethoxy-2-(4-(methoxycarbonyl)phenyl)piperidin-1-yl)difluoromethyl)-5-methoxy-7-methyl-1H-indole-1-carboxylate

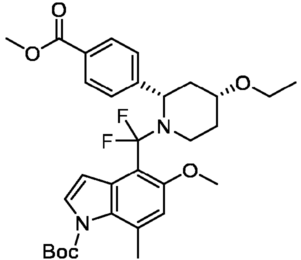

Intermediate 2-3 was added to a 10 mL three-necked flask, dissolved in dichloromethane, followed by the addition of triethylamine and then intermediate 14-2. The reaction was carried out overnight at room temperature. After the reaction was completed, the reaction was quenched with saturated ammonium chloride solution. The organic phase was concentrated under reduced pressure and subjected to column chromatography to obtain the title compound. MS m/z (ESI): 573.30 [M+1].

### Example 117 Synthesis of Compound 32: 4-((2S,4R)-1-(Difluoro(5-methoxy-7-methyl-1H-indol-4-yl)methyl)-4-ethoxypiperidin-2-yl)benzoic acid

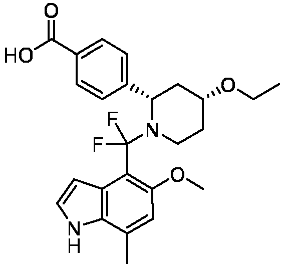

Compound 32-1 was placed in a 10 mL three-necked flask, dissolved in tetrahydrofuran and methanol, followed by the addition of a 1 M aqueous lithium hydroxide solution. The mixture was heated to 45°C and maintained at this temperature overnight. After the reaction was completed, the reaction mixture was concentrated under reduced pressure and subjected to reversed-phase preparative chromatography (gradient elution with aqueous hydrochloric acid: acetonitrile) to obtain the title compound. MS m/z (ESI): 459.21 [M+1].

### Example 118 Synthesis of Compound 33-1: tert-Butyl 4-(difluoro((2S,4S)-2-(4-(methoxycarbonyl)phenyl)-4-(2,2,2-trifluoroethoxy)piperidin-1-yl)methyl)-5-methoxy-7-methyl-1H-indole-1-carboxylate

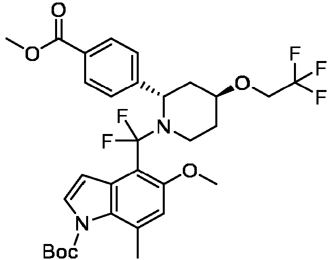

Intermediate 3-2 was added to a 10 mL three-necked flask, dissolved in dichloromethane, followed by the addition of triethylamine and then intermediate 14-2. The reaction was carried out overnight at room temperature. After the reaction was completed, the reaction was quenched with saturated ammonium chloride solution. The organic phase was concentrated under reduced pressure and subjected to column chromatography to obtain the title compound. MS m/z (ESI): 627.30 [M+1].

### Example 119 Synthesis of Compound 33: 4-((2S,4S)-1-(Difluoro(5-methoxy-7-methyl-1H-indol-4-yl)methyl)-4-(2,2,2-trifluoroethoxy)piperidin-2-yl)benzoic acid

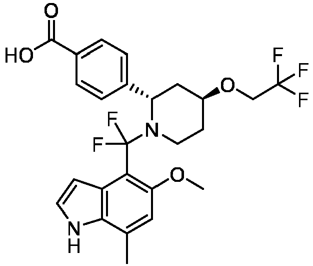

Compound 33-1 was placed in a 10 mL three-necked flask, dissolved in tetrahydrofuran and methanol, followed by the addition of a 1 M aqueous lithium hydroxide solution. The mixture was heated to 45°C and maintained at this temperature overnight. After the reaction was completed, the reaction mixture was concentrated under reduced pressure and subjected to reversed-phase preparative chromatography (gradient elution with aqueous hydrochloric acid: acetonitrile) to obtain the title compound. MS m/z (ESI): 513.21 [M+1].

### Example 120 Synthesis of Compound 34-1: tert-Butyl 4-(difluoro((2S,4R)-2-(4-(methoxycarbonyl)phenyl)-4-(2,2,2-trifluoroethoxy)piperidin-1-yl)methyl)-5-methoxy-7-methyl-1H-indole-1-carboxylate

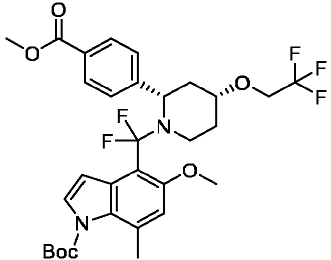

Intermediate 4-2 was added to a 10 mL three-necked flask, dissolved in dichloromethane, followed by the addition of triethylamine and then intermediate 14-2. The reaction was carried out overnight at room temperature. After the reaction was completed, the reaction was quenched with saturated ammonium chloride solution. The organic phase was concentrated under reduced pressure and subjected to column chromatography to obtain the title compound. MS m/z (ESI): 627.30 [M+1].

### Example 121 Synthesis of Compound 34: 4-((2S,4R)-1-(Difluoro(5-methoxy-7-methyl-1H-indol-4-yl)methyl)-4-(2,2,2-trifluoroethoxy)piperidin-2-yl)benzoic acid

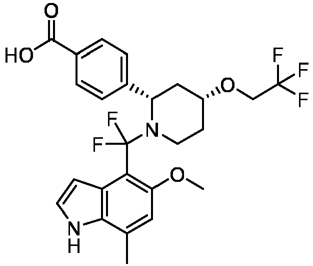

Compound 34-1 was placed in a 10 mL three-necked flask, dissolved in tetrahydrofuran and methanol, followed by the addition of a 1 M aqueous lithium hydroxide solution. The mixture was heated to 45°C and maintained at this temperature overnight. After the reaction was completed, the reaction mixture was concentrated under reduced pressure and subjected to reversed-phase preparative chromatography (gradient elution with aqueous hydrochloric acid: acetonitrile) to obtain the title compound. MS m/z (ESI): 513.21 [M+1].

### Example 122 Synthesis of Compound 35-1: tert-Butyl 4-(((2S,4S)-4-(2,2-difluoroethoxy)-2-(4-(methoxycarbonyl)phenyl)piperidin-1-yl)difluoromethyl)-5-methoxy-7-methyl-1H-indole-1-carboxylate

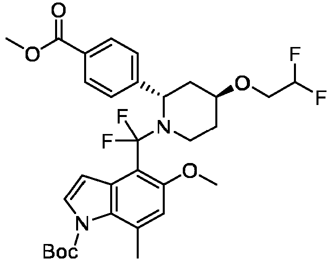

Intermediate 5-2 was added to a 10 mL three-necked flask, dissolved in dichloromethane, followed by the addition of triethylamine and then intermediate 14-2. The reaction was carried out overnight at room temperature. After the reaction was completed, the reaction was quenched with saturated ammonium chloride solution. The organic phase was concentrated under reduced pressure and subjected to column chromatography to obtain the title compound. MS m/z (ESI): 609.30 [M+1].

### Example 123 Synthesis of Compound 35: 4-((2S,4S)-1-(Difluoro(5-methoxy-7-methyl-1H-indol-4-yl)methyl)-4-(2,2-difluoroethoxy)piperidin-2-yl)benzoic acid

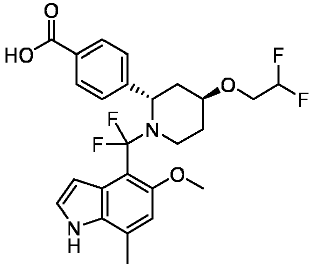

Compound 35-1 was placed in a 10 mL three-necked flask, dissolved in tetrahydrofuran and methanol, followed by the addition of a 1 M aqueous lithium hydroxide solution. The mixture was heated to 45°C and maintained at this temperature overnight. After the reaction was completed, the reaction mixture was concentrated under reduced pressure and subjected to reversed-phase preparative chromatography (gradient elution with aqueous hydrochloric acid: acetonitrile) to obtain the title compound. MS m/z (ESI): 495.21 [M+1].

### Example 124 Synthesis of Compound 36-1: tert-Butyl 4-(((2S,4R)-4-(2,2-difluoroethoxy)-2-(4-(methoxycarbonyl)phenyl)piperidin-1-yl)difluoromethyl)-5-methoxy-7-methyl-1H-indole-1-carboxylate

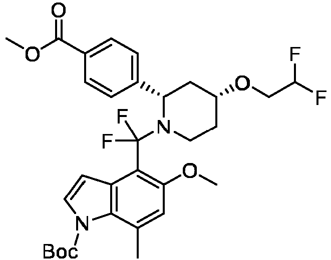

Intermediate 6-2 was added to a 10 mL three-necked flask, dissolved in dichloromethane, followed by the addition of triethylamine and then intermediate 14-2. The reaction was carried out overnight at room temperature. After the reaction was completed, the reaction was quenched with saturated ammonium chloride solution. The organic phase was concentrated under reduced pressure and subjected to column chromatography to obtain the title compound. MS m/z (ESI): 609.30 [M+1].

### Example 125 Synthesis of Compound 36: 4-((2S,4R)-1-(Difluoro(5-methoxy-7-methyl-1H-indol-4-yl)methyl)-4-(2,2-difluoroethoxy)piperidin-2-yl)benzoic acid

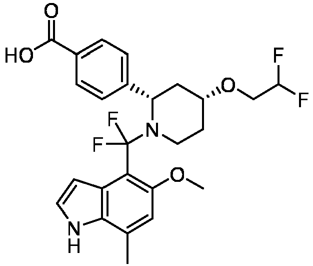

Compound 36-1 was placed in a 10 mL three-necked flask, dissolved in tetrahydrofuran and methanol, followed by the addition of a 1 M aqueous lithium hydroxide solution. The mixture was heated to 45°C and maintained at this temperature overnight. After the reaction was completed, the reaction mixture was concentrated under reduced pressure and subjected to reversed-phase preparative chromatography (gradient elution with aqueous hydrochloric acid: acetonitrile) to obtain the title compound. MS m/z (ESI): 495.21 [M+1].

### Example 126 Synthesis of Compound 37-1: tert-Butyl 4-(difluoro((2S,4S)-4-(2-fluoroethoxy)-2-(4-(methoxycarbonyl)phenyl)piperidin-1-yl)methyl)-5-methoxy-7-methyl-1H-indole-1-carboxylate

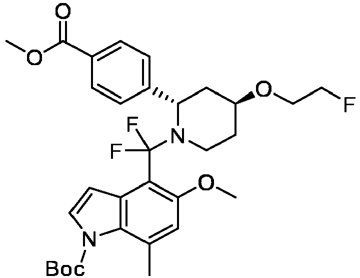

Intermediate 7-2 was added to a 10 mL three-necked flask, dissolved in dichloromethane, followed by the addition of triethylamine and then intermediate 14-2. The reaction was carried out overnight at room temperature. After the reaction was completed, the reaction was quenched with saturated ammonium chloride solution. The organic phase was concentrated under reduced pressure and subjected to column chromatography to obtain the title compound. MS m/z (ESI): 591.30 [M+1].

### Example 127 Synthesis of Compound 37: 4-((2S,4S)-1-(Difluoro(5-methoxy-7-methyl-1H-indol-4-yl)methyl)-4-(2-fluoroethoxy)piperidin-2-yl)benzoic acid

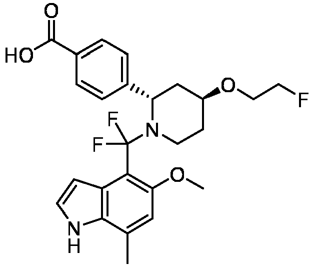

Compound 37-1 was placed in a 10 mL three-necked flask, dissolved in tetrahydrofuran and methanol, followed by the addition of a 1 M aqueous lithium hydroxide solution. The mixture was heated to 45°C and maintained at this temperature overnight. After the reaction was completed, the reaction mixture was concentrated under reduced pressure and subjected to reversed-phase preparative chromatography (gradient elution with aqueous hydrochloric acid: acetonitrile) to obtain the title compound. MS m/z (ESI): 477.21 [M+1].

### Example 128 Synthesis of Compound 38-1: tert-Butyl 4-(difluoro((2S,4R)-4-(2-fluoroethoxy)-2-(4-(methoxycarbonyl)phenyl)piperidin-1-yl)methyl)-5-methoxy-7-methyl-1H-indole-1-carboxylate

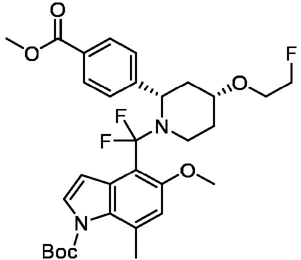

Intermediate 8-2 was added to a 10 mL three-necked flask, dissolved in dichloromethane, followed by the addition of triethylamine and then intermediate 14-2. The reaction was carried out overnight at room temperature. After the reaction was completed, the reaction was quenched with saturated ammonium chloride solution. The organic phase was concentrated under reduced pressure and subjected to column chromatography to obtain the title compound. MS m/z (ESI): 591.30 [M+1].

### Example 129 Synthesis of Compound 38: 4-((2S,4R)-1-(Difluoro(5-methoxy-7-methyl-1H-indol-4-yl)methyl)-4-(2-fluoroethoxy)piperidin-2-yl)benzoic acid

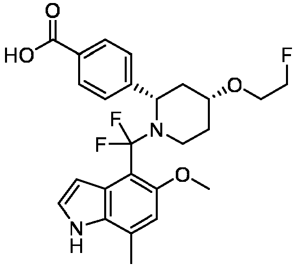

Compound 38-1 was placed in a 10 mL three-necked flask, dissolved in tetrahydrofuran and methanol, followed by the addition of a 1 M aqueous lithium hydroxide solution. The mixture was heated to 45°C and maintained at this temperature overnight. After the reaction was completed, the reaction mixture was concentrated under reduced pressure and subjected to reversed-phase preparative chromatography (gradient elution with aqueous hydrochloric acid: acetonitrile) to obtain the title compound. MS m/z (ESI): 477.21 [M+1].

### Example 130 Synthesis of Compound 39-1: tert-Butyl 4-(((2S,4S)-4-(cyclopropylmethoxy)-2-(4-(methoxycarbonyl)phenyl)piperidin-1-yl)difluoromethyl)-5-methoxy-7-methyl-1H-indole-1-carboxylate

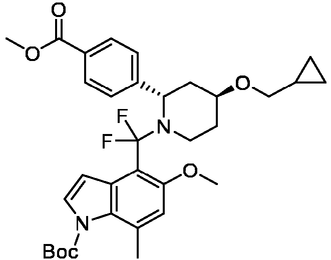

Intermediate 9-2 was added to a 10 mL three-necked flask, dissolved in dichloromethane, followed by the addition of triethylamine and then intermediate 14-2. The reaction was carried out overnight at room temperature. After the reaction was completed, the reaction was quenched with saturated ammonium chloride solution. The organic phase was concentrated under reduced pressure and subjected to column chromatography to obtain the title compound. MS m/z (ESI): 599.30 [M+1].

### Example 131 Synthesis of Compound 39: 4-((2S,4S)-4-(Cyclopropylmethoxy)-1-(fluoro(5-methoxy-7-methyl-1H-indol-4-yl)methyl)piperidin-2-yl)benzoic acid

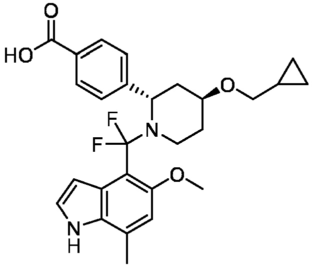

Compound 39-1 was placed in a 10 mL three-necked flask, dissolved in tetrahydrofuran and methanol, followed by the addition of a 1 M aqueous lithium hydroxide solution. The mixture was heated to 45°C and maintained at this temperature overnight. After the reaction was completed, the reaction mixture was concentrated under reduced pressure and subjected to reversed-phase preparative chromatography (gradient elution with aqueous hydrochloric acid: acetonitrile) to obtain the title compound. MS m/z (ESI): 485.21 [M+1].

### Example 132 Synthesis of Compound 40-1: tert-Butyl 4-(((2R,4R)-4-(cyclopropylmethoxy)-2-(4-(methoxycarbonyl)phenyl)piperidin-1-yl)difluoromethyl)-5-methoxy-7-methyl-1H-indole-1-carboxylate

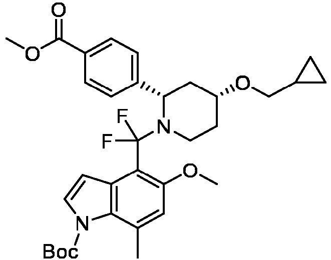

Intermediate 10-2 was added to a 10 mL three-necked flask, dissolved in dichloromethane, followed by the addition of triethylamine and then intermediate 14-2. The reaction was carried out overnight at room temperature. After the reaction was completed, the reaction was quenched with saturated ammonium chloride solution. The organic phase was concentrated under reduced pressure and subjected to column chromatography to obtain the title compound. MS m/z (ESI): 599.30 [M+1].

### Example 133 Synthesis of Compound 40: 4-((2S,4R)-4-(Cyclopropylmethoxy)-1-(fluoro(5-methoxy-7-methyl-1H-indol-4-yl)methyl)piperidin-2-yl)benzoic acid

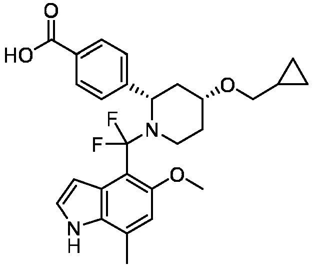

Compound 40-1 was placed in a 10 mL three-necked flask, dissolved in tetrahydrofuran and methanol, followed by the addition of a 1 M aqueous lithium hydroxide solution. The mixture was heated to 45°C and maintained at this temperature overnight. After the reaction was completed, the reaction mixture was concentrated under reduced pressure and subjected to reversed-phase preparative chromatography (gradient elution with aqueous hydrochloric acid: acetonitrile) to obtain the title compound. MS m/z (ESI): 485.21 [M+1].

### Example 134

### Synthesis of Intermediate 15-1:

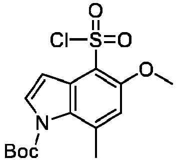

*tert-*Butyl 5-methoxy-7-methyl-1H-indole-1-carboxylate was placed in a 100 mL three-necked flask, and the internal temperature was lowered to 0°C. Dichloromethane was added for dissolution, followed by the dropwise addition of sulfonyl chloride. After the reaction was completed, the reaction was quenched with saturated brine. The mixture was then extracted with dichloromethane, washed three times with brine, and the organic phase was concentrated under reduced pressure and subjected to column chromatography to obtain the title compound.MS m/z (ESI): 360.10 [M+1].

### Synthesis of Intermediate 15-2

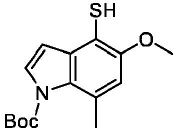

Intermediate 15-1 was placed in a 100 mL single-necked flask, dissolved in formic acid, followed by the addition of 10% wet palladium on carbon and triphenylphosphine. The reaction was carried out overnight for 12 hours. After the reaction was completed, the mixture was filtered, concentrated under reduced pressure, and subjected to column chromatography to obtain the title compound. MS m/z (ESI): 295.10 [M+1].

### Synthesis of Compound 41-1: tert-Butyl 4-(((2S,4S)-4-ethoxy-2-(4-(methoxycarbonyl)phenyl)piperidin-1-yl)(methyl)amino)-5-methoxy-7-methyl-1H-indole-1-carboxylate

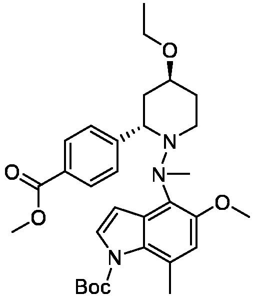

Intermediate 1-8 was placed in a 10 mL three-necked flask, and intermediate 12-2 solid was added, followed by the addition of 2-dicyclohexylphosphino-2',4',6'-triisopropylbiphenyl and tris(dibenzylideneacetone)dipalladium, and the final addition of sodium *tert*-butoxide solid. The mixture was dissolved in toluene, and the system was purged with nitrogen three times. The reaction was heated to 100°C and carried out overnight. After the reaction was completed, the reaction was quenched with a heated saturated aqueous ammonium chloride solution. The organic phase was concentrated under reduced pressure and subjected to column chromatography to obtain the title compound. MS m/z (ESI): 552.30 [M+1].

### Synthesis of Compound 41: 4-((2S,4S)-4-Ethoxy-1-((5-methoxy-7-methyl-1H-indol-4-yl)(methyl)amino)piperidin-2-yl)benzoic acid

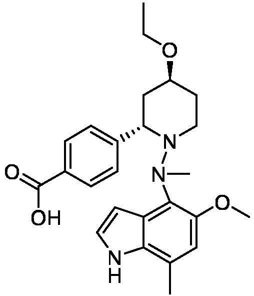

Compound 41-1 was placed in a 10 mL three-necked flask, dissolved in tetrahydrofuran and methanol, followed by the addition of a 1 M aqueous lithium hydroxide solution. The mixture was heated to 45°C and maintained at this temperature overnight. After the reaction was completed, the reaction mixture was concentrated under reduced pressure and subjected to reversed-phase preparative chromatography (gradient elution with aqueous hydrochloric acid: acetonitrile) to obtain the title compound. MS m/z (ESI): 438.21 [M+1].

### Example 135

### Synthesis of Compound 42-1: tert-Butyl 4-(((2S,4R)-4-ethoxy-2-(4-(methoxycarbonyl)phenyl)piperidin-1-yl)(methyl)amino)-5-methoxy-7-methyl-1H-indole-1-carboxylate

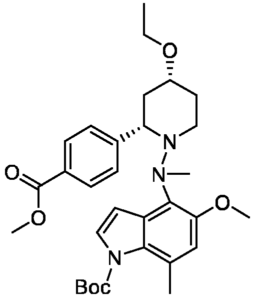

Intermediate 2-6 was placed in a 10 mL three-necked flask, and intermediate 12-2 solid was added, followed by the addition of 2-dicyclohexylphosphino-2',4',6'-triisopropylbiphenyl and tris(dibenzylideneacetone)dipalladium, and the final addition of sodium *tert*-butoxide solid. The mixture was dissolved in toluene, and the system was purged with nitrogen three times. The reaction was heated to 100°C and carried out overnight. After the reaction was completed, the reaction was quenched with a heated saturated aqueous ammonium chloride solution. The organic phase was concentrated under reduced pressure and subjected to column chromatography to obtain the title compound. MS m/z (ESI): 552.30 [M+1].

### Synthesis of Compound 42: 4-((2S,4R)-4-Ethoxy-1-((5-methoxy-7-methyl-1H-indol-4-yl)(methyl)amino)piperidin-2-yl)benzoic acid

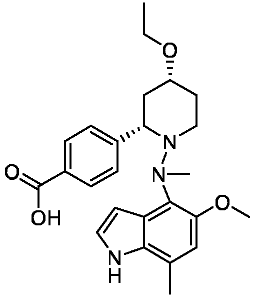

Compound 42-1 was placed in a 10 mL three-necked flask, dissolved in tetrahydrofuran and methanol, followed by the addition of a 1 M aqueous lithium hydroxide solution. The mixture was heated to 45°C and maintained at this temperature overnight. After the reaction was completed, the reaction mixture was concentrated under reduced pressure and subjected to reversed-phase preparative chromatography (gradient elution with aqueous hydrochloric acid: acetonitrile) to obtain the title compound. MS m/z (ESI): 438.21 [M+1].

### Example 136

### Synthesis of Compound 43-1: tert-Butyl 5-methoxy-4-(((2S,4S)-2-(4-(methoxycarbonyl)phenyl)-4-(2,2,2-trifluoroethoxy)piperidin-1-yl)(methyl)amino)-7-methyl-1H-indole-1-carboxylate

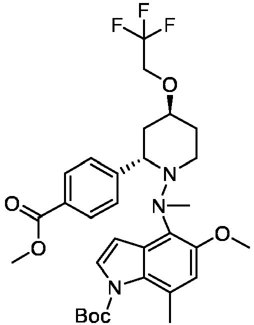

Intermediate 3-5 was placed in a 10 mL three-necked flask, and intermediate 12-2 solid was added, followed by the addition of 2-dicyclohexylphosphino-2',4',6'-triisopropylbiphenyl and tris(dibenzylideneacetone)dipalladium, and the final addition of sodium *tert*-butoxide solid. The mixture was dissolved in toluene, and the system was purged with nitrogen three times. The reaction was heated to 100°C and carried out overnight. After the reaction was completed, the reaction was quenched with a heated saturated aqueous ammonium chloride solution. The organic phase was concentrated under reduced pressure and subjected to column chromatography to obtain the title compound. MS m/z (ESI): 606.30 [M+1].

### Synthesis of Compound 43: 4-((2S,4S)-1-((5-Methoxy-7-methyl-1H-indol-4-yl)(methyl)amino)-4-(2,2,2-trifluoroethoxy)piperidin-2-yl)benzoic acid

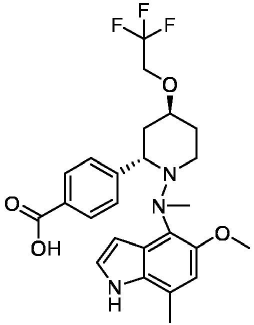

Compound 43-1 was placed in a 10 mL three-necked flask, dissolved in tetrahydrofuran and methanol, followed by the addition of a 1 M aqueous lithium hydroxide solution. The mixture was heated to 45°C and maintained at this temperature overnight. After the reaction was completed, the reaction mixture was concentrated under reduced pressure and subjected to reversed-phase preparative chromatography (gradient elution with aqueous hydrochloric acid: acetonitrile) to obtain the title compound. MS m/z (ESI): 492.21 [M+1].

### Example 137

### Synthesis of Compound 44-1: tert-Butyl 5-methoxy-4-(((2S,4R)-2-(4-(methoxycarbonyl)phenyl)-4-(2,2,2-trifluoroethoxy)piperidin-1-yl)(methyl)amino)-7-methyl-1H-indole-1-carboxylate

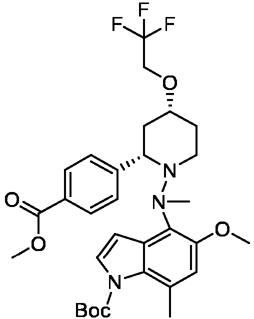

Intermediate 4-5 was placed in a 10 mL three-necked flask, and intermediate 12-2 solid was added, followed by the addition of 2-dicyclohexylphosphino-2',4',6'-triisopropylbiphenyl and tris(dibenzylideneacetone)dipalladium, and the final addition of sodium *tert*-butoxide solid. The mixture was dissolved in toluene, and the system was purged with nitrogen three times. The reaction was heated to 100°C and carried out overnight. After the reaction was completed, the reaction was quenched with a heated saturated aqueous ammonium chloride solution. The organic phase was concentrated under reduced pressure and subjected to column chromatography to obtain the title compound. MS m/z (ESI): 606.30 [M+1].

### Synthesis of Compound 44: 4-((2S,4R)-1-((5-Methoxy-7-methyl-1H-indol-4-yl)(methyl)amino)-4-(2,2,2-trifluoroethoxy)piperidin-2-yl)benzoic acid

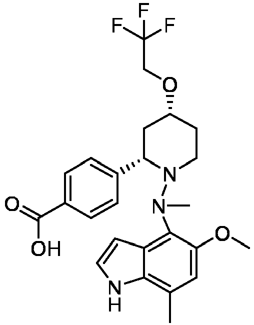

Compound 44-1 was placed in a 10 mL three-necked flask, dissolved in tetrahydrofuran and methanol, followed by the addition of a 1 M aqueous lithium hydroxide solution. The mixture was heated to 45°C and maintained at this temperature overnight. After the reaction was completed, the reaction mixture was concentrated under reduced pressure and subjected to reversed-phase preparative chromatography (gradient elution with aqueous hydrochloric acid: acetonitrile) to obtain the title compound. MS m/z (ESI): 492.21 [M+1].

### Example 138

### Synthesis of Compound 45-1: tert-Butyl 4-(((2S,4S)-4-(2,2-difluoroethoxy)-2-(4-(methoxycarbonyl)phenyl)piperidin-1-yl)(methyl)amino)-5-methoxy-7-methyl-1H-indole-1-carboxylate

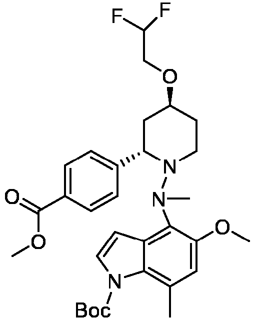

Intermediate 5-5 was placed in a 10 mL three-necked flask, and intermediate 12-2 solid was added, followed by the addition of 2-dicyclohexylphosphino-2',4',6'-triisopropylbiphenyl and tris(dibenzylideneacetone)dipalladium, and the final addition of sodium *tert*-butoxide solid. The mixture was dissolved in toluene, and the system was purged with nitrogen three times. The reaction was heated to 100°C and carried out overnight. After the reaction was completed, the reaction was quenched with a heated saturated aqueous ammonium chloride solution. The organic phase was concentrated under reduced pressure and subjected to column chromatography to obtain the title compound. MS m/z (ESI): 588.30 [M+1].

### Synthesis of Compound 45: 4-((2S,4S)-1-((5-Methoxy-7-methyl-1H-indol-4-yl)(methyl)amino)-4-(2,2-difluoroethoxy)piperidin-2-yl)benzoic acid

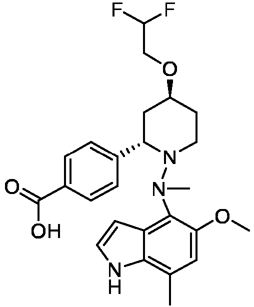

Compound 45-1 was placed in a 10 mL three-necked flask, dissolved in tetrahydrofuran and methanol, followed by the addition of a 1 M aqueous lithium hydroxide solution. The mixture was heated to 45°C and maintained at this temperature overnight. After the reaction was completed, the reaction mixture was concentrated under reduced pressure and subjected to reversed-phase preparative chromatography (gradient elution with aqueous hydrochloric acid: acetonitrile) to obtain the title compound. MS m/z (ESI): 474.21 [M+1].

### Example 139

### Synthesis of Compound 46-1: tert-Butyl 4-(((2S,4R)-4-(2,2-difluoroethoxy)-2-(4-(methoxycarbonyl)phenyl)piperidin-1-yl)(methyl)amino)-5-methoxy-7-methyl-1H-indole-1-carboxylate

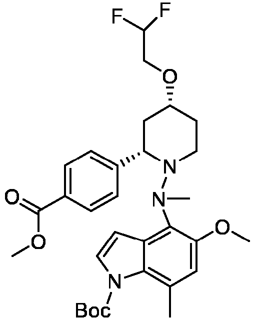

Intermediate 6-5 was placed in a 10 mL three-necked flask, and intermediate 12-2 solid was added, followed by the addition of 2-dicyclohexylphosphino-2',4',6'-triisopropylbiphenyl and tris(dibenzylideneacetone)dipalladium, and the final addition of sodium *tert*-butoxide solid. The mixture was dissolved in toluene, and the system was purged with nitrogen three times. The reaction was heated to 100°C and carried out overnight. After the reaction was completed, the reaction was quenched with a heated saturated aqueous ammonium chloride solution. The organic phase was concentrated under reduced pressure and subjected to column chromatography to obtain the title compound. MS m/z (ESI): 588.30 [M+1].

### Synthesis of Compound 46: 4-((2S,4R)-1-((5-Methoxy-7-methyl-1H-indol-4-yl)(methyl)amino)-4-(2,2-difluoroethoxy)piperidin-2-yl)benzoic acid

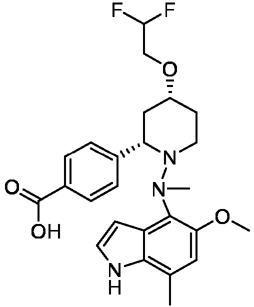

Compound 46-1 was placed in a 10 mL three-necked flask, dissolved in tetrahydrofuran and methanol, followed by the addition of a 1 M aqueous lithium hydroxide solution. The mixture was heated to 45°C and maintained at this temperature overnight. After the reaction was completed, the reaction mixture was concentrated under reduced pressure and subjected to reversed-phase preparative chromatography (gradient elution with aqueous hydrochloric acid: acetonitrile) to obtain the title compound. MS m/z (ESI): 474.21 [M+1].

### Example 140

### Synthesis of compound 47-1: tert-Butyl 4-(((2S,4S)-4-(2-fluoroethoxy)-2-(4-(methoxycarbonyl)phenyl)piperidin-1-yl)(methyl)amino)-5-methoxy-7-methyl-1H-indole-1-carboxylate

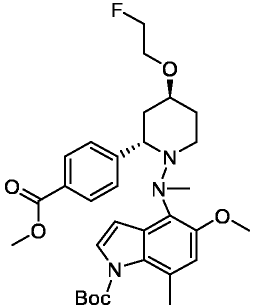

Intermediate 7-5 was placed in a 10 mL three-necked flask, and intermediate 12-2 solid was added, followed by the addition of 2-dicyclohexylphosphino-2',4',6'-triisopropylbiphenyl and tris(dibenzylideneacetone)dipalladium, and the final addition of sodium *tert-butoxide* solid. The mixture was dissolved in toluene, and the system was purged with nitrogen three times. The reaction was heated to 100°C and carried out overnight. After the reaction was completed, the reaction was quenched with a heated saturated aqueous ammonium chloride solution. The organic phase was concentrated under reduced pressure and subjected to column chromatography to obtain the title compound. MS m/z (ESI): 570.30 [M+1].

### Synthesis of Compound 47: 4-((2S,4S)-1-((5-Methoxy-7-methyl-1H-indol-4-yl)(methyl)amino)-4-(2-difluoroethoxy)piperidin-2-yl)benzoic acid

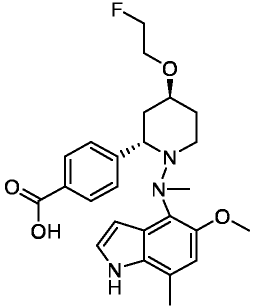

Compound 47-1 was placed in a 10 mL three-necked flask, dissolved in tetrahydrofuran and methanol, followed by the addition of a 1 M aqueous lithium hydroxide solution. The mixture was heated to 45°C and maintained at this temperature overnight. After the reaction was completed, the reaction mixture was concentrated under reduced pressure and subjected to reversed-phase preparative chromatography (gradient elution with aqueous hydrochloric acid: acetonitrile) to obtain the title compound. MS m/z (ESI): 456.21 [M+1].

### Example 141

### Synthesis of Compound 48-1: tert-Butyl 4-(((2S,4R)-4-(2-fluoroethoxy)-2-(4-(methoxycarbonyl)phenyl)piperidin-1-yl)(methyl)amino)-5-methoxy-7-methyl-1H-indole-1-carboxylate

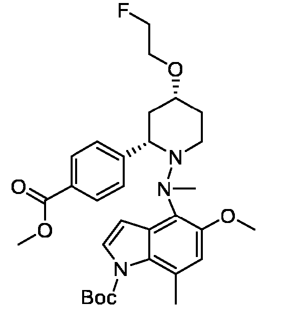

Intermediate 8-5 was placed in a 10 mL three-necked flask, and intermediate 12-2 solid was added, followed by the addition of 2-dicyclohexylphosphino-2',4',6'-triisopropylbiphenyl and tris(dibenzylideneacetone)dipalladium, and the final addition of sodium *tert*-butoxide solid. The mixture was dissolved in toluene, and the system was purged with nitrogen three times. The reaction was heated to 100°C and carried out overnight. After the reaction was completed, the reaction was quenched with a heated saturated aqueous ammonium chloride solution. The organic phase was concentrated under reduced pressure and subjected to column chromatography to obtain the title compound. MS m/z (ESI): 570.30 [M+1].

### Synthesis of Compound 48: 4-((2S,4R)-1-((5-Methoxy-7-methyl-1H-indol-4-yl)(methyl)amino)-4-(2-difluoroethoxy)piperidin-2-yl)benzoic acid

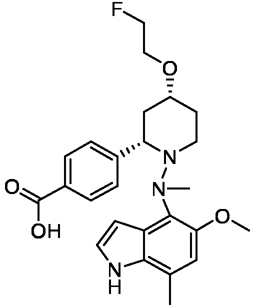

Compound 48-1 was placed in a 10 mL three-necked flask, dissolved in tetrahydrofuran and methanol, followed by the addition of a 1 M aqueous lithium hydroxide solution. The mixture was heated to 45°C and maintained at this temperature overnight. After the reaction was completed, the reaction mixture was concentrated under reduced pressure and subjected to reversed-phase preparative chromatography (gradient elution with aqueous hydrochloric acid: acetonitrile) to obtain the title compound. MS m/z (ESI): 456.21 [M+1].

### Example 142

### Synthesis of compound 49-1: tert-Butyl 4-(((2S,4S)-4-(cyclopropylmethoxy)-2-(4-(methoxycarbonyl)phenyl)piperidin-1-yl)(methyl)amino)-5-methoxy-7-methyl-1H-indole-1-carboxylate

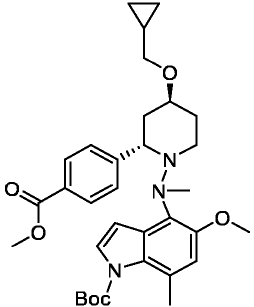

Intermediate 9-5 was placed in a 10 mL three-necked flask, and intermediate 12-2 solid was added, followed by the addition of 2-dicyclohexylphosphino-2',4',6'-triisopropylbiphenyl and tris(dibenzylideneacetone)dipalladium, and the final addition of sodium *tert*-butoxide solid. The mixture was dissolved in toluene, and the system was purged with nitrogen three times. The reaction was heated to 100°C and carried out overnight. After the reaction was completed, the reaction was quenched with a heated saturated aqueous ammonium chloride solution. The organic phase was concentrated under reduced pressure and subjected to column chromatography to obtain the title compound. MS m/z (ESI): 578.30 [M+1].

### Synthesis of Compound 49: 4-((2S,4S)-4-(Cyclopropylmethoxy)-1-((5-methoxy-7-methyl-1H-indol-4-yl)(methyl)amino)piperidin-2-yl)benzoic acid.

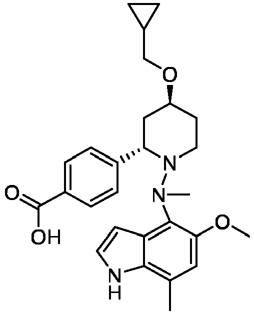

Compound 49-1 was placed in a 10 mL three-necked flask, dissolved in tetrahydrofuran and methanol, followed by the addition of a 1 M aqueous lithium hydroxide solution. The mixture was heated to 45°C and maintained at this temperature overnight. After the reaction was completed, the reaction mixture was concentrated under reduced pressure and subjected to reversed-phase preparative chromatography (gradient elution with aqueous hydrochloric acid: acetonitrile) to obtain the title compound. MS m/z (ESI): 464.21 [M+1].

### Example 143

### Synthesis of Compound 50-1: tert-Butyl 4-(((2S,4R)-4-(cyclopropylmethoxy)-2-(4-(methoxycarbonyl)phenyl)piperidin-1-yl)(methyl)amino)-5-methoxy-7-methyl-1H-indole-1-carboxylate

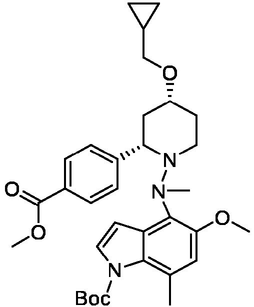

Intermediate 10-5 was placed in a 10 mL three-necked flask, and intermediate 12-2 solid was added, followed by the addition of 2-dicyclohexylphosphino-2',4',6'-triisopropylbiphenyl and tris(dibenzylideneacetone)dipalladium, and the final addition of sodium *tert*-butoxide solid. The mixture was dissolved in toluene, and the system was purged with nitrogen three times. The reaction was heated to 100°C and carried out overnight. After the reaction was completed, the reaction was quenched with a heated saturated aqueous ammonium chloride solution. The organic phase was concentrated under reduced pressure and subjected to column chromatography to obtain the title compound. MS m/z (ESI): 578.30 [M+1].

### Synthesis of Compound 50: 4-((2S,4R)-4-(Cyclopropylmethoxy)-1-((5-methoxy-7-methyl-1H-indol-4-yl)(methyl)amino)piperidin-2-yl)benzoic acid.

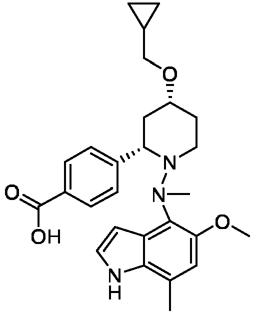

Compound 50-1 was placed in a 10 mL three-necked flask, dissolved in tetrahydrofuran and methanol, followed by the addition of a 1 M aqueous lithium hydroxide solution. The mixture was heated to 45°C and maintained at this temperature overnight. After the reaction was completed, the reaction mixture was concentrated under reduced pressure and subjected to reversed-phase preparative chromatography (gradient elution with aqueous hydrochloric acid: acetonitrile) to obtain the title compound. MS m/z (ESI): 464.21 [M+1].

### Example 144

### Synthesis of compound 51-1: tert-Butyl 4-(((2S,4S)-4-ethoxy-2-(4-(methoxycarbonyl)phenyl)piperidin-1-yl)sulfonyl)-5-methoxy-7-methyl-1H-indole-1-carboxylate

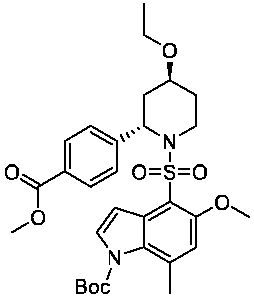

Intermediate 1-5 was added to a 10 mL three-necked flask, dissolved in dichloromethane, followed by the addition of triethylamine and then intermediate 15-1. The reaction was carried out overnight at room temperature. After the reaction was completed, the reaction was quenched with saturated ammonium chloride solution. The organic phase was concentrated under reduced pressure and subjected to column chromatography to obtain the title compound. MS m/z (ESI): 555.30 [M+1].

### Synthesis of Compound 51: 4-((2S,4S)-4-Ethoxy-1-((5-methoxy-7-methyl-1H-indol-4-yl)sulfonyl)piperidin-2-yl)benzoic acid

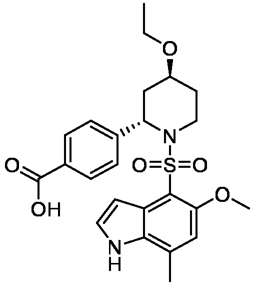

Compound 51-1 was placed in a 10 mL three-necked flask, dissolved in tetrahydrofuran and methanol, followed by the addition of a 1 M aqueous lithium hydroxide solution. The mixture was heated to 45°C and maintained at this temperature overnight. After the reaction was completed, the reaction mixture was concentrated under reduced pressure and subjected to reversed-phase preparative chromatography (gradient elution with aqueous hydrochloric acid: acetonitrile) to obtain the title compound. MS m/z (ESI): 474.21 [M+1].

### Example 145

### Synthesis of compound 52-1: tert-Butyl 4-(((2S,4R)-4-ethoxy-2-(4-(methoxycarbonyl)phenyl)piperidin-1-yl)sulfonyl)-5-methoxy-7-methyl-1H-indole-1-carboxylate

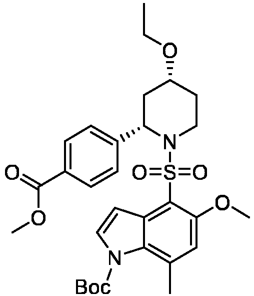

Intermediate 2-3 was added to a 10 mL three-necked flask, dissolved in dichloromethane, followed by the addition of triethylamine and then intermediate 15-1. The reaction was carried out overnight at room temperature. After the reaction was completed, the reaction was quenched with saturated ammonium chloride solution. The organic phase was concentrated under reduced pressure and subjected to column chromatography to obtain the title compound. MS m/z (ESI): 555.30 [M+1].

### Synthesis of Compound 52: 4-((2S,4R)-4-Ethoxy-1-((5-methoxy-7-methyl-1H-indol-4-yl)sulfonyl)piperidin-2-yl)benzoic acid

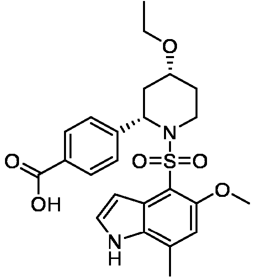

Compound 52-1 was placed in a 10 mL three-necked flask, dissolved in tetrahydrofuran and methanol, followed by the addition of a 1 M aqueous lithium hydroxide solution. The mixture was heated to 45°C and maintained at this temperature overnight. After the reaction was completed, the reaction mixture was concentrated under reduced pressure and subjected to reversed-phase preparative chromatography (gradient elution with aqueous hydrochloric acid: acetonitrile) to obtain the title compound. MS m/z (ESI): 474.21 [M+1].

### Example 146

### Synthesis of compound 53-1: tert-Butyl 5-methoxy-4-(((2S,4S)-2-(4-(methoxycarbonyl)phenyl)-4-(2,2,2-trifluoroethoxy)piperidin-1-yl)sulfonyl)-7-methyl-1H-indole-1-carboxylate

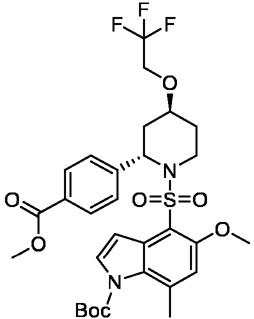

Intermediate 3-2 was added to a 10 mL three-necked flask, dissolved in dichloromethane, followed by the addition of triethylamine and then intermediate 15-1. The reaction was carried out overnight at room temperature. After the reaction was completed, the reaction was quenched with saturated ammonium chloride solution. The organic phase was concentrated under reduced pressure and subjected to column chromatography to obtain the title compound. MS m/z (ESI): 641.30 [M+1].

### Synthesis of Compound 53: 4-((2S,4S)-4-Ethoxy-1-((5-methoxy-7-methyl-1H-indol-4-yl)sulfonyl)piperidin-2-yl)benzoic acid

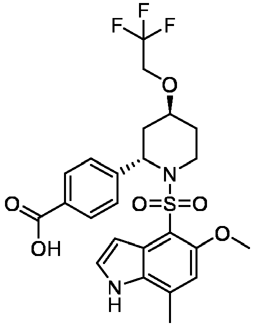

Compound 53-1 was placed in a 10 mL three-necked flask, dissolved in tetrahydrofuran and methanol, followed by the addition of a 1 M aqueous lithium hydroxide solution. The mixture was heated to 45°C and maintained at this temperature overnight. After the reaction was completed, the reaction mixture was concentrated under reduced pressure and subjected to reversed-phase preparative chromatography (gradient elution with aqueous hydrochloric acid: acetonitrile) to obtain the title compound. MS m/z (ESI): 527.21 [M+1].

### Example 147

### Synthesis of Compound 54-1: tert-Butyl 5-methoxy-4-(((2S,4R)-2-(4-(methoxycarbonyl)phenyl)-4-(2,2,2-trifluoroethoxy)piperidin-1-yl)sulfonyl)-7-methyl-1H-indole-1-carboxylate

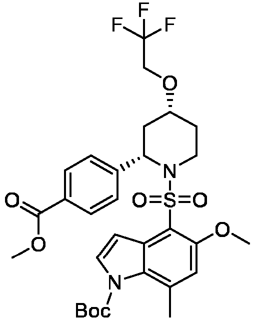

Intermediate 4-2 was added to a 10 mL three-necked flask, dissolved in dichloromethane, followed by the addition of triethylamine and then intermediate 15-1. The reaction was carried out overnight at room temperature. After the reaction was completed, the reaction was quenched with saturated ammonium chloride solution. The organic phase was concentrated under reduced pressure and subjected to column chromatography to obtain the title compound. MS m/z (ESI): 641.30 [M+1].

### Synthesis of Compound 54: 4-((2S,4R)-4-Ethoxy-1-((5-methoxy-7-methyl-1H-indol-4-yl)sulfonyl)piperidin-2-yl)benzoic acid

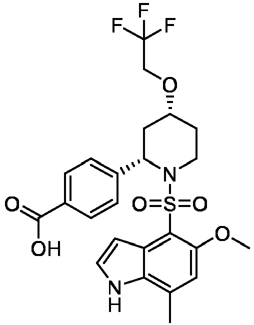

Compound 54-1 was placed in a 10 mL three-necked flask, dissolved in tetrahydrofuran and methanol, followed by the addition of a 1 M aqueous lithium hydroxide solution. The mixture was heated to 45°C and maintained at this temperature overnight. After the reaction was completed, the reaction mixture was concentrated under reduced pressure and subjected to reversed-phase preparative chromatography (gradient elution with aqueous hydrochloric acid: acetonitrile) to obtain the title compound. MS m/z (ESI): 527.21 [M+1].

### Example 148

### Synthesis of compound 55-1: tert-Butyl 5-methoxy-4-(((2S,4S)-2-(4-(methoxycarbonyl)phenyl)-4-(2,2-difluoroethoxy)piperidin-1-yl)sulfonyl)-7-methyl-1H-indole-1-carboxylate

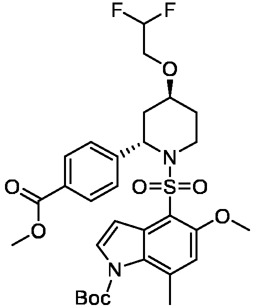

Intermediate 5-2 was added to a 10 mL three-necked flask, dissolved in dichloromethane, followed by the addition of triethylamine and then intermediate 15-1. The reaction was carried out overnight at room temperature. After the reaction was completed, the reaction was quenched with saturated ammonium chloride solution. The organic phase was concentrated under reduced pressure and subjected to column chromatography to obtain the title compound. MS m/z (ESI): 623.30 [M+1].

### Synthesis of Compound 55: tert-Butyl 5-methoxy-4-(((2S,4S)-2-(4-(methoxycarbonyl)phenyl)-4-(2,2-difluoroethoxy)piperidin-1-yl)sulfonyl)-7-methyl-1H-indole-1-carboxylate

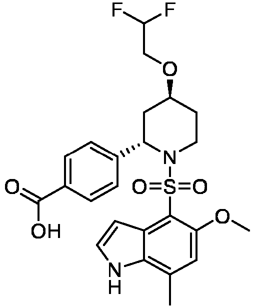

Compound 55-1 was placed in a 10 mL three-necked flask, dissolved in tetrahydrofuran and methanol, followed by the addition of a 1 M aqueous lithium hydroxide solution. The mixture was heated to 45°C and maintained at this temperature overnight. After the reaction was completed, the reaction mixture was concentrated under reduced pressure and subjected to reversed-phase preparative chromatography (gradient elution with aqueous hydrochloric acid: acetonitrile) to obtain the title compound. MS m/z (ESI): 509.21 [M+1].

### Example 149

### Synthesis of compound 56-1: tert-Butyl 5-methoxy-4-(((2S,4R)-2-(4-(methoxycarbonyl)phenyl)-4-(2,2-difluoroethoxy)piperidin-1-yl)sulfonyl)-7-methyl-1H-indole-1-carboxylate

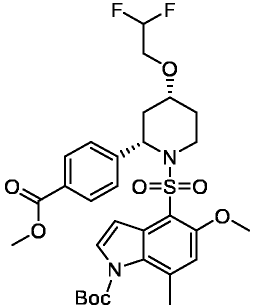

Intermediate 6-2 was added to a 10 mL three-necked flask, dissolved in dichloromethane, followed by the addition of triethylamine and then intermediate 15-1. The reaction was carried out overnight at room temperature. After the reaction was completed, the reaction was quenched with saturated ammonium chloride solution. The organic phase was concentrated under reduced pressure and subjected to column chromatography to obtain the title compound. MS m/z (ESI): 623.30 [M+1].

### Synthesis of Compound 56: tert-Butyl 5-methoxy-4-(((2S,4SR)-2-(4-(methoxycarbonyl)phenyl)-4-(2,2-difluoroethoxy)piperidin-1-yl)sulfonyl)-7-methyl-1H-indole-1-carboxylate

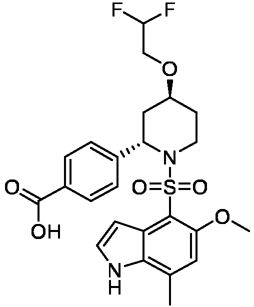

Compound 56-1 was placed in a 10 mL three-necked flask, dissolved in tetrahydrofuran and methanol, followed by the addition of a 1 M aqueous lithium hydroxide solution. The mixture was heated to 45°C and maintained at this temperature overnight. After the reaction was completed, the reaction mixture was concentrated under reduced pressure and subjected to reversed-phase preparative chromatography (gradient elution with aqueous hydrochloric acid: acetonitrile) to obtain the title compound. MS m/z (ESI): 509.21 [M+1].

### Example 150

### Synthesis of Compound 57-1: tert-Butyl 4-(((2S,4S)-4-(2-fluoroethoxy)-2-(4-(methoxycarbonyl)phenyl)piperidin-1-yl)sulfonyl)-5-methoxy-7-methyl-1H-indole-1-carboxylate

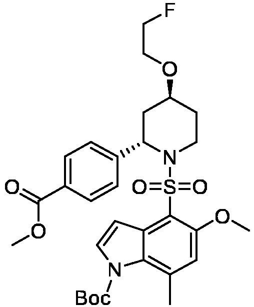

Intermediate 7-2 was added to a 10 mL three-necked flask, dissolved in dichloromethane, followed by the addition of triethylamine and then intermediate 15-1. The reaction was carried out overnight at room temperature. After the reaction was completed, the reaction was quenched with saturated ammonium chloride solution. The organic phase was concentrated under reduced pressure and subjected to column chromatography to obtain the title compound. MS m/z (ESI): 605.30 [M+1].

### Synthesis of Compound 57: 4-((2S,4S)-4-(2-Fluoroethoxy)-1-((5-methoxy-7-methyl-1H-indol-4-yl)sulfonyl)piperidin-2-yl)benzoic acid

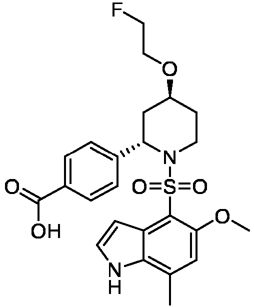

Compound 57-1 was placed in a 10 mL three-necked flask, dissolved in tetrahydrofuran and methanol, followed by the addition of a 1 M aqueous lithium hydroxide solution. The mixture was heated to 45°C and maintained at this temperature overnight. After the reaction was completed, the reaction mixture was concentrated under reduced pressure and subjected to reversed-phase preparative chromatography (gradient elution with aqueous hydrochloric acid: acetonitrile) to obtain the title compound. MS m/z (ESI): 491.21 [M+1].

### Example 151 Synthesis of Compound 58-1: tert-Butyl 4-(((2S,4R)-4-(2-fluoroethoxy)-2-(4-(methoxycarbonyl)phenyl)piperidin-1-yl)sulfonyl)-5-methoxy-7-methyl-1H-indole-1-carboxylate

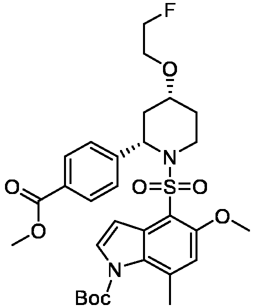

Intermediate 8-2 was added to a 10 mL three-necked flask, dissolved in dichloromethane, followed by the addition of triethylamine and then intermediate 15-1. The reaction was carried out overnight at room temperature. After the reaction was completed, the reaction was quenched with saturated ammonium chloride solution. The organic phase was concentrated under reduced pressure and subjected to column chromatography to obtain the title compound. MS m/z (ESI): 605.30 [M+1].

### Synthesis of Compound 58: 4-((2S,4R)-4-(2-Fluoroethoxy)-1-((5-methoxy-7-methyl-1H-indol-4-yl)sulfonyl)piperidin-2-yl)benzoic acid

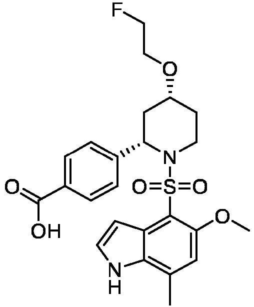

Compound 58-1 was placed in a 10 mL three-necked flask, dissolved in tetrahydrofuran and methanol, followed by the addition of a 1 M aqueous lithium hydroxide solution. The mixture was heated to 45°C and maintained at this temperature overnight. After the reaction was completed, the reaction mixture was concentrated under reduced pressure and subjected to reversed-phase preparative chromatography (gradient elution with aqueous hydrochloric acid: acetonitrile) to obtain the title compound. MS m/z (ESI): 491.21 [M+1].

### Example 152

### Synthesis of Compound 59-1: tert-Butyl 4-(((2S,4S)-4-(cyclopropylmethoxy)-2-(4-(methoxycarbonyl)phenyl)piperidin-1-yl)sulfonyl)-5-methoxy-7-methyl-1H-indole-1-carboxylate

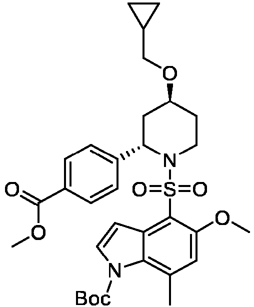

Intermediate 9-2 was added to a 10 mL three-necked flask, dissolved in dichloromethane, followed by the addition of triethylamine and then intermediate 15-1. The reaction was carried out overnight at room temperature. After the reaction was completed, the reaction was quenched with saturated ammonium chloride solution. The organic phase was concentrated under reduced pressure and subjected to column chromatography to obtain the title compound. MS m/z (ESI): 613.30 [M+1].

### Synthesis of Compound 59: 4-((2S,4S)-4-(Cyclopropylmethoxy)-1-((5-methoxy-7-methyl-1H-indol-4-yl)sulfonyl)piperidin-2-yl)benzoic acid

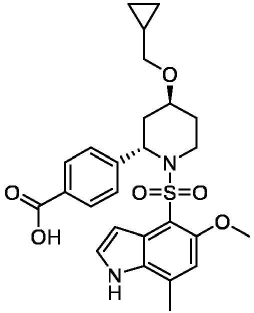

Compound 59-1 was placed in a 10 mL three-necked flask, dissolved in tetrahydrofuran and methanol, followed by the addition of a 1 M aqueous lithium hydroxide solution. The mixture was heated to 45°C and maintained at this temperature overnight. After the reaction was completed, the reaction mixture was concentrated under reduced pressure and subjected to reversed-phase preparative chromatography (gradient elution with aqueous hydrochloric acid: acetonitrile) to obtain the title compound. MS m/z (ESI): 499.21 [M+1].

### Example 153

### Synthesis of Compound 60-1: tert-Butyl 4-(((2S,4R)-4-(cyclopropylmethoxy)-2-(4-(methoxycarbonyl)phenyl)piperidin-1-yl)sulfonyl)-5-methoxy-7-methyl-1H-indole-1-carboxylate

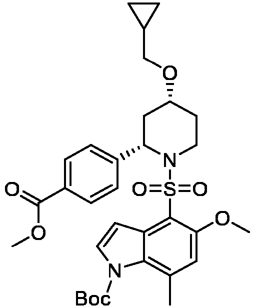

Intermediate 10-2 was added to a 10 mL three-necked flask, dissolved in dichloromethane, followed by the addition of triethylamine and then intermediate 15-1. The reaction was carried out overnight at room temperature. After the reaction was completed, the reaction was quenched with saturated ammonium chloride solution. The organic phase was concentrated under reduced pressure and subjected to column chromatography to obtain the title compound. MS m/z (ESI): 613.30 [M+1].

### Synthesis of Compound 60: 4-((2S,4R)-4-(Cyclopropylmethoxy)-1-((5-methoxy-7-methyl-1H-indol-4-yl)sulfonyl)piperidin-2-yl)benzoic acid

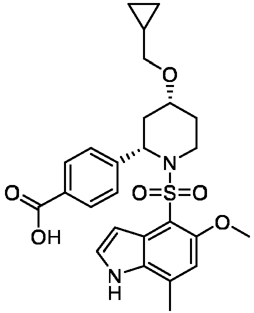

Compound 60-1 was placed in a 10 mL three-necked flask, dissolved in tetrahydrofuran and methanol, followed by the addition of a 1 M aqueous lithium hydroxide solution. The mixture was heated to 45°C and maintained at this temperature overnight. After the reaction was completed, the reaction mixture was concentrated under reduced pressure and subjected to reversed-phase preparative chromatography (gradient elution with aqueous hydrochloric acid: acetonitrile) to obtain the title compound. MS m/z (ESI): 499.21 [M+1].

### Example 154

### 1. Synthesis of Compound 61-1: Benzyl (2S)-4-cyclobutyl-4-hydroxy-2-(4-(methoxycarbonyl)phenyl)piperidine-1-carboxylate

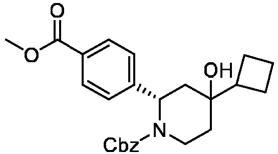

Intermediate 1-2 was placed in a 500 mL four-necked flask, and 50 mL of tetrahydrofuran was added to the reaction flask. The system was purged with nitrogen, and the internal temperature was lowered to 0°C, then further reduced to -78°C. Cyclobutylmagnesium bromide was added dropwise while maintaining the internal temperature at -0°C until the dropwise addition was completed. After 5 hours, the reaction was quenched with saturated ammonium chloride, extracted three times with ethyl acetate, and the organic phases were combined and concentrated under reduced pressure to obtain the title compound as a white solid (9.5 g, yield: 95.4%). MS m/z (ESI): 424.20 [M+1].

### 2. Synthesis of 61-2: Methyl 4-((2S)-4-cyclobutyl-4-hydroxypiperidin-2-yl)benzoate

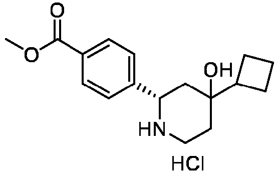

The intermediate 61-1 obtained in step 1 was placed in a single-necked reaction flask, followed by the addition of 10 mL of methanol, then 2 N hydrochloric acid, and subsequently 10% palladium on carbon. The system was purged with hydrogen three times, and the reaction was carried out overnight. After the reaction was completed, the mixture was subjected to suction filtration and then concentrated under reduced pressure to obtain a white solid. The solid was slurried with methyl *tert-*butyl ether for impurity removal, then filtered to obtain the title compound as a white solid powder. MS m/z (ESI): 326.20 [M+1].

### 3. Synthesis of Compound 61-3: tert-Butyl 4-((2S)-4-cyclobutyl-4-hydroxy-2-(4-(methoxycarbonyl)phenyl)piperidine-1-carbonyl)-5-methoxy-7-methyl-1H-indole-1-carboxylate

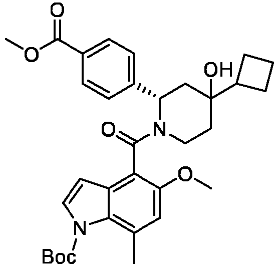

The intermediate 61-2 prepared in step 2 was added to a 10 mL three-necked flask and dissolved in dichloromethane. *N,N-*Diisopropylethylamine was added, and the mixture was placed in an ice bath, followed by the addition of intermediate 11-1. After stirring for 10 min, tert-butyl 2-(7-azabenzotriazol-1-yl)-*N,N,N',N'*-tetramethyluronium hexafluorophosphate was added. After the reaction was completed, dichloromethane and saturated brine were added, and the organic phase was concentrated under reduced pressure to obtain the title compound. MS m/z (ESI): 577.26 [M+1].

### 4. Synthesis of Compound 61: 4-((2S)-4-Cyclobutyl-4-hydroxy-1-(5-methoxy-7-methyl-1H-indole-4-carbonyl)piperidin-2-yl)benzoic acid

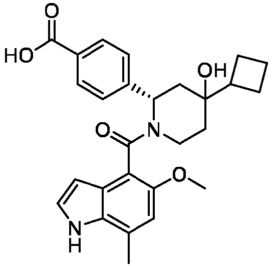

The compound 61-3 obtained in step 3 was placed in a 10 mL three-necked flask, dissolved in tetrahydrofuran and methanol, followed by the addition of a 1 M aqueous lithium hydroxide solution. The mixture was heated to 45°C and maintained at this temperature overnight. After the reaction was completed, the reaction mixture was concentrated under reduced pressure and subjected to reversed-phase preparative chromatography (gradient elution with aqueous hydrochloric acid: acetonitrile) to obtain the final product, which was a racemic compound. MS m/z (ESI): 463.20 [M+1], ¹HNMR (400 MHz, MeOD) δ 8.07 - 7.78 (m, 2H), 7.69 - 7.25 (m, 3H), 7.13 - 6.56 (m, 1H), 6.02 (dd, J = 34.2, 4.7 Hz, 1H), 4.09 - 3.84 (m, 3H), 3.61 - 3.34 (m, 2H), 2.64 - 2.24 (m, 5H), 1.98 (dd, J = 14.5, 6.8 Hz, 2H), 1.93 - 1.77 (m, 4H), 1.68 (ddd, J = 25.0, 16.4, 9.9 Hz, 2H), 1.34 (ddd, J = 29.8, 17.9, 8.3 Hz, 2H).

### Example 155

### 1. Synthesis of Compound 62-1: Benzyl (2S)-4-cyclopropyl-4-hydroxy-2-(4-(methoxycarbonyl)phenyl)piperidine-1-carboxylate

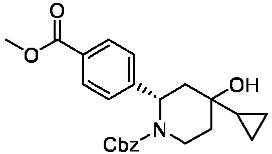

Intermediate 1-2 was placed in a 500 mL four-necked flask, and 50 mL of tetrahydrofuran was added to the reaction flask. The system was purged with nitrogen, and the internal temperature was lowered to 0°C, then further reduced to -78°C. Cyclopropylmagnesium bromide was added dropwise while maintaining the internal temperature at -0°C until the dropwise addition was completed. After 5 hours, the reaction was quenched with saturated ammonium chloride, extracted three times with ethyl acetate, and the organic phases were combined and concentrated under reduced pressure to obtain the title compound as a white solid (9.5 g, yield: 95.4%). MS m/z (ESI): 410.20 [M+1].

### 2. Synthesis of 62-2: Methyl 4-((2S)-4-cyclopropyl-4-hydroxypiperidin-2-yl)benzoate

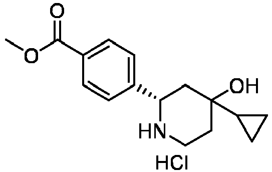

The intermediate 62-1 obtained in step 1 was placed in a single-necked reaction flask, followed by the addition of 10 mL of methanol, then 2 N hydrochloric acid, and subsequently 10% palladium on carbon. The system was purged with hydrogen three times, and the reaction was carried out overnight. After the reaction was completed, the mixture was subjected to suction filtration and then concentrated under reduced pressure to obtain a white solid. The solid was slurried with methyl *tert-*butyl ether for impurity removal, then filtered to obtain the title compound as a white solid powder. MS m/z (ESI): 276.20 [M+1].

### 3. Synthesis of Compound 62-3: tert-Butyl 4-((2S)-4-cyclopropyl-4-hydroxy-2-(4-(methoxycarbonyl)phenyl)piperidine-1-carbonyl)-5-methoxy-7-methyl-1H-indole-1-carboxylate

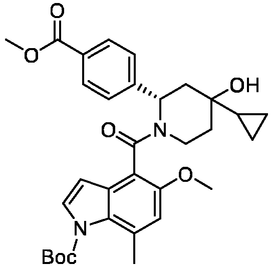

The intermediate 62-2 prepared in step 2 was added to a 10 mL three-necked flask and dissolved in dichloromethane. *N,N*-Diisopropylethylamine was added, and the mixture was placed in an ice bath, followed by the addition of intermediate 11-1. After stirring for 10 min, *tert*-butyl 2-(7-azabenzotriazol-1-yl)-*N,N,N',N'*-tetramethyluronium hexafluorophosphate was added. After the reaction was completed, dichloromethane and saturated brine were added, and the organic phase was concentrated under reduced pressure to obtain the title compound. MS m/z (ESI): 563.26 [M+1].

### 4. Synthesis of Compound 62: 4-((2S)-4-Cyclopropyl-4-hydroxy-1-(5-methoxy-7-methyl-1H-indole-4-carbonyl)piperidin-2-yl)benzoic Acid

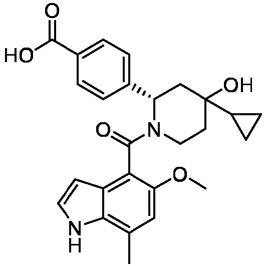

The compound 62-3 obtained in step 3 was placed in a 10 mL three-necked flask, dissolved in tetrahydrofuran and methanol, followed by the addition of a 1 M aqueous lithium hydroxide solution. The mixture was heated to 45°C and maintained at this temperature overnight. After the reaction was completed, the reaction mixture was concentrated under reduced pressure and subjected to reversed-phase preparative chromatography (gradient elution with aqueous hydrochloric acid: acetonitrile) to obtain the final product. MS m/z (ESI): 463.20 [M+1], ¹HNMR (400 MHz, MeOD) δ 8.11-7.76 (m, 2H), 7.72-7.09 (m, 3H), 6.90-6.54 (m, 1H), 6.33-5.96 (m, 1H), 4.06-3.39 (m, 5H), 2.67 (s, 1H), 2.58-2.33 (m, 4H), 2.29-1.21 (m, 3H), 1.04-0.79 (m, 1H), 0.74-0.12 (m, 3H).

### Example 156

### 1. Synthesis of Compound 63-1: Benzyl (2S,4S)-4-methoxy-2-(4-(methoxycarbonyl)phenyl)piperidine-1-carboxylate

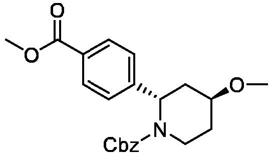

Sodium hydride was placed in a 50 mL three-necked flask, and 10 mL of anhydrous N,N-dimethylformamide was added. The flask was placed in an ice bath. After 10 min, intermediate 1-3 was dissolved in 10 mL of anhydrous N,N-dimethylformamide and added dropwise to the reaction flask. After 30 min, iodoethane was added dropwise. After the dropwise addition was completed, water and ethyl acetate were added 30 min later. The organic phase was concentrated under reduced pressure to obtain the title compound as a colorless oil. MS m/z (ESI): 384.20 [M+1].

### 2. Synthesis of Compound 63-2: Methyl 4-((2S,4S)-4-methoxypiperidin-2-yl)benzoate

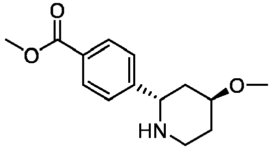

The intermediate 63-1 obtained in step 1 was placed in a single-necked reaction flask, followed by the addition of 10 mL of methanol and subsequently 10% palladium on carbon. The system was purged with hydrogen three times, and the reaction was carried out overnight. After the reaction was completed, the mixture was subjected to suction filtration and then concentrated under reduced pressure to obtain a white solid. The solid was slurried with methyl *tert-butyl* ether for impurity removal, then filtered to obtain the title compound as a white solid powder. MS m/z (ESI): 250.20 [M+1]. ¹HNMR (400 MHz, CDCl₃) δ 9.76 (s, 2H), 8.01 (d, J = 7.7 Hz, 2H), 7.69 (d, J = 7.9 Hz, 2H), 4.36 (s, 1H), 3.92 (s, 3H), 3.70 (s, 1H), 3.33 (s, 3H), 3.00 (d, J = 75.9 Hz, 2H), 2.13 (ddd, J = 82.1, 47.9, 14.0 Hz, 4H).

### 3. Synthesis of Compound 63-3: tert-Butyl 5-methoxy-4-((2S,4S)-4-methoxy-2-(4-(methoxycarbonyl)phenyl)piperidine-1-carbonyl)-7-methyl-1H-indole-1-carboxylate

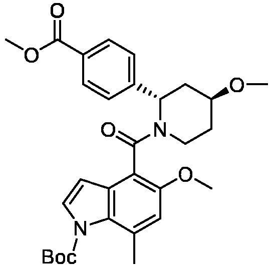

The intermediate 63-2 prepared in step 2 was added to a 10 mL three-necked flask and dissolved in dichloromethane. *N,N*-Diisopropylethylamine was added, and the mixture was placed in an ice bath, followed by the addition of intermediate 11-1. After stirring for 10 min, tert-butyl 2-(7-azabenzotriazol-1-yl)-*N,N,N',N'*-tetramethyluronium hexafluorophosphate was added. After the reaction was completed, dichloromethane and saturated brine were added, and the organic phase was concentrated under reduced pressure to obtain the title compound. MS m/z (ESI): 564.26 [M+1]. ¹HNMR (400 MHz, CDCl₃) δ 8.11 - 7.92 (m, 2H), 7.64 - 7.53 (m, 2H), 7.49 - 7.42 (m, 1H), 6.57 (ddd, J = 35.5, 15.3, 12.5 Hz, 2H), 4.00 - 3.61 (m, 6H), 3.51 (d, J = 14.4 Hz, 1H), 3.38 - 3.24 (m, 4H), 3.01 - 2.70 (m, 2H), 2.63 (d, J = 31.7 Hz, 3H), 2.09 - 1.75 (m, 2H), 1.64 - 1.58 (m, 9H), 1.41 - 1.14 (m, 2H).

### 4. Synthesis of Compound 63: 4-((2S,4S)-4-Methoxy-1-(5-methoxy-7-methyl-1H-indole-4-carbonyl)piperidin-2-yl)benzoic acid

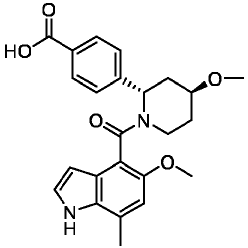

The compound 63-3 obtained in step 3 was placed in a 10 mL three-necked flask, dissolved in tetrahydrofuran and methanol, followed by the addition of a 1 M aqueous lithium hydroxide solution. The mixture was heated to 45°C and maintained at this temperature overnight. After the reaction was completed, the reaction mixture was concentrated under reduced pressure and subjected to reversed-phase preparative chromatography (gradient elution with aqueous hydrochloric acid: acetonitrile) to obtain the title compound. MS m/z (ESI): 423.20 [M+1], ¹HNMR (600 MHz, MeOD) δ 8.13 - 7.88 (m, 2H), 7.76 - 7.45 (m, 2H), 7.32 (dt, J = 68.1, 33.3 Hz, 1H), 6.77 (dd, J = 67.1, 35.1 Hz, 1H), 6.35 (dd, J = 19.9, 3.0 Hz, 1H), 3.99 - 3.88 (m, 3H), 3.68 - 3.40 (m, 2H), 3.37 - 3.33 (m, 3H), 2.99 - 2.80 (m, 2H), 2.60 - 2.46 (m, 3H), 1.87 (ddd, J = 34.4, 30.0, 24.2 Hz, 2H), 1.76 - 1.20 (m, 2H).

### Example 157

### 1. Synthesis of compound 64-1: Benzyl (2S,4S)-4-cyclobutoxy-2-(4-(methoxycarbonyl)phenyl)piperidine-1-carboxylate

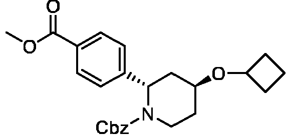

Sodium hydride was placed in a 50 mL three-necked flask, and 10 mL of anhydrous *N,N*-dimethylformamide was added. The flask was placed in an ice bath. After 10 min, intermediate 1-3 was dissolved in 10 mL of anhydrous *N,N*-dimethylformamide and added dropwise to the reaction flask. After 30 min, iodocyclobutane was added dropwise. After the dropwise addition was completed, water and ethyl acetate were added 30 min later. The organic phase was concentrated under reduced pressure to obtain the title compound as a colorless oil. MS m/z (ESI): 424.20 [M+1]. ¹HNMR (400 MHz, CDCl₃) δ 8.00 (d, J = 8.4 Hz, 2H), 7.38 - 7.26 (m, 7H), 5.19 (s, 2H), 4.24 (d, J = 12.6 Hz, 1H), 4.02 - 3.94 (m, 1H), 3.92 (d, J = 5.1 Hz, 3H), 3.40 - 3.29 (m, 1H), 2.80 (td, J = 13.6, 2.9 Hz, 1H), 2.61 (d, J = 13.3 Hz, 1H), 2.16 (ddt, J = 10.9, 9.0, 7.2 Hz, 2H), 2.00 - 1.78 (m, 4H), 1.66 - 1.58 (m, 3H), 1.52 - 1.41 (m, 2H), 1.34 - 1.24 (m, 1H).

### 2. Synthesis of compound 64-2: Methyl 4-((2S,4S)-4-cyclobutoxypiperidin-2-yl)benzoate

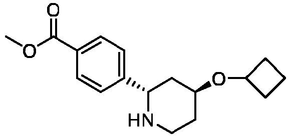

The intermediate 64-1 obtained in step 1 was placed in a single-necked reaction flask, followed by the addition of 10 mL of methanol and subsequently 10% palladium on carbon. The system was purged with hydrogen three times, and the reaction was carried out overnight. After the reaction was completed, the mixture was subjected to suction filtration and then concentrated under reduced pressure to obtain a white solid. The solid was slurried with methyl *tert-butyl* ether for impurity removal, then filtered to obtain the title compound as a white solid powder. MS m/z (ESI): 290.20 [M+1].

### 3. Synthesis of Compound 64-3: tert-Butyl 4-((2S,4S)-4-(cyclobutyloxy)-2-(4-(methoxycarbonyl)phenyl)piperidine-1-carbonyl)-5-methoxy-7-methyl-1H-indole-1-carboxylate

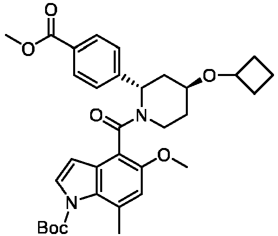

The intermediate 64-2 prepared in step 2 was added to a 10 mL three-necked flask and dissolved in dichloromethane. *N,N*-Diisopropylethylamine was added, and the mixture was placed in an ice bath, followed by the addition of intermediate 11-1. After stirring for 10 min, tert-butyl 2-(7-azabenzotriazol-1-yl)-*N,N,N',N'*-tetramethyluronium hexafluorophosphate was added. After the reaction was completed, dichloromethane and saturated brine were added, and the organic phase was concentrated under reduced pressure to obtain the title compound. MS m/z (ESI): 577.26 [M+1].

### 4. Synthesis of Compound 64: 4-((2S,4S)-4-Cyclobutoxy-1-(5-methoxy-7-methyl-1H-indole-4-carbonyl)piperidin-2-yl)benzoic acid

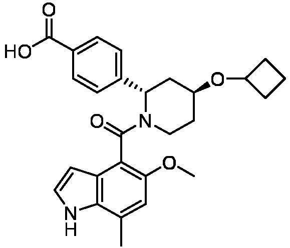

The compound 64-3 obtained in step 3 was placed in a 10 mL three-necked flask, dissolved in tetrahydrofuran and methanol, followed by the addition of a 1 M aqueous lithium hydroxide solution. The mixture was heated to 45°C and maintained at this temperature overnight. After the reaction was completed, the reaction mixture was concentrated under reduced pressure and subjected to reversed-phase preparative chromatography (gradient elution with aqueous hydrochloric acid: acetonitrile) to obtain the title compound. MS m/z (ESI): 463.20 [M+1], ¹HNMR (400 MHz, MeOD) δ 8.15-7.87 (m, 2H), 7.70-7.22 (m, 3H), 7.05-6.63 (m, 1H), 6.34 (dt, J = 12.6, 6.7 Hz, 1H), 4.18-3.78 (m, 4H), 3.64-3.40 (m, 2H), 3.07-2.68 (m, 2H), 2.51 (d, J = 28.6 Hz, 2H), 2.18 (d, J = 5.5 Hz, 2H), 1.90 (dt, J = 17.9, 10.2 Hz, 3H), 1.67 (dd, J = 21.0, 10.8 Hz, 2H), 1.58-1.41 (m, 2H), 1.38-1.13 (m, 2H).

### Example 158

### 1. Synthesis of Compound 65-1: Benzyl (2S)-4-hydroxy-2-(4-(methoxycarbonyl)phenyl)-4-propylpiperidine-1-carboxylate

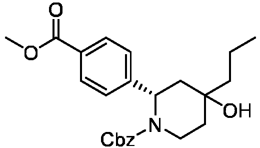

Intermediate 1-2 was placed in a 500 mL four-necked flask, and 50 mL of tetrahydrofuran was added to the reaction flask. The system was purged with nitrogen, and the internal temperature was lowered to 0°C, then further reduced to -78°C. Cyclopropylmagnesium bromide was added dropwise while maintaining the internal temperature at -0°C until the dropwise addition was completed. After 5 hours, the reaction was quenched with saturated ammonium chloride, extracted three times with ethyl acetate, and the organic phases were combined and concentrated under reduced pressure to obtain the title compound as a white solid (9.5 g, yield: 95.4%). MS m/z (ESI): 412.20 [M+1].

### 2. Synthesis of Compound 65-2: Benzyl (S)-2-(4-(methoxycarbonyl)phenyl)-4-propyl-3,6-dihydropyridine-1(2H)-carboxylate

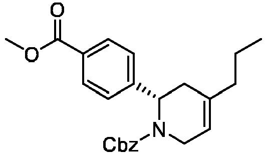

The intermediate 65-1 obtained in step 1 was placed in a 500 mL four-necked flask, and 50 mL of tetrahydrofuran was added to the reaction flask. The system was purged with nitrogen, and the internal temperature was lowered to 0°C, then further reduced to -0°C. Burgess reagent was added while maintaining the internal temperature at -0°C until the dropwise addition was completed. After 5 hours, the reaction was quenched with saturated ammonium chloride, extracted three times with ethyl acetate, and the organic phases were combined and concentrated under reduced pressure to obtain the title compound as a white solid. MS m/z (ESI): 394.20 [M+1].

### 3. Synthesis of compound 65-3: Methyl 4-((2S)-4-propylpiperidin-2-yl)benzoate

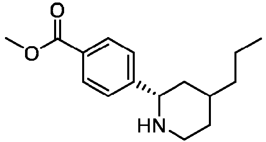

The intermediate 65-2 obtained in step 2 was placed in a single-necked reaction flask, followed by the addition of 10 mL of methanol and subsequently 10% palladium on carbon. The system was purged with hydrogen three times, and the reaction was carried out overnight. After the reaction was completed, the mixture was subjected to suction filtration and then concentrated under reduced pressure to obtain a white solid. The solid was slurried with methyl *tert-*butyl ether for impurity removal, then filtered to obtain the title compound as a white solid powder. MS m/z (ESI): 262.20 [M+1].

### 4. Synthesis of compound 65-4: tert-Butyl 5-methoxy-4-((2S)-2-(4-(methoxycarbonyl)phenyl)-4-propylpiperidine-1-carbonyl)-7-methyl-1H-indole-1-carboxylate

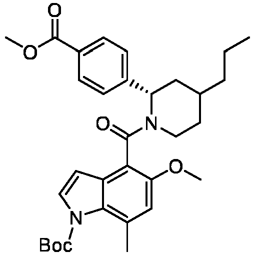

The intermediate 65-3 prepared in step 3 was added to a 10 mL three-necked flask and dissolved in dichloromethane. *N,N-*Diisopropylethylamine was added, and the mixture was placed in an ice bath, followed by the addition of intermediate 11-1. After stirring for 10 min, *tert*-butyl 2-(7-azabenzotriazol-1-yl)-*N,N,N',N'*-tetramethyluronium hexafluorophosphate was added. After the reaction was completed, dichloromethane and saturated brine were added, and the organic phase was concentrated under reduced pressure to obtain the title compound. MS m/z (ESI): 549.26 [M+1].

### 5. Synthesis of Compound 65: 4-((2S)-1-(5-Methoxy-7-methyl-1H-indole-4-carbonyl)-4-propylpiperidin-2-yl)benzoic acid

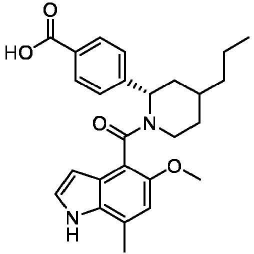

The compound 65-4 obtained in step 4 was placed in a 10 mL three-necked flask, dissolved in tetrahydrofuran and methanol, followed by the addition of a 1 M aqueous lithium hydroxide solution. The mixture was heated to 45°C and maintained at this temperature overnight. After the reaction was completed, the reaction mixture was concentrated under reduced pressure and subjected to reversed-phase preparative chromatography (gradient elution with aqueous hydrochloric acid: acetonitrile) to obtain the final product, which was a racemic compound.

MS m/z (ESI): 435.20 [M+1], ¹HNMR (400 MHz, MeOD) δ 8.11-7.76 (m, 2H), 7.72-7.09 (m, 3H), 6.90-6.54 (m, 1H), 6.33-5.96 (m, 1H), 4.06-3.39 (m, 5H), 2.67 (s, 1H), 2.58-2.33 (m, 4H), 2.29-1.21 (m, 3H), 1.04-0.79 (m, 1H), 0.74-0.12 (m, 3H).

### Example 159

1.
The compound 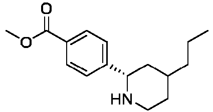 of Example 158 was separated using the following chromatographic conditions:
Chromatographic column: CHIRALPAK^{®} IB, 10 µm, 30 * 250 mm
Mobile phase A: HEX + 0.2% DEA
Mobile phase B: ETOH + 0.2% DEA
Detection wavelength: 214 nm/254 nm
Flow rate: 25 mL/min
Column temperature: RT
Isocratic elution program: mobile phase A: mobile phase B = 95:5 (V/V).

Chiral isomers compound 159-1A and compound 159-1B were separated with retention times of 6.063 min and 8.812 min, respectively. 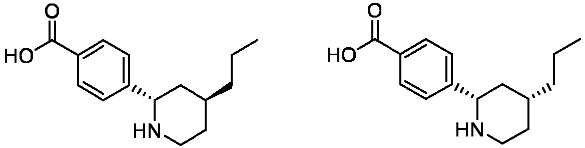 159-1A or 159-1B and 159-1A or 159-1B

2.
Compound 159A and compound 159B were prepared using compound 159-1A and compound 159-1B in step 1 as starting materials, respectively, by the methods of steps 4 and 5 of Example 158.
Compound 159A: ¹H NMR(400 MHz, MeOD) δ 8.11 - 7.76 (m, 2H), 7.72 - 7.09 (m, 3H), 6.90 - 6.54 (m, 1H), 6.33 - 5.96(m, 1H), 4.06 - 3.39 (m, 5H), 2.67 (s, 1H), 2.58 - 2.33 (m, 4H), 2.29 - 1.21 (m, 3H), 1.04 - 0.79 (m, 1H),0.74 - 0.12 (m, 3H)
Compound 159B: ¹H NMR(400 MHz, MeOD) δ 8.11 - 7.76 (m, 2H), 7.72 - 7.09 (m, 3H), 6.90 - 6.54 (m, 1H), 6.33 - 5.96(m, 1H), 4.06 - 3.39 (m, 5H), 2.67 (s, 1H), 2.58 - 2.33 (m, 4H), 2.29 - 1.21 (m, 3H), 1.04 - 0.79 (m, 1H),0.74 - 0.12 (m, 3H) 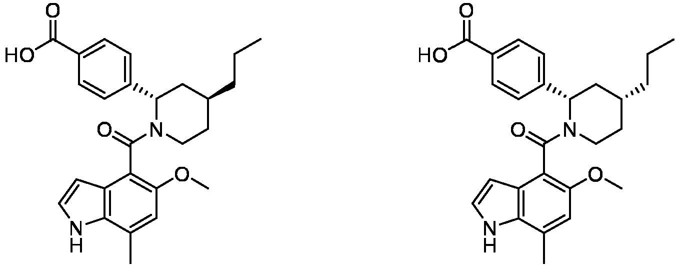 compound 159A or compound 159B or compound 159A or compound 159B .

### Effect Example 1: Optical Surface Plasmon Resonance (SPR) Binding Affinity Assay

The SPR experiment was conducted at 25°C using PBS buffer supplemented with 0.05%(v/v) P20 and 5% DMSO as the running buffer. The Biacore 8K instrument from GE Healthcare was employed for analysis. The CM3 chip (GE Healthcare) was activated with 400 mM EDC and 100 mM NHS at a flow rate of 30 µL/min for 420 s. Complement factor B was diluted to 50 µg/mL with 10 mM sodium acetate (pH 40), then covalently immobilized onto the sensor chip through conjugating at a flow rate of 10 µL/min for 1200 s (protein immobilization level: 25,000 RU). Subsequently, the sensor chip was blocked by treating with 1 M ethanolamine hydrochloride at a flow rate of 10 µL/min for 300 s. The concentration of the test compound was 500 µM, with a binding time of 120 s and a dissociation time of 300 s. The data analysis was performed using a 1:1 binding model (Biacore Insight Evaluation Software Version 2.0.1512933).

The assay results show that all compounds of the present disclosure exhibit affinity activity towards complement factor B, with a KD₅₀ value ranging from 0.1 nM to 500 nM.

| Compound No. | Compound structure | KD₅₀ (nM) |
|---|---|---|
| LNP023 Positive control | 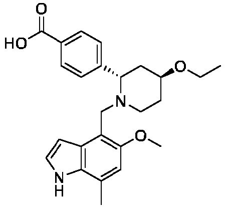 | 8 |
| Compound 1 of Example 55 | 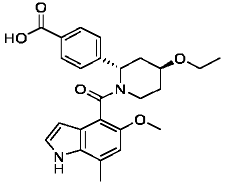 | 10-30 |
| Compound 9 of Example 71 | 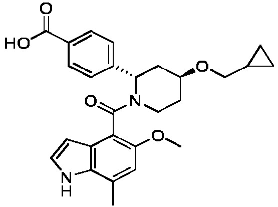 | 10-30 |
| Final product of Example 156 | 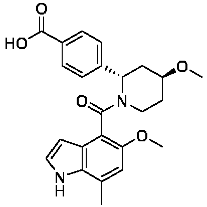 | 10-30 |
| Final product of Example 154 | 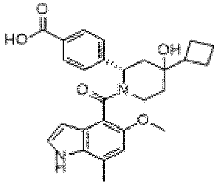 | 10-30 |
| Final product of Example 157 | 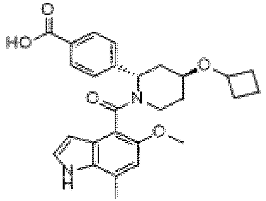 | 10-30 |
| Final product of Example 155 | 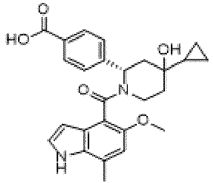 | 10-30 |
| Final product of Example 158 | 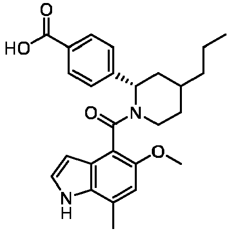 | 5-20 |
| Final product of Example 159 | 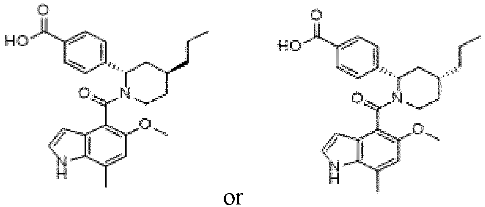 | Compound 159A |
| | | 5-20 |
| | | Compound 159B |
| | | 5-20 |
| Compound 11 of Example 75 | 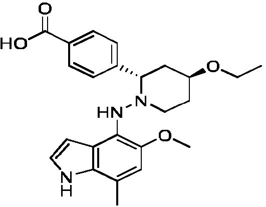 | 5-20 |
| Compound 51 of Example 144 | 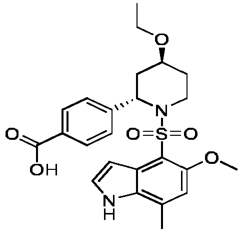 | 10-30 |

### Effect Example 2: Complement Hemolytic Activity Assay

The complement hemolytic activity assay was conducted with reference to the method described by Xuan Yuan et al., Haematologica (2017) 102:466-475. Prior to the experiment, the optimal concentration of normal human serum (NHS) required to achieve 100% lysis of rabbit erythrocytes (RE) was determined by titration. In this experiment, NHS was diluted in GVBO buffer (0.1% gelatin, 5 mM Veronal, 145 mM NaCl, 0.025% NaN, pH 7.3, Complement Technology) containing 10 mM Mg-EGTA and incubated with various concentration gradients of test compounds at 37°C for 15 min. The RE (obtained from healthy Japanese white rabbits) was newly suspended in GVBO buffer containing 10 mM Mg-EGTA, adjusted to a final concentration of 1 × 10⁸ cells/mL, and incubated at 37°C for 30 min. The positive control group (100% lysis) was composed of GVBO buffer containing 10 mM Mg-EGTA with NHS and RE but no test compound. The negative control group (0% lysis) was composed of GVBO buffer containing 10 mM Mg-EGTA with inactivated NHS (heated at 56°C for 30 min or 65°C for 5 min) and RE but no test compound. The sample was centrifuged at 2000 g for 5 min, and the supernatant was then collected. The absorbance at 415 nm (A415) was measured using a microplate reader (Molecular Devices SpectraMax i3X). The IC₅₀ value was calculated by applying nonlinear regression with GraphPad Prism 7.0 software from the percentage of hemolysis as a function of the test compound concentration.

The assay results show that all compounds of the present disclosure exhibit hemolytic activity against the alternative complement pathway, with IC₅₀ values ranging from 0.1 nM to 500 nM.

### Effect Example 3: Alternative Pathway Inhibition Assay of PNH-like Erythrocyte

**Principle:** PNH, as an indication for complement inhibitors, was typically tested using the Ham test, which states that acidified serum was capable of activating the alternative pathway, leading to hemolysis of PNH erythrocytes, while normal erythrocytes remained unaffected. It was reported in the literature that AET could disrupt the complement regulatory proteins on the surface of normal human erythrocytes, resulting in PNH-like erythrocytes.
**Reagents and instrument:** PBS buffer, normal human serum (NHS), Alsever's solution, microplate reader, MgCl₂, EGTA, EDTA, HCl, normal human erythrocytes, AET; anti-CD-55 antibody (BRIC216; Sigma)
PBS buffer: Potassium dihydrogen phosphate (KH₂PO₄): 0.24 g/L, disodium hydrogen phosphate (Na₂HPO₄): 1.44 g/L, sodium chloride (NaCl): 8 g/L, potassium chloride (KCl): 0.2 g/L, pH 7.4
PBSE buffer: PBS, 10 mM EDTA; pH 7.4
PBSMg buffer: PBS, 1 mM Mg++; pH 6.4
10x Mg-EGTA stock solution: 25 mM MgCl₂, 80 mM EGTA; pH 6.4
10x EDTA stock solution: 100 mM EDTA; pH 6.4
**Method:** The inhibition of alternative pathway-mediated erythrocyte lysis was evaluated using an *in vitro* model of PNH. Normal human erythrocytes were sensitized with 2-aminoethylisothiouronium bromide (AET) to inactivate complement regulatory proteins on the cell surface and induce a PNH-like phenotype.

2 mL of normal human erythrocytes were mixed with 2 mL of Alsever's solution, washed three times with PBS, and centrifuged at 2500× g, with the upper 10% of the erythrocytes being removed each time. The washed 1 mL erythrocytes were sensitized by incubation with 4 mL of 8% w/v AET (pH 8.0) at 37°C for 10 min, followed by three additional washes with PBS. Subsequently, the concentration of erythrocytes in PBS (erythrocyte stock solution) was adjusted with PBS such that an absorbance of 1.5-2.0 at 412 nm (indicating hemoglobin release) was achieved upon dilution of 10 µL erythrocyte stock solution with 190 µL water.

1.5 mL of the erythrocyte stock solution was mixed with anti-CD55 antibody (BRIC216; Sigma/ final concentration of 6.7 µg/mL) and incubated on ice for 30 min. After being washed once with PBS, the treated erythrocytes were resuspended in PBS-Mg to the initial volume of 1.5 mL. The following AP-mediated lysis assay was performed on this erythrocyte preparation with induced PNH phenotype.

The normal human serum was acidified with 0.2 M HCl to pH 6.4, and supplemented with MgCl₂ and EGTA to final concentrations of 2.5 and 8 mM, respectively. 80 µL of this serum mixture was mixed with 10 µL of PBS-Mg (negative blank) or 10 µL of samples at various concentrations. Normal human serum was acidified with 0.2 M HCl to pH 6.4, supplemented with EDTA to a final concentration of 10 mM, and 80 µL of this serum mixture was mixed with 10 µL of PBS-Mg (negative blank) as a blank positive control. The sample was incubated with serum on ice for 5 min, and then 10 µL of sensitized erythrocytes were added. The final serum concentration in the reaction mixture was approximately 64% v/v, incubated at 37°C for 60 min, and the reaction was quenched by adding 50 µL of ice-cold PBS containing 10 mM EDTA. The remaining unlysed cells were centrifuged at 2500x g for 10 min, and 100 µL of the supernatant was transferred to a 96-well plate to measure its absorbance at 412 nm. The amount of hemoglobin released during erythrocyte lysis was thereby quantified. A 405 represented the relative lysis of PNH-like erythrocytes (expressed in %; set to 0% for the PC sample and 100% for the NC sample). The absorbance was measured using a microplate reader (Molecular Devices SpectraMax i3X). The IC₅₀ value was calculated by applying nonlinear regression with GraphPad Prism 9.0 software from the percentage of hemolysis as a function of the test compound concentration.

### Results:

| Compound No. | Compound structure | IC₅₀ (nM) |
|---|---|---|
| LNP023 Positive control | 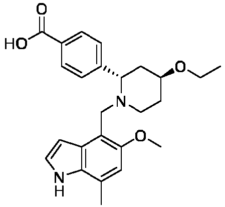 | 224 |
| Compound 1 of Example 55 | 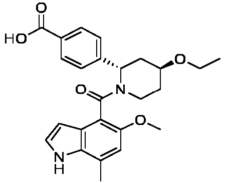 | 1000-1500 |
| Compound 9 of Example 71 | 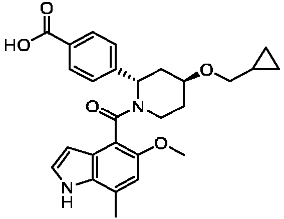 | 6000-6500 |
| Final product of Example 156 | 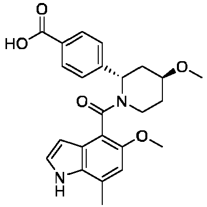 | 1000-1500 |
| Final product of Example 154 | 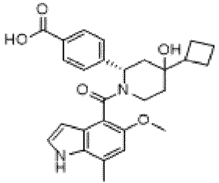 | 4000-4500 |
| Final product of Example 157 | 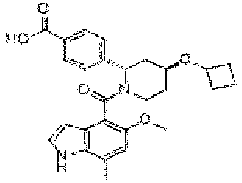 | 4000-4500 |
| Final product of Example 155 | 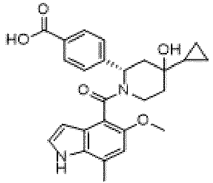 | 1000-1500 |
| Final product of Example 158 | 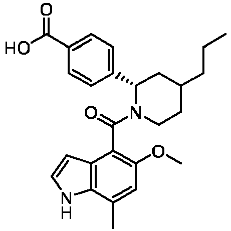 | 50-250 |
| Final product of Example 159 | 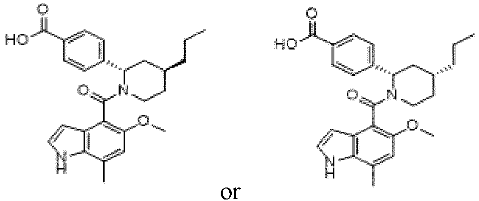 | Compound 159A |
| | | 50-250 |
| | | Compound 159B |
| | | 50-250 |
| Compound 11 of Example 75 | 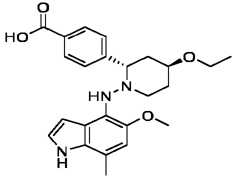 | 100-300 |
| Compound 51 of Example 144 | 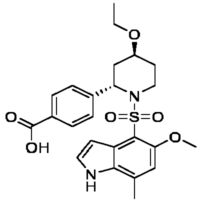 | 2000-2500 |
| Compound 51 of Example 144 | 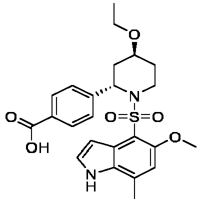 | 2000-2500 |

### Effect Example 4: Human Complement Factor B ELISA Assay

4.2 mg (approximately 0.0091 mmol) of the sample compound was dissolved in 455 µL of DMSO (molecular biology grade) to prepare a 20 mM stock solution A of the sample compound.
1 2000 µM 100 µL of the stock solution A of the sample compound was taken and added with 900 µL of diluent to form liquid 1;
2 500 µM 100 µL of liquid 1 was taken and added with 300 µL of diluent to form liquid 2;
3 125 µM 100 µL of liquid 2 was taken and added with 300 µL of diluent to form liquid 3;
4 31.25 µM 100 µL of liquid 3 was taken and added with 300 µL of diluent to form liquid 4;
5 7.8125 µM 100 µL of liquid 4 was taken and added with 300 µL of diluent to form liquid 5;
6 0.488281 µM 100 µL of liquid 5 was taken and added with 300 µL of diluent to form liquid 6;
7 0.12207 µM 100 µL of liquid 6 was taken and added with 300 µL of diluent to form liquid 7;
8 0.030518 µM 100 µL of liquid 7 was taken and added with 300 µL of diluent to form liquid 8;
9 0.007629 µM 100 µL of liquid 8 was taken and added with 300 µL of diluent to form liquid 9.

100 µL of the above samples 1 to 9 were taken and diluted 20-fold by adding 1900 µL of the diluent to obtain samples 1° to 9'.

8.8 µL of the diluent was added to the wells of a 96T plate, followed by the addition of 0.2 µL of CVF-Bb (0.1 µmol/mL) and then 1 µL of the sample compounds 1' to 9'. The mixture was incubated at room temperature for 1 hour;
Then, 9 µL of the successfully incubated samples were taken and added to the wells of the 96T plate, followed by the addition of 1 µL complement C3 protein, and the reaction was carried out at a constant temperature of 37°C for 2 hours;
The 9 µL of the aforementioned sample-free liquid was mixed with 1 µL of complement C3 protein as the negative control;
10 µL of the successfully incubated sample was taken, and the inhibitory concentration was determined by ELISA. The OD value at 450 nm was measured, and a curve was plotted. The obtained data were analyzed using GraphPad Prism 7.0 software, and a Log concentration-inhibition rate curve was generated using a nonlinear regression model to calculate the IC₅₀ value.

The assay results show that all compounds of the present disclosure exhibit inhibitory activity against the alternative complement pathway, with IC₅₀ values ranging from 0.1 nM to 500 nM.

| Compound No. | Compound structure | ELISA IC₅₀ value (nM) |
|---|---|---|
| LNP023 Positive control | 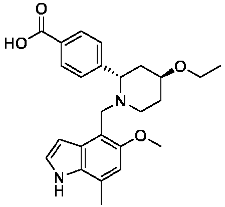 | 24 |
| Compound 1 of Example 55 | 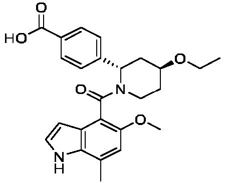 | 150-300 |
| Final product of Example 156 | 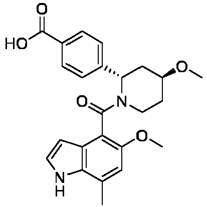 | 150-300 |
| Final product of Example 154 | 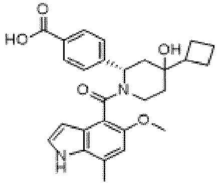 | 400-450 |
| Final product of Example 157 | 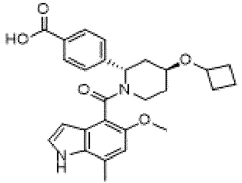 | 400-450 |
| Final product of Example 155 | 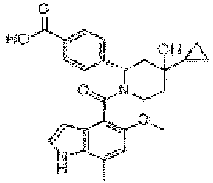 | 150-300 |
| Final product of Example 158 | 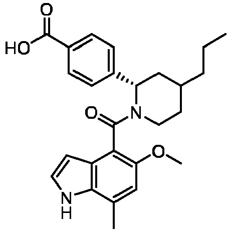 | 5-30 |
| Final product of Example 159 | 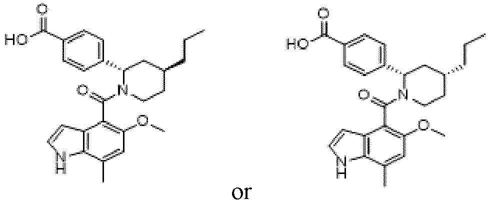 | Compound 159A |
| | | 5-30 |
| | | Compound 159B |
| | | 5-30 |
| Compound 11 of Example 75 | 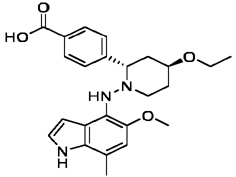 | 10-30 |
| Compound 51 of Example 144 | 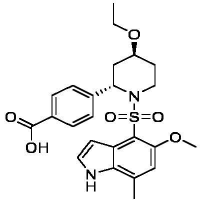 | 300-500 |

### Effect Example 5: Pharmacokinetic Activity Assay

**Experimental objective:** The test substance was administered to mice via single-dose intravenous and oral gavage routes, and its plasma concentration was measured to evaluate the pharmacokinetic characteristics in mice.

**Experimental animals:** C57 mice or other available test mice.

### Experimental methods:

IV dose: 1 mg/kg; vehicle: 0.5% DMSO or 0.5% MeOH-5% pharmaceutical grade polyethylene glycol-15 hydroxystearate-PBS
PO dose: 10 mg/kg; vehicle: 0.5% DMSO or 0.5% MeOH-0.1% Tween 80-30% PEG300-70% PBS
Sample collection: 0.03 mL of blood samples were collected from the saphenous vein in experimental animals by puncture at each time point, and the actual blood collection time was recorded. All blood samples were added to commercially available EDTA-K2 anticoagulant tubes with a specification of 1.5 mL (supplied by Jiangsu Kangjian Medical Apparatus Co., Ltd.). After collection, within half an hour, the blood sample was centrifuged at 4°C, 3000 g for 10 min to aspirate the supernatant plasma, which was put in dry ice immediately, and stored in a refrigerator at -80°C for LCMS/MS analysis.
Blood sampling points: 5 min, 15 min, 30 min, 1 hour, 2 hours, 4 hours, 6 hours, 8 hours, 12 hours, 20 hours, 24 hours.
Test compounds: positive compound LNP023, the final product of Example 158, the final products (compound 159A and compound 159B) of Example 159, and compound 11 of Example 75.
**Experimental results:** Compared with the positive compound LNP023, the clearance rate and half-life during *in vivo* metabolism are found to be comparable or superior for the final product of Example 158, the final products (compound 159A and compound 159B) of Example 159, and compound 11 of Example 75.

### Effect Example 6: In vivo Efficacy Assay of LPS-induced Complement Activation in Mice

**Experimental objective:** To investigate the inhibitory effect of the compounds of the present disclosure on complement activation in mice induced by LPS stimulation.

**Experimental animals:** Female C57BL/6J mice, 7 to 9 weeks old, weighing between 17 to 23 g.

**Experimental procedure:** 100 µg of lipopolysaccharide (LPS) in Salmonella typhimurium (Sigma) dissolved in 100 µL of sterile PBS was intraperitoneally injected to induce complement activation in mice. Negative control animals were intraperitoneally injected 100 µL of sterile PBS and administered alone by tube feeding (PO). Positive control animals were intraperitoneally injected LPS and PO drug vehicles (0.5% (w/v) methyl cellulose and 0.5% (v/v) Tween 80).

Sample collection: 0.3 mL of blood samples were collected from the orbital venous plexus. All blood samples were added to commercially available EDTA-K2 anticoagulant tubes with a specification of 1.5 mL. After collection, within half an hour, the blood sample was centrifuged at 4°C, 3000 g for 10 min to aspirate the supernatant plasma, which was put in dry ice immediately, and stored in a refrigerator at -80°C for Western blot analysis of downstream C3d protein levels after complement activation.

Sample analysis: Mouse plasma (5 µL) + Lysis buffer (27.5 µL) + Loading buffer (12.5 µL) + Reducing buffer (5 µL) were mixed well and incubated at 100°C for 15 min with a loading volume of 5 µL/well, i.e., a loading volume of plasma of 0.5 µL per well.

**Test compounds:** positive compound LNP023, the final product of Example 158, the final products (compound 159A and compound 159B) of Example 159, and compound 11 of Example 75.

**Experimental results:** The final product of Example 158, the final products (compound 159A and compound 159B) of Example 159, and the compound 11 of Example 75 are all superior or equivalent to positive LNP023 in relieving bone swelling in mice in the mouse inflammation model.

### Effect Example 7: In vivo Efficacy Assay of Passive Heymann Nephritis in Rats

**Experimental objective:** To investigate the ability of the compounds of the present disclosure to improve renal function of Sheep Anti-Rat Fx1A Serum induced Heymann nephritis in rats, including the evaluation of reducing proteinuria level and improving renal tissue damage.

**Experimental animals:** Male SD rats, 7 to 10 weeks old, weighing between 200 to 300 g.

### Experimental procedure:

### 1. Modeling:

Urine was collected from rats on D-2 before administration. On D1, the first day of the experiment, animals in the control group (group 1) were administered 5 mL/kg of Sheep Non-Immune serum via a single tail vein injection; animals in the model and administration groups (groups 2 to 6) were administered 5 mL/kg of Sheep Anti-Rat FxlA Serum via a single tail vein injection.

### 2. Administration:

The rats were treated by intragastric administration twice a day with an interval of 8 hours and a volume of 10 mL/kg. On D1, 1 hour before modeling, animals in groups 1 and 2 were administered blank vehicle 20% PEG400/10% Solutol/70% water; animals in group 3 were administered LNP023 (60 mpk); animals in groups 4 to 6 were administered different concentrations of synthetic compounds (5 mpk, 20 mpk, and 60 mpk); 8 hours after the first administration, each group was administered again with the same dose and volume as the first administration. From D2 to D14, animals in each group were administered different compounds or vehicles for 14 consecutive days (including the first day) according to the dose, volume, administration method, and frequency on D1.

### 3. Sample collection:

Urine collection: Urine samples were collected from rats on D-2 before administration, and 2 to 4 hours and 4 to 6 hours after the first administration on D4, D6, D8, D11, and D14 after administration, transferred to EP tubes and stored in a refrigerator at -80°C to -60°C for the detection of urine protein and creatinine in rats.

Kidney sample collection: All animals were euthanized with CO₂ inhalation anesthesia on Day 15, and bilateral kidneys were collected. The left kidney was cut transversely, and the right kidney was cut longitudinally. Half of the transversely cut kidney (left side) and half of the longitudinally cut kidney (right side) were fixed in formalin (placed in the same EP tube).

### 4. Sample analysis:

(1) Urine creatinine (uCRE) in rats was normally loaded with a 10-fold dilution (10 µL of sample + 90 µL of saline (0.9% saline)) (Decrease mode); if exceeding the upper detection limit, it was 20-fold diluted (5 µL of sample + 95 µL of saline (0.9% saline)) (Increase mode). Urine total protein (uTP) in rats was detected without dilution; if exceeding the upper detection limit, it was 10-fold diluted (10 µL of sample + 90 µL of saline (0.9% saline)) first (Decrease mode); if still exceeding the upper detection limit after 10-fold dilution, it was 100-fold diluted (2 µL of sample + 198 µL of saline (0.9% saline)) (Increase mode). Data were read by the analytical instrument HITACHI LST008AS(P).
(2) Kidney pathological scoring: The degree of drug damage to rat kidneys (paraffin sections) was analyzed by HE staining. Scoring criteria: 0 means normal, 1 means little cell infiltration in mesangium, 2 means more cell infiltration in mesangium, 3 means proliferation of several mesangial cells and infiltration of several mesangial cells in glomeruli, 4 means urinary casts and atrophy in renal tubule, and formation and sclerosis of glomerular crescent.

Test compounds: positive compound LNP023, the final product of Example 158, the final products (compound 159A and compound 159B) of Example 159, and compound 11 of Example 75.

**Experimental results:** The final product of Example 158, the final products (compound 159A and compound 159B) of Example 159, and the compound 11 of Example 75 all performed better than the positive control LNP023 in the kidney disease model through urine and kidney pathology analysis.

## Claims

1. A compound of formula (I), a stereoisomer thereof, a tautomer thereof, an isotopically labeled compound thereof, or a pharmaceutically acceptable salt of any one of the foregoing: 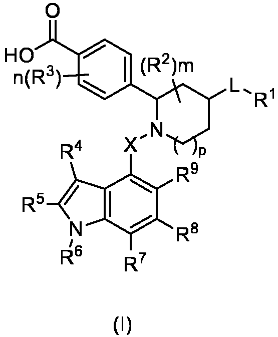 wherein
p is 0, 1, or 2;
L is a single bond, NR⁰, O, or S;
R¹ is hydrogen, C₁₋₆ alkyl, C₂₋₆ alkenyl, C₂₋₆ alkynyl, C₃₋₆ cycloalkyl, C₃₋₆ cycloalkenyl, C₃₋₆ cycloalkynyl, -C₁₋₃ alkyl-C₃₋₆ cycloalkyl, "3- to 12-membered heterocycloalkyl with 1, 2, or 3 heteroatoms selected from 1, 2, or 3 types of N, O, and S", -C₁₋₃ alkyl-"3- to 12-membered heterocycloalkyl with 1, 2, or 3 heteroatoms selected from 1, 2, or 3 types of N, O, and S", or the following group substituted by one or more R^{a}: C₁₋₆ alkyl, C₂₋₆ alkenyl, C₂₋₆ alkynyl, C₃₋₆ cycloalkyl, C₃₋₆ cycloalkenyl, C₃₋₆ cycloalkynyl, -C₁₋₃ alkyl-C₃₋₆ cycloalkyl, "3- to 12-membered heterocycloalkyl with 1, 2, or 3 heteroatoms selected from 1, 2, or 3 types of N, O, and S", or -C₁₋₃ alkyl-"3- to 12-membered heterocycloalkyl with 1, 2, or 3 heteroatoms selected from 1, 2, or 3 types of N, O, and S";
m and n are each independently 0, 1, 2, 3, or 4;
R², R³, R⁴, R⁵, R⁷, R⁸, and R⁹ are each independently hydrogen, halogen, OH, CN, NO₂, NH₂, C₁₋₆ alkyl, C₁₋₆ alkoxy, C₂₋₆ alkenyl, C₂₋₆ alkynyl, C₃₋₆ cycloalkyl, C₃₋₆ cycloalkenyl, C₃₋₆ cycloalkynyl, -C₁₋₃ alkyl-C₃₋₆ cycloalkyl, or the following group substituted by one or more R^{b}: C₁₋₆ alkyl, C₁₋₆ alkoxy, C₂₋₆ alkenyl, C₂₋₆ alkynyl, C₃₋₆ cycloalkyl, C₃₋₆ cycloalkenyl, C₃₋₆ cycloalkynyl, or -C₁₋₃ alkyl-C₃₋₆ cycloalkyl;
R⁰ and R⁶ are each independently hydrogen, C₁₋₆ alkyl, C₃₋₆ cycloalkyl, C₃₋₆ cycloalkenyl, C₃₋₆ cycloalkynyl, -C₁₋₃ alkyl-C₃₋₆ cycloalkyl, or the following group substituted by one or more R^{c}: C₁₋₆ alkyl, C₃₋₆ cycloalkyl, C₃₋₆ cycloalkenyl, C₃₋₆ cycloalkynyl, or -C₁₋₃ alkyl-C₃₋₆ cycloalkyl;
X is -C(=O)-, -C(=NR¹⁰)-, -C(=S)-, -CFR¹¹-, -NR¹²-, -CR¹³R¹⁴-CR¹⁵R¹⁶-, -S-, or -S(=O)₂-;
R¹¹, R¹³, R¹⁴, R¹⁵, and R¹⁶ are each independently hydrogen, halogen, OH, CN, NO₂, NH₂, C₁₋₆ alkyl, C₃₋₆ cycloalkyl, or the following group substituted by one or more R^{d}: C₁₋₆ alkyl or C₃₋₆ cycloalkyl;
R¹⁰ and R¹² are each independently hydrogen, C₁₋₆ alkyl, or C₁₋₆ alkyl substituted by one or more R^{e};
R^{a}, R^{b}, R^{c}, R^{d}, and R^{e} are each independently halogen, OH, CN, NO₂, or NH₂.

2. The compound of formula (I), the stereoisomer thereof, the tautomer thereof, the isotopically labeled compound thereof, or the pharmaceutically acceptable salt of any one of the foregoing according to claim 1, wherein the compound of formula (I) satisfies one or more of the following conditions:
(1) in R⁰, R¹, R², R³, R⁴, R⁵, R⁶, R⁷, R⁸, R⁹, R¹⁰, R¹¹, R¹², R¹³, R¹⁴, R¹⁵, and R¹⁶, the C₁₋₆ alkyl in the C₁₋₆ alkyl and substituted C₁₋₆ alkyl is independently methyl, ethyl, *n*-propyl, isopropyl, *n*-butyl, isobutyl, *tert-butyl,* pentyl, isopentyl, neopentyl, or hexyl, such as methyl or ethyl;
(2) in R², R³, R⁴, R⁵, R⁷, R⁸, and R⁹, the C₁₋₆ alkoxy in the C₁₋₆ alkoxy and substituted C₁₋₆ alkyl is independently methoxy, ethoxy, n-propoxy, isopropoxy, n-butoxy, isobutoxy, *sec-*butoxy, or *tert*-butoxy, such as methoxy;
(3) in R⁰, R¹, R², R³, R⁴, R⁵, R⁶, R⁷, R⁸, R⁹, R¹¹, R¹³, R¹⁴, R¹⁵, and R¹⁶, the C₃₋₆ cycloalkyl in the C₃₋₆ cycloalkyl, -C₁₋₃ alkyl-C₃₋₆ cycloalkyl, substituted C₃₋₆ cycloalkyl, and substituted - C₁₋₃ alkyl-C₃₋₆ cycloalkyl is independently cyclopropyl, cyclobutyl, cyclopentyl, or cyclohexyl, such as cyclopropyl or cyclobutyl;
(4) in R², R³, R⁴, R⁵, R⁷, R⁸, R⁹, R¹¹, R¹³, R¹⁴, R¹⁵, R¹⁶, R^{a}, R^{b}, R^{c}, R^{d}, and R^{e}, the halogen is independently fluorine, chlorine, bromine, or iodine, such as fluorine.

3. The compound of formula (I), the stereoisomer thereof, the tautomer thereof, the isotopically labeled compound thereof, or the pharmaceutically acceptable salt of any one of the foregoing according to claim 1, wherein the compound of formula (I) satisfies one or more of the following conditions:
(1) L is a single bond, O, or S;
(2) p is 1;
(3) R¹ is C₁₋₆ alkyl, C₃₋₆ cycloalkyl, -C₁₋₃ alkyl-C₃₋₆ cycloalkyl, or the following group substituted by one or more R^{a}: C₁₋₆ alkyl, C₃₋₆ cycloalkyl, or -C₁₋₃ alkyl-C₃₋₆ cycloalkyl;
(4) each R^{a} is independently halogen, such as fluorine;
(5) R², R³, R⁴, R⁵, R⁶, and R⁸ are hydrogen;
(6) R⁷ is C₁₋₆ alkyl, such as methyl;
(7) X is -C(=O)-, -CFR¹¹-, -NR¹²-, -S-, or -S(=O)₂-;
(8) R⁹ is C₁₋₆ alkoxy, such as methoxy;
(9) R¹¹ is H or halogen;
(10) R¹² is hydrogen or C₁₋₆ alkyl;
(11) m is 0;
(12) n is 0.

4. The compound of formula (I), the stereoisomer thereof, the tautomer thereof, the isotopically labeled compound thereof, or the pharmaceutically acceptable salt of any one of the foregoing according to claim 1, wherein the compound of formula (I) satisfies one or more of the following conditions:
(1) L is a single bond or O;
(2) X is -C(=O)-, -NR¹²-, or -S(=O)₂-;
(3) R² is hydrogen or OH;
(4) R³, R⁴, R⁵, R⁶, and R⁸ are hydrogen;
(5) R¹² is hydrogen;
(6) m is 0 or 1, such as 0.

5. The compound of formula (I), the stereoisomer thereof, the tautomer thereof, the isotopically labeled compound thereof, or the pharmaceutically acceptable salt of any one of the foregoing according to claim 1, wherein the compound of formula (I) satisfies one or more of the following conditions:
(1) L is a single bond, 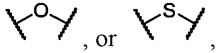 preferably a single bond or 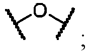
(2) R¹ is 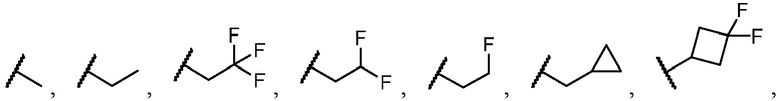 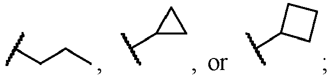 preferably 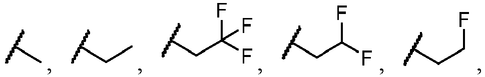 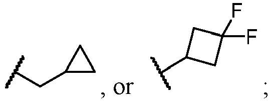
(3) X is 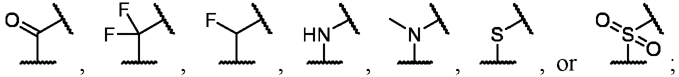 preferably 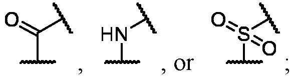 more preferably

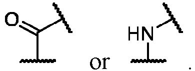

6. The compound of formula (I), the stereoisomer thereof, the tautomer thereof, the isotopically labeled compound thereof, or the pharmaceutically acceptable salt of any one of the foregoing according to claim 5, wherein the compound of formula (I) satisfies one or more of the following conditions:
(1) 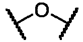 is 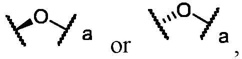 wherein a-terminus is connected to R¹;
(2) 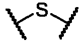 is 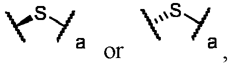 wherein a-terminus is connected to R¹;
(3) 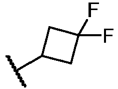 is

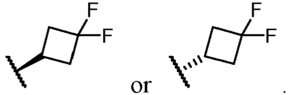

7. The compound of formula (I), the stereoisomer thereof, the tautomer thereof, the isotopically labeled compound thereof, or the pharmaceutically acceptable salt of any one of the foregoing according to claim 1, wherein R¹ is 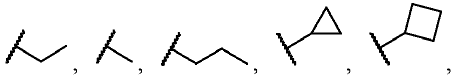 or 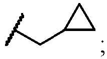 preferably

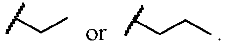

8. The compound of formula (I), the stereoisomer thereof, the tautomer thereof, the isotopically labeled compound thereof, or the pharmaceutically acceptable salt of any one of the foregoing according to claim 1, wherein the compound of formula (I) satisfies one or more of the following conditions:
(1) -L-R¹ is 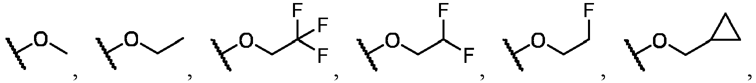 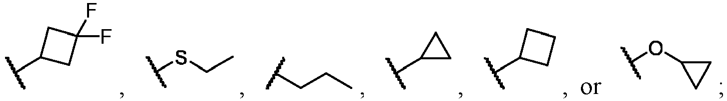 preferably 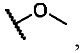 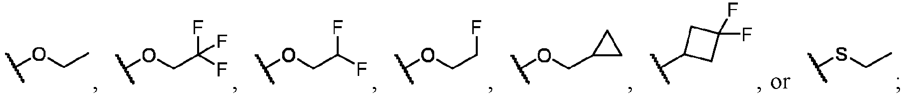
(2) 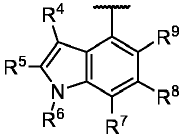 is

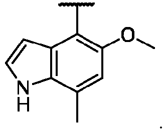

9. The compound of formula (I), the stereoisomer thereof, the tautomer thereof, the isotopically labeled compound thereof, or the pharmaceutically acceptable salt of any one of the foregoing according to claim 8, wherein the compound of formula (I) satisfies one or more of the following conditions:
(1) 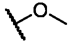 is 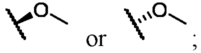
(2) 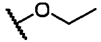 is 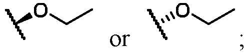
(3) 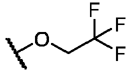 is 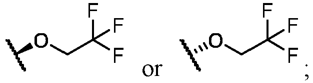
(4) 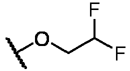 is 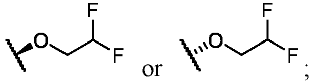
(5) 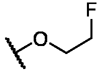 is 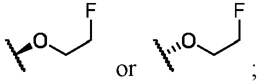
(6) 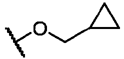 is 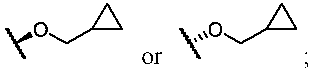
(7) 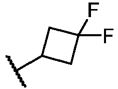 is 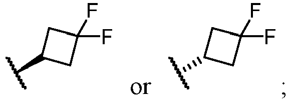
(8) 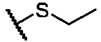 is 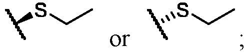
(9) 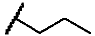 is 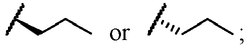
(10) 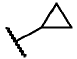 is 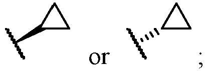
(11) 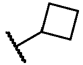 is 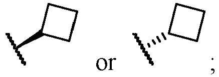
(12) 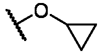 is

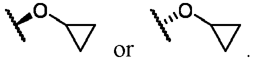

10. The compound of formula (I), the stereoisomer thereof, the tautomer thereof, the isotopically labeled compound thereof, or the pharmaceutically acceptable salt of any one of the foregoing according to claim 1, wherein -L-R¹ is 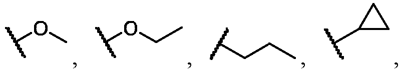 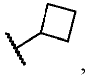 or 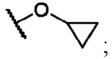 more preferably

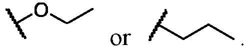

11. The compound of formula (I), the stereoisomer thereof, the tautomer thereof, the isotopically labeled compound thereof, or the pharmaceutically acceptable salt of any one of the foregoing according to any one of claims 1-10, wherein the formula (I) is as shown in formula (I)-1, formula (I)-2, formula (I)-3, or formula (I)-4: 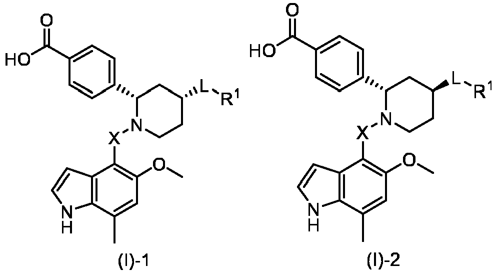 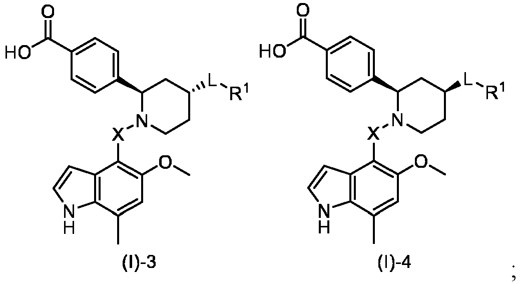 preferably, the formula (I) is as shown in formula (I)-1 or formula (I)-2; more preferably, the formula (I) is as shown in formula (I)-2.

12. The compound of formula (I), the stereoisomer thereof, the tautomer thereof, the isotopically labeled compound thereof, or the pharmaceutically acceptable salt of any one of the foregoing according to claim 1, wherein the compound of formula (I) is selected from any one of the following compounds:

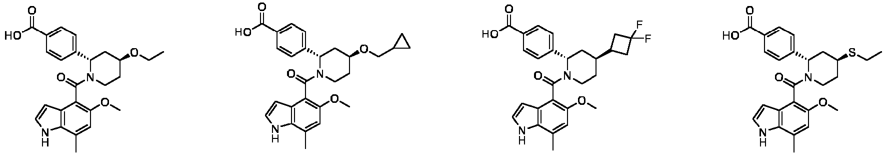

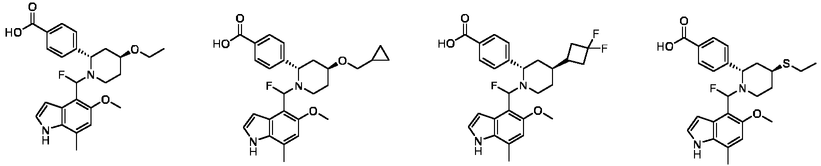

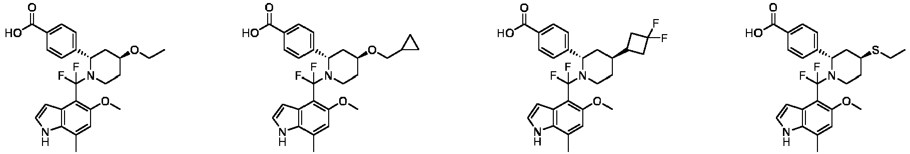

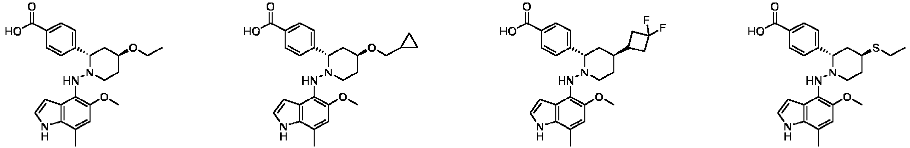

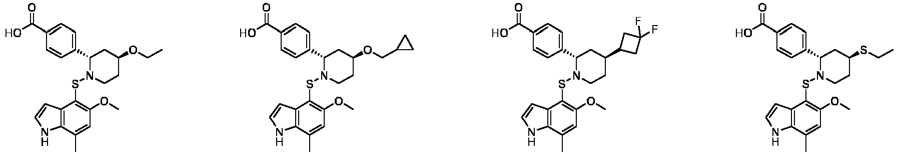

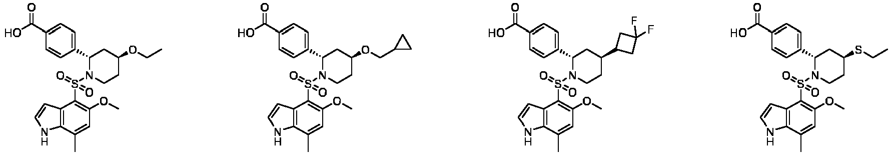

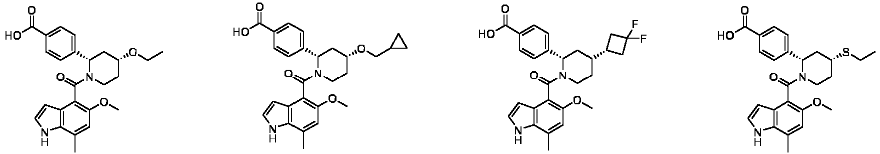

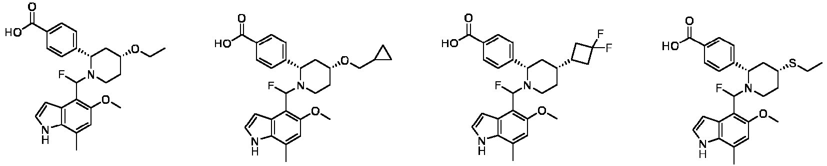

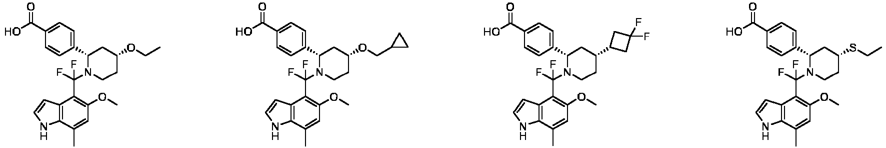

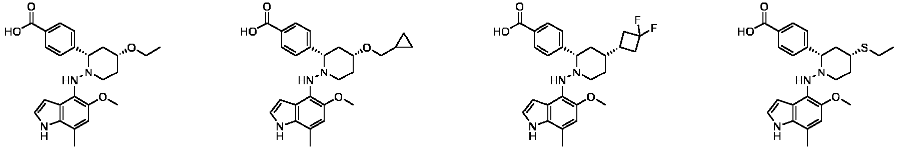

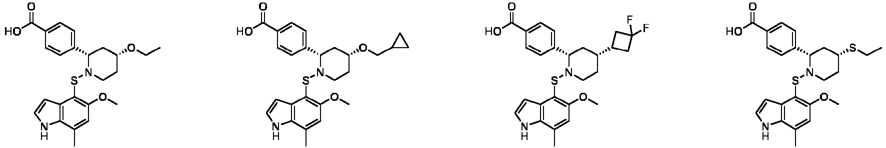

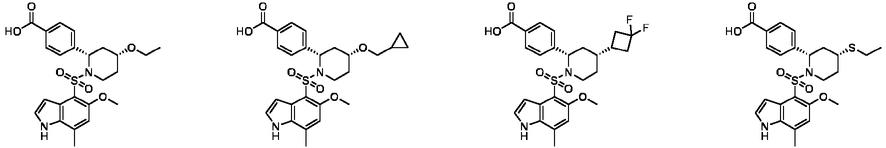

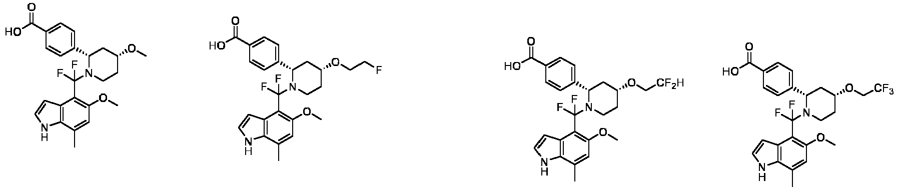

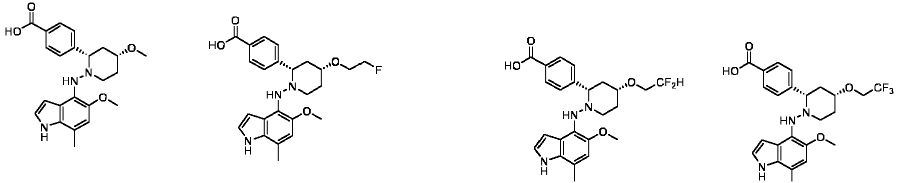

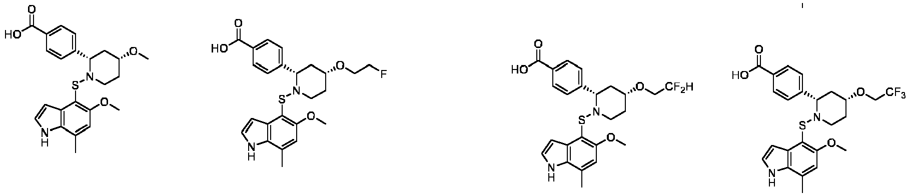

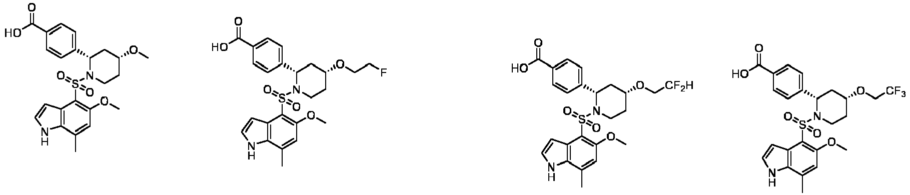

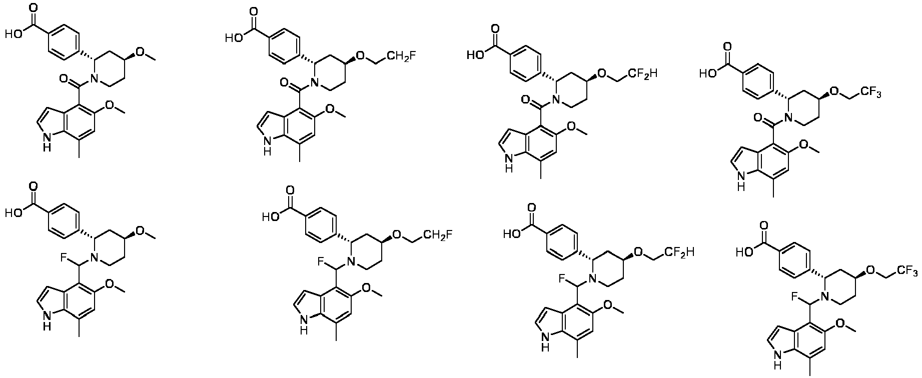

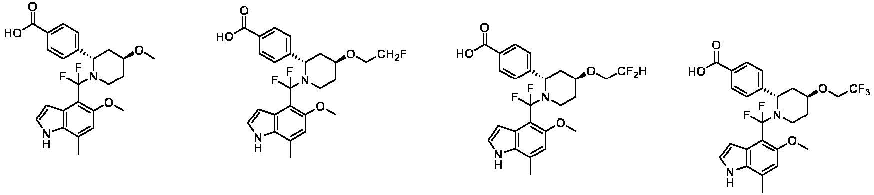

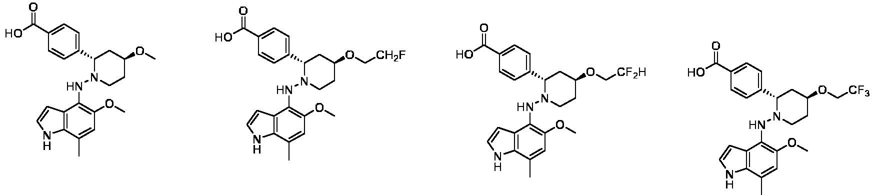

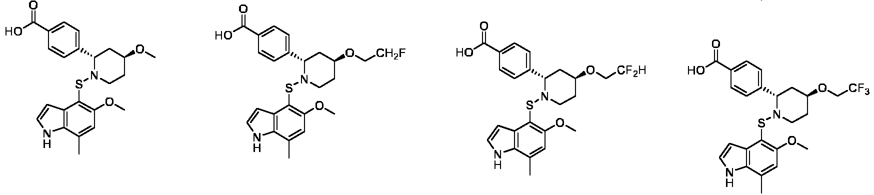

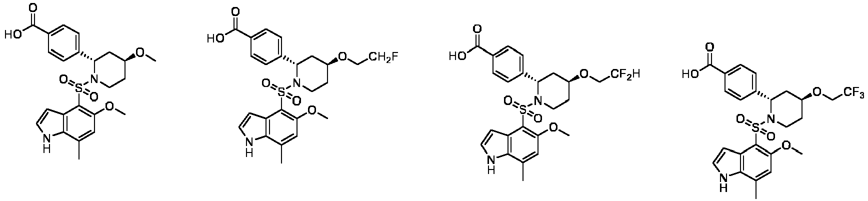

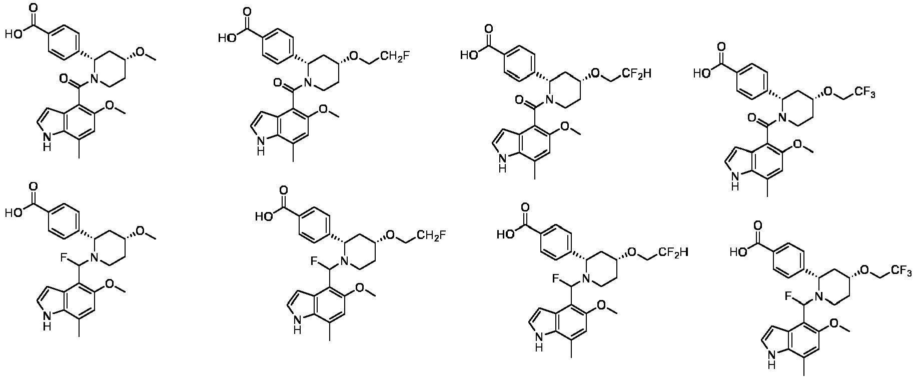

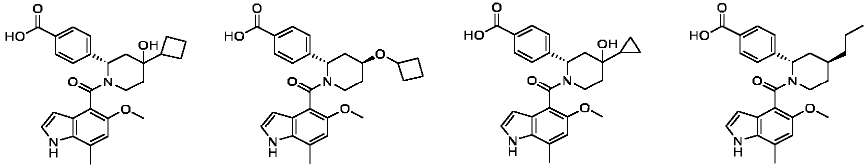

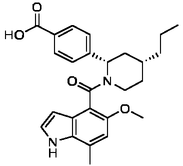

13. A pharmaceutical composition, comprising:
(1) the compound of formula (I), the stereoisomer thereof, the tautomer thereof, the isotopically labeled compound thereof, or the pharmaceutically acceptable salt of any one of the foregoing according to any one of claims 1-10; and
(2) a pharmaceutically acceptable carrier.

14. A use of the compound of formula (I), the stereoisomer thereof, the tautomer thereof, the isotopically labeled compound thereof, or the pharmaceutically acceptable salt of any one of the foregoing according to any one of claims 1-10, or the pharmaceutical composition according to claim 10;
the use satisfies one or more of the following conditions:
(1) in the manufacture of a medicament for treating and/or preventing a complement factor B-mediated disease;
preferably, the complement factor B-mediated disease is a hematologic disease, an autoimmune disease, an inflammatory disease, a neurological disorder, or a disease associated with abnormal complement system function;
further, the complement factor B-mediated disease is paroxysmal nocturnal hemoglobinuria, C3 glomerulopathy, macular degeneration, or age-related macular degeneration;
(2) in the manufacture of a complement factor B inhibitor.

15. A compound of formula (II)-1, formula (II)-2, or formula (II)-3, 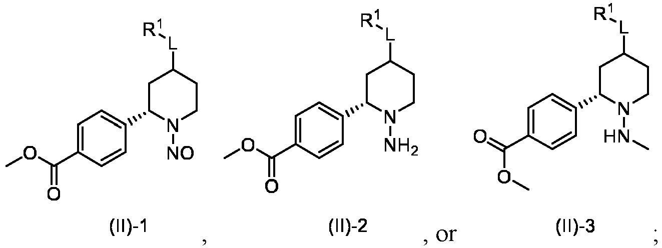
wherein L and R¹ are as defined in any one of claims 1-10;
preferably, the compound of formula (II)-1 is selected from any one of the following compounds: 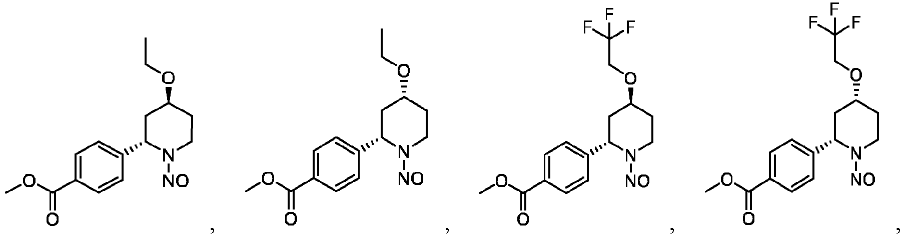 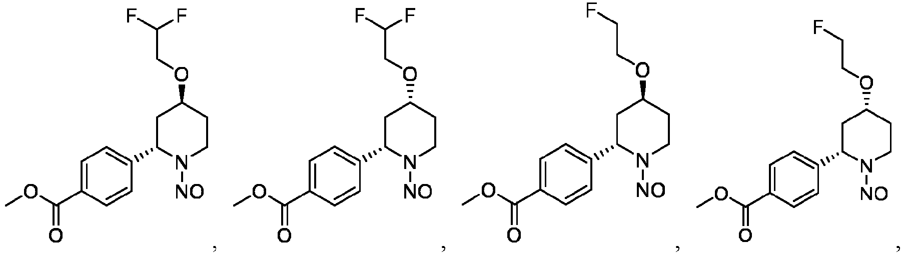 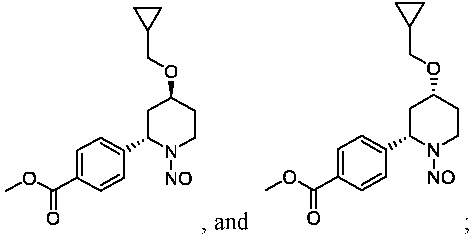
preferably, the intermediate compound of formula (II)-2 is selected from any one of the following compounds: 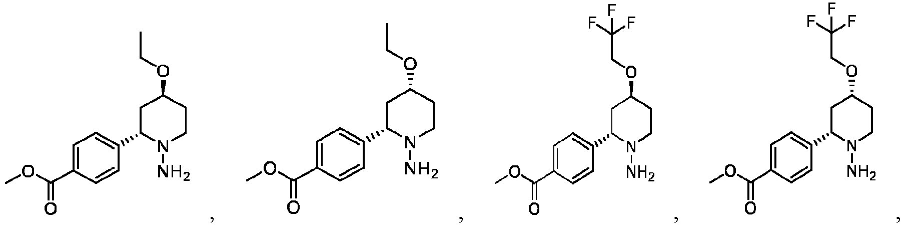 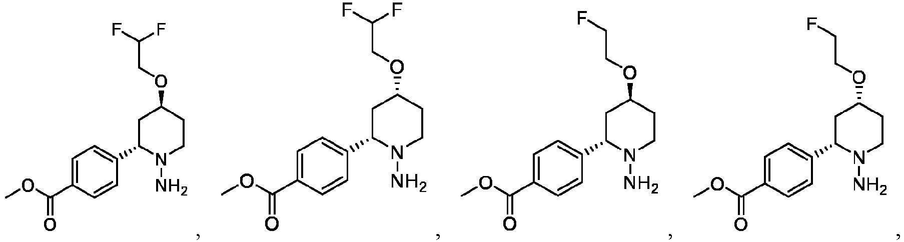 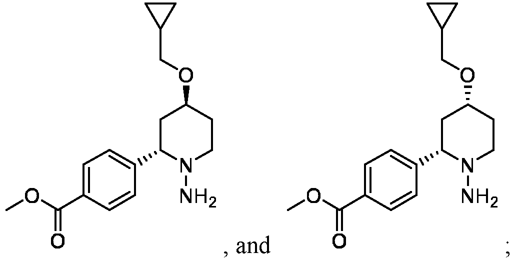
preferably, the intermediate compound of formula (II)-3 is selected from any one of the following compounds:

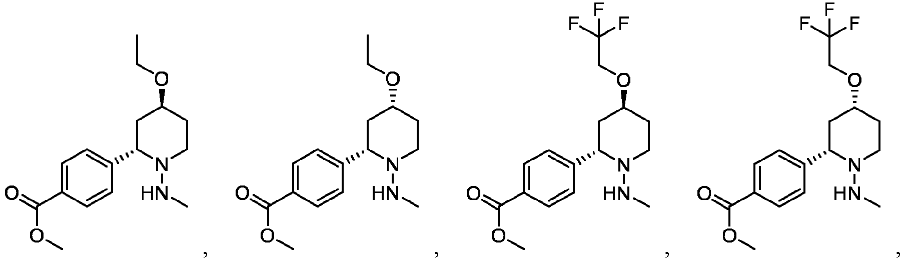

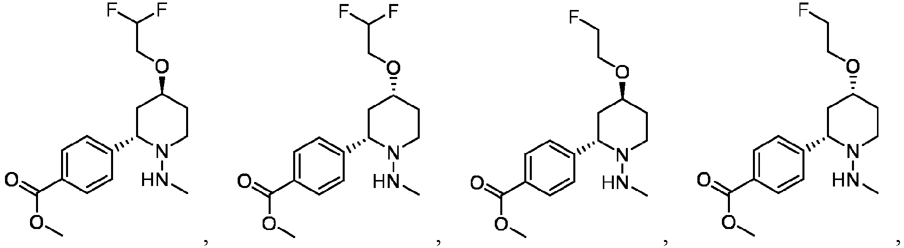

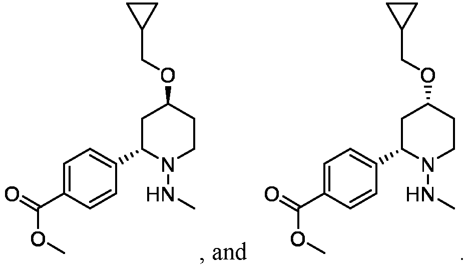

16. A compound selected from any one of the following compounds: 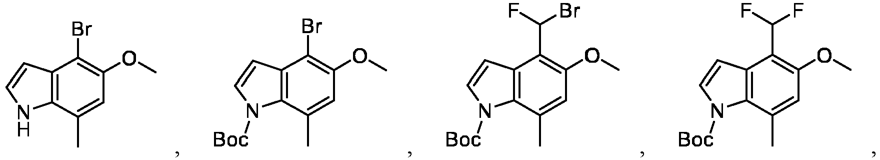 and
